(19) Europäisches Patentamt
European Patent Office
Office européen des brevets

(11) EP 3 643 780 A1

(12) # EUROPEAN PATENT APPLICATION
published in accordance with Art. 153(4) EPC

(43) Date of publication:
**29.04.2020 Bulletin 2020/18**

(21) Application number: **18820091.9**

(22) Date of filing: **04.06.2018**

(51) Int Cl.:
*C12N 15/09* (2006.01) *C12N 5/0735* (2010.01)
*C12N 5/10* (2006.01) *C12Q 1/02* (2006.01)

(86) International application number:
**PCT/JP2018/021430**

(87) International publication number:
**WO 2018/235583 (27.12.2018 Gazette 2018/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.06.2017 JP 2017120024**
**12.12.2017 JP 2017237420**

(71) Applicant: **Foundation for Biomedical Research and Innovation at Kobe**
**Kobe-shi**
**Hyogo 650-0047 (JP)**

(72) Inventors:
- **KAWAMATA, Shin**
**Kobe-shi**
**Hyogo 650-0047 (JP)**
- **YAMAMOTO, Takako**
**Kobe-shi**
**Hyogo 650-0047 (JP)**
- **TAKENAKA, Chiemi**
**Kobe-shi**
**Hyogo 650-0047 (JP)**

(74) Representative: **Weickmann & Weickmann PartmbB**
**Postfach 860 820**
**81635 München (DE)**

(54) **METHOD FOR PREDICTING DIFFERENTIATION ABILITY OF PLURIPOTENT STEM CELL, AND REAGENT FOR SAME**

(57) Provided is a marker capable of predicting differentiation resistance of a pluripotent stem cell while it is in an undifferentiated state, and a method for rapidly evaluating differentiation potential of the pluripotent stem cell by using expression of the marker as an index. Also provided is a means for searching for culture conditions suitable for maintaining the differentiation potential of a pluripotent stem cell by using expression of the differentiation resistance prediction marker as an index. Further provided is a method for evaluating a medium for a pluripotent stem cell by using expression of the differentiation resistance prediction marker as an index.

A method for predicting a differentiation potential of a pluripotent stem cell comprising measuring an expression level of CHD7of the human pluripotent stem cell. A method for evaluating a medium for a human pluripotent stem cell comprising measuring an expression level of CHD7 of the pluripotent stem cell.

EP 3 643 780 A1

## Description

[Technical Field]

The present invention relates to a method for predicting differentiation potential of a pluripotent stem cell, and a reagent and a kit therefor. The present invention also relates to a method for evaluating a medium for a pluripotent stem cell, and a reagent and a kit therefor. The present invention further relates to a method for reducing or eliminating differentiation resistance of a pluripotent stem cell.

[Background Art]

Embryonic stem cell (ESC) and induced pluripotent stem cell (iPSC) are pluripotent stem cells (PSCs) having two characteristics: the ability to proliferate in an undifferentiated state (self-proliferation ability) and the potential to differentiate into three germ layer lineages (differentiation potential) in response to differentiation stimuli. However, differentiation potential cannot be verified until an external differentiation stimulus is applied. While the mechanism by which PSC stops self-proliferation and switches to the initiation of differentiation and a series of processes thereof are extremely important in understanding the biology of PSC, they have not been fully elucidated.
This problem is particularly important when cell therapy using PSC-derived cells is to be performed. The inclusion of undifferentiated cells in the final product of PSC-derived differentiated cells creates a risk of tumorigenesis. Therefore, it is clinically highly valuable to understand the process of the initiation of differentiation.
The group of Okano and Yamanaka et al. disclosed a method for selecting iPSC-derived differentiated cells with a reduced risk of tumorigenesis by inducing secondary neurosphere (SNS) from iPSCs and using Nanog gene expression in the SNS as an index (patent document 1). Furthermore, the group considered that the differentiation resistance of iPSCs is an inherent property of the iPSC clone rather than the property due to differentiation-inducing conditions, and disclosed a method for evaluating differentiation resistance of iPSC by using the expression of Nanog gene in SNS derived from the iPSCs as an index (patent document 2).
However, all of these methods require time and labor to once differentiate iPSCs into SNS for evaluation of the differentiation resistance. If a marker capable of predicting differentiation resistance while PSCs are in an undifferentiated state is found, a safe cell therapy agent without a risk of tumorigenesis can be provided quickly and easily. However, such differentiation marker has not yet been reported.

[Document List]

[Patent documents]

patent document 1: Japanese Translation of PCT International Application Publication No. 2011-530273
patent document 2: Japanese Translation of PCT International Application Publication No. 2012-527887

[SUMMARY OF THE INVENTION]

[Problems to be Solved by the Invention]

An object of the present invention is to provide a marker capable of predicting differentiation resistance of a pluripotent stem cell (PSC) while it is in an undifferentiated state, and provide a method for rapidly evaluating differentiation potential of the pluripotent stem cell by using expression of the marker as an index.
Another object of the present invention is to provide a means for searching for culture conditions suitable for maintaining the differentiation potential of a pluripotent stem cell by using expression of the aforementioned differentiation resistance prediction marker as an index.
A still another object of the present invention is to provide a method for evaluating a medium for a pluripotent stem cell by using expression of the aforementioned differentiation resistance prediction marker as an index.
A further object of the present invention is to provide a method for reducing or eliminating differentiation resistance of a pluripotent stem cell.

[Means of Solving the Problems]

PSC shows resistance to differentiation if it acquires genetic abnormality during long-term culture or the reprogramming process in the case of iPSC. Genetic abnormality can be tested in a timely manner by using the state-of-the-art sequencing techniques, and PSC and PSC derivative used in cell therapy can be frequently subjected to genetic screening to eliminate genetically abnormal cells. However, even PSC with a normal karyotype sometimes shows differentiation resistance caused by epigenetic modification due to culture conditions, and it is necessary to regularly confirm the quality of PSC by testing the differentiation potential of the PSC by embryoid body (EB) formation assay or cytokine-induced differentiation assay.

In the course of performing an EB formation assay of PSCs, the present inventors have found that certain culture conditions confer differentiation resistance to both ESCs and iPSCs. That is, when PSCs were cultured with commercially available Essential 8 medium (Thermo Fisher Scientific, hereinafter to be also referred to as "Es8") or Stem-Partner (registered trade mark) Human iPS/ES cells medium (KYOKUTO PHARMACEUTICAL INDUSTRIAL CO., LTD., hereinafter to be also referred to as "SPM") (Takenaka et al., PLoS One 10(6), 2015), EBs were formed. However, when the PSCs were transferred to a commercially available ReproFF2 medium (REPROCELL, hereinafter to be also referred to as "RFF2") and cultured for 5 passages or more, it lost differentiation potential and no longer formed EBs. When the PSCs were placed back in Es8 or SPM and further cultured for 5 passages or more, the differentiation potential was recovered. The present inventors have found Chromodomain Helicase DNA binding protein 7 (hereinafter to be also referred to as "CHD7") as one of the gene candidates relating to the reversible alteration in the differentiation potential of PSC based on the analyses by principal component analysis (hereinafter to be also referred to as PCA) and GeneChip analysis. The effect of PSC culture with Es8 and RFF2 on the expression of CHD7 gene was examined to find that the expression of CHD7 gene was remarkably suppressed by culturing with RFF2, whereas CHD7 gene showed a moderate expression level in PSCs by culturing with Es8. When CHD7 mRNA was introduced into PSCs cultured with RFF2 and CHD7 was upregulated, PSCs spontaneously initiated to differentiate, and undifferentiated cell maintaining culture system did not support proliferation of differentiated cells. On the other hand, when CHD7 siRNA was introduced into PSCs cultured with Es8 and CHD7 was downregulated, the differentiation potential of PSCs were partially impaired. That is, it was clarified that PSC can proliferate in an undifferentiated state if CHD7 expression does not exceed a certain upper limit; however, when the expression falls below a threshold value level, PSC no longer responds to differentiation stimuli, and PSC having a CHD7 expression range between the upper limit and the threshold value maintains the property that can respond to differentiation stimuli. From the above results, the present inventors have confirmed that differentiation potential/differentiation resistance of PSC can be predicted using the expression level of CHD7 in PSC in an undifferentiated state as an index, which resulted in the completion of the present invention.

Accordingly, the present invention provides the following.

[1] A method for predicting a differentiation potential of a pluripotent stem cell comprising measuring an expression level of CHD7 of the human pluripotent stem cell.

[2] The method of [1], wherein a human pluripotent stem cell having aforementioned expression level of CHD7 of not less than 1500 copies in 5 ng of the total RNA is predicted to show a differentiation potential in response to a differentiation stimulus.

[3] The method of [2], wherein the aforementioned expression level of CHD7 is not less than 2710 copies in 5 ng of the total RNA.

[4] The method of [2] or [3], wherein the aforementioned expression level of CHD7 is an expression level of a human pluripotent stem cell cultured for not less than 5 passages with Essential 8 medium or Stem-Partner (registered trade mark) Human iPS/ES cells medium.

[5] The method of any of [1] to [4], wherein the aforementioned human pluripotent stem cell is an embryonic stem cell or an induced pluripotent stem cell.

[6] A method for evaluating a medium for a human pluripotent stem cell comprising measuring an expression level of CHD7 of the pluripotent stem cell.

[7] The method of [6], wherein the aforementioned human pluripotent stem cell is a human pluripotent stem cell cultured for not less than 5 passages with the test medium.

[8] The method of [6] or [7], wherein the test medium is evaluated as being capable of maintaining the human pluripotent stem cell to show a differentiation potential in response to a differentiation stimulus, when the aforementioned expression level of CHD7 is not less than 1500 copies in 5 ng of the total RNA.

[9] The method of [8], wherein the aforementioned expression level of CHD7 is not less than 2710 copies in 5 ng of the total RNA.

[10] The method of any of [6] to [9], wherein the aforementioned human pluripotent stem cell is an embryonic stem cell or an induced pluripotent stem cell.

[11] A reagent or kit for predicting a differentiation potential of a human pluripotent stem cell and/or evaluating a medium for human pluripotent stem cell, comprising a substance capable of detecting an expression of CHD7.

[12] A differentiation-inducing agent for a human pluripotent stem cell comprising a nucleic acid encoding CHD7.

[13] The method of [1], wherein the human pluripotent stem cell is predicted to show a differentiation potential in response to a differentiation stimulus when the aforementioned expression level of CHD7 is a protein level not less than two times a CHD7 protein level of a human pluripotent stem cell showing a differentiation resistance.

[14] The method of [13], wherein the aforementioned expression level of CHD7 is an expression level of a human pluripotent stem cell cultured for not less than 5 passages with Essential 8 medium or Stem-Partner (registered trade mark) Human iPS/ES cells medium.

[15] The method of [13] or [14] wherein the aforementioned human pluripotent stem cell showing the differentiation resistance is a human pluripotent stem cell cultured for not less than 5 passages with ReproFF2 medium.

[Effect of the Invention]

According to the present invention, whether a human pluripotent stem cell (PSC) shows a differentiation potential in response to a differentiation stimulus can be predicted in an undifferentiated state before applying a differentiation stimulus. In addition, culture conditions suitable for maintaining a human pluripotent stem cell in a state holding a property showing a differentiation potential in response to a differentiation stimulus can be found. Furthermore, a medium suitable for culturing a human pluripotent stem cell can be evaluated by measuring an expression level of CHD7 of a human pluripotent stem cell. Moreover, differentiation resistant can be reduced or eliminated by increasing an expression level of CHD7 (selecting cell with high expression) in a human pluripotent stem cell, particularly iPSC population.

[Brief Description of the Drawings]

Fig. 1 shows the differentiation potential of PSC altered depending on the culture conditions. KhES-1 cells in a single cell suspension were seeded on a dish coated with rhVitronectin-N (Thermo Fisher Scientific, hereinafter to be also referred to as "VNT-N"), and cultured for 5 passages with Essential 8 medium (hereinafter also referred to as "Es8") (upper left photograph). The cells were then collected for embryoid body (EB) formation (lower left photograph) or transferred to ReproFF2 medium (RFF2) (upper center photograph). KhES-1 cells were cultured for 5 passages and collected for EB formation assay (lower center photograph) or transferred again to Es8 (upper right photograph). KhES-1 cells were cultured for 5 passages and EB formation assay was performed (lower right photograph). Photographs of the KhES-1 culture using either Es8 or RFF2 medium on the first day of culture (upper) and photographs of EB on the 14th day (lower) are shown. The gene expression profiles of the cells under the aforementioned culture conditions were determined by qRT-PCR scorecard panel and shown under the photographs (scale bar: 1.0 mm).

Fig. 2 shows the potential of iPSCs to EB formation altered depending on the culture conditions. iPSCs (PFX#9) in a single cell suspension were seeded on a dish coated with VNT-N and cultured for 5 passages with Es8 (upper left photograph). The cells were then collected for EB formation assay (lower left photograph) or transferred to ReproFF2 medium (RFF2) (upper center photograph). PFX#9 cells were cultured for 5 passages and collected for EB formation assay (lower center photograph) or transferred again to Es8 (upper right photograph). PFX#9 cells were cultured for 5 passages and EB formation assay was performed (lower right photograph). Photographs of the PFX#9 culture using either Es8 or RFF2 medium on the first day of culture (upper) and photographs of EB on the 14th day in EB formation assay (lower) are shown. The gene expression profiles of the cells under the aforementioned culture conditions were determined by qRT-PCR scorecard panel and shown under the photographs (scale bar: 1.0 mm) .

Fig. 3 shows comparison of methylation of PSCs cultured under various conditions. A. The methylation status of the cells were determined by Illumina Human Methylation Bead Chip. Average methylation scores of 6 PSC samples (3 samples of iPSC (PFX#9) and 3 samples of ESC (KhES-1, H9)) cultured with RFF2 are shown. The score was determined by comparison with an average of 6 PSC samples (1 sample of PFX#9 cultured with SPM, 1 sample of KhES-1 cultured with SPM, 1 sample of H9 cultured with SPM, 1 sample of PFX#9 cultured with Es8, 2 samples of H9 cultured with Es8) with SPM or Es8. The number of genes in the promoter region or genes in all regions that showed the methylation score exceeding 0.2 by culturing with RFF2 or SPM and Es8 is shown in Venn diagram. B. shows clustering of methylation patterns in the promoter region of PSC cultured with RFF2, SPM or Es8. lane #1 - 3: the results of iPSCs (PFX#9) cultured with RFF2 medium, lane #4 - 6: the results of ESCs (KhES-1) cultured with RFF2 from 6 independent experiments, lane #7: the results of PFX#9 cells with SPM, lane #8: the results of KhES-1 cells with SPM, lane #9: the results of H9 ESCs with SPM, lane #10: the results of PFX#9 cell with Es8, lane #11, 12: the results of H9 with Es8 from 6 independent experiments.

Fig. 4 shows gene expression of CHD7 and self-growth factors POU5F1, SOX2, NANOG and EP300 in KhES-1 cultured with Es8 (P5 and P15) or RFF2 medium (P9 and P18) as determined by qRT-PCR. The relative amount is shown in bar graphs with the expression level in P5 using Es8 as 1. The passage numbers of (P) were added to the bars.

Fig. 5 shows schematic diagrams of CHD7 isoform 1, isoform 2 and isoform X4 and mRNA transcripts. A. CHD7 isoform 1, isoform 2 and isoform X4 having function domains and the location of PCR primer set are shown. B. CHD7 mRNA transcript (isoform 2), dominant negative transcripts and the target domains of siRNA used for evaluation of the function of CHD7 are shown.

Fig. 6 shows CHD7 isoforms detected by Western blotting. Whole cell lysates (5.3 μg) from KhES-1 cells cultured with Es8 (P11) or RFF2 (P21) were applied to each lane. Antibodies against human CHD7 were used to detect CHD7 isoform 1 (expected mass 336 kDa), isoform 2 (110 kDa) and isoform X4 (183 kDa). Signal was visualized with horseradish peroxidase.

Fig. 7 shows down regulation of CHD7 by siCHD7 transfection. A. Illustration of the protocol for EB formation and siCHD7 transfection. $2\times10^5$ KhES-1 cells were seeded on a 6-well dish coated with rhVitronectin-N (recombinant human vitronectin-N) (hereinafter to be also referred to as "VTN-N") and cultured with Es8 on day -1. Cells were transfected with small double-stranded interfering RNA against CHD7 (siCHD7) or non-specific control siRNA (mock) (day 0). After 24 hr from the transfection of siRNA, the cells were transferred to a low-attachment plate, cultured for 24 hr with Essential 6 (Es6) supplemented with ROCK (Rho-associated protein kinase) inhibitor (RI). The medium was changed to fresh Es6 medium and the cells were further cultured for 72 hr. B. Dependence of the introduced amount of siCHD7 on CHD7 gene expression level is shown. C. CHD7 gene expression level after siRNA introduction is shown. Expression of CHD7 or CHD7 in KhES-1 cells transfected with siCHD7 or control siRNA (mock) or non-transfected cells was determined by qRT-PCR (time course sampling, left) or Western blotting (sampled on day 2, right). The gene expression of CHD7 was normalized by the average of CHD7 expression measured independently three times in KhES-1 cells cultured with Es8. The representative datasets obtained from 3 independent experiments are shown.

Fig. 8 shows photographs of non-transfected EBs (upper panel), siCHD7-transfected KhES-1 cells (middle panel), and control siRNA-transfected KhES-1 cells (mock, lower panel) on day 4, day 5 and day 14. The gene expression profiles of KhES-1 cells under the specified conditions determined by qRT-PCR scorecard panel are shown under the corresponding photographs (scale bar: 1.0 mm). The representative datasets obtained from 3 independent experiments are shown.

Fig. 9 shows that downregulation of CHD7 supports the survival of ESCs cultured with nutrient-depleted medium. A. Illustration of a protocol for differentiation induction by nutrient-depleted Es8 and transfection of siCHD7. On day 0, cells were transfected with siCHD7 or control siRNA (mock) after medium (Es8) change. The medium was changed every 2 days (days 2 and 4). For cell counting and determination of gene expression by qRT-PCR, the cells were harvested 42 hr (day 2), 72 hr and 96 hr after transfection of siRNA. In the case of normal culture, ESCs were cultured with Es8. The medium was changed every day, and passage was performed every 3 days to maintain an undifferentiated state. B. Gene expression of CHD7 in KhES-1 cells transfected with siCHD7 or control siRNA (mock) or non-untransfected cells on day 0, day 1, day 2, day 3 and day 4 is shown. The gene expression of CHD7 was normalized by the average of CHD7 expression measured by qRT-PCR independently three times in KhES-1 cells cultured with Es8. The representative datasets obtained from 3 independent experiments are shown. C. Line graph plotting the number of non-transfected KhES-1 cells (non-transfected), siCHD7-transfected KhES-1 cells (siCHD7), and mock-transfected KhES-1 cells (mock) is shown. The representative datasets obtained from 3 independent experiments are shown.

Fig. 10 shows photographs of non-transfected regularly cultured KhES-1 cells (upper panels, medium changed every day), siCHD7-transfected KhES-1 cells (middle panels, medium changed every 2 days) and control siRNA-transfected KhES-1 cells (mock) (lower panels, medium changed every 2 days), each on day 2, day 3 and day 4. The gene expression profiles determined by qRT-PCR scorecard panel of KhES-1 are shown under each photograph (scale bar: 1 mm).

Fig. 11 shows that upregulation of CHD7 isoform 2 induces "spontaneous" differentiation in ESCs cultured with RFF2. A. Protocol for cell culture and transfection with mCHD7 is shown. On day 0 and day 1, KhES-1 cells were transfected with mCHD7 or control mRNA (mock) (2 times in total). On day 2, the cells were passaged, reseeded on a 6-well plate at $2\times10^5$ cells/well, and further cultured for 24 hr. B. The gene expression of CHD7 after introduction of mCHD7 or control mRNA (mock) (day 0) and CHD7 were determined by qRT-PCR (time course sampling, left) and Western blotting (sampled on day 3, right). The gene expression of CHD7 was normalized by the average of CHD7 expression measured independently three times in KhES-1 cells cultured with RFF2 medium (NT: non-transfected control). C. A graph showing the proliferation of transfected or non-transfected KhES-1 cells is shown. The proliferation of mock-transfected KhES-1 cells (mock) is comparable to that of non-transfected control (non-transfected). On the other hand, proliferation of mCHD7-transfected KhES-1 cells (mCHD7) decreased on day 2 and day 3. The representative datasets obtained from 3 independent experiments are shown.

Fig. 12 shows photographs of non-transfected KhES-1 cells (upper panels), mCHD7-transfected KhES-1 cells (middle panels) and control mRNA-transfected KhES-1 cells (lower panels, mock), each on day 1, day 2 and day 3 cultured with RFF2 medium. Their gene expression profiles determined by qRT-PCR scorecard panel are shown under the photographs. The representative datasets obtained from 3 independent experiments are shown (scale bar: 1 mm).

Fig. 13 shows that transfection of CHD7 dominant negative (DN) mRNA transcript inhibits or reduces differentiation potential and cell proliferation in ESCs. A. Illustration of the protocol for transfection of CHD7 DN mRNA transcript and EB formation assay is shown. CHD7 DN1 is a transcript of chromodomain mRNA and CHD7 DN2 is a transcript of SANT-SLIDE domain mRNA (Fig. 5B). On day 0, transcripts were transfected into KhES-1 cells, the cells were transferred to low-attachment plates 24 hr later and subsequently cultured for 24 hr for EB formation with Es6 medium containing ROCK Inhibitor (RI). Microscopic observation of EBs and analysis of gene expression profile by qRT-PCR scorecard panel were performed on day 3 after DN mRNA transfection. B. Graphs showing CHD7 DN1 and CHD7 DN2 expression levels determined by qRT-PCR. C. Photographs of EBs derived from non-transfected KhES-1 cells, CHD7 DN1-transfected KhES-1 cells, CHD7 DN2-transfected KhES-1 cells, CHD7 DN1- and CHD7 DN2-transfected KhES-1 cells, and mock mRNA-transfected KhES-1 cells on day 3.

Fig. 14 shows that ESC overexpressing CHD7 could not be generated in culture using Es8. A. protocol for cell culture and transfection of CHD7 isoform 2 is shown. B. CHD7 expression on day 1 and day 2 was evaluated by qRT-PCR. The gene expression of CHD7 was normalized by the average of CHD7 expression measured independently three times in KhES-1 cells cultured with Es8. C. Photographs showing the number and state of non-transfected KhES-1 cells and CHD7- or mock-transfected KhES-1 cells on day 1 and day 2. The number of cells in one well is shown in the upper right corner of the photographs.

Fig. 15 shows that downregulation of CHD7 disrupted proliferation of ESCs cultured with Es8. A. The protocol for siCHD7 transfection is shown. KhES-1 cells were transfected with siCHD7 or nonspecific control siRNA (mock) on day 0 and day 1. The medium was changed every day (MC). On days 0 - 3, the cells were harvested for cell counting and CHD7 expression was determined by qRT-PCR. B. The CHD7 gene expression in siCHD7- or control siRNA (mock)-transfected KhES-1 cells, and non-transfected KhES-1 cells was determined by qRT-PCR. The gene expression of CHD7 was normalized by the average of CHD7 expression measured independently three times in KhES-1 cells cultured with Es8. C. Photographs of non-transfected KhES-1 cells (upper panel), and siCHD7-transfected KhES-1 cells (middle panel) or control siRNA-transfected KhES-1 cells (mock, lower panel) KhES-1 cells on day 3 are shown. The number of cells is shown in the upper right corner of the photographs. D. Graph showing the cell numbers.

Fig. 16 shows the gene expression profile of human CHD7 based on GeneChip data, and shows that the CHD7 level mediated differentiation potential of PSCs. Average human CHD7 expression during early embryogenic development of three different samples (Embryos before implantation) was compared with expression of human CHD7 of human PSCs cultured under various conditions (Human PSCs on feeder or feeder-less) and expression of human CHD7 of EBs on day 14 (EB at day 14). All samples were normalized by MAS5 method.

Fig. 17 shows copy number of CHD7 of PSC before EB formation and the gene expression profile of EBs on day 14. ESC (H9, KhES-1) or iPSC (PFX#9, 201B7, SHh#2) was cultured on feeder cells and with hPSC medium, or seeded and cultured in single cells on VTN-N and with Es8 (hereinafter to be also referred to as "Single-cell suspension method"). The copy number of CHD7 isoform 1 mRNA before EB formation was detected by digital PCR. The gene expression profile of EB derived from each cell on day 14 was determined by qRT-PCR scorecard panel and shown in a bar graph (N: VTN-N-coated dish).

Fig. 18 shows copy number of CHD7 mRNA of iPSCs with lower copy number of CHD7 mRNA and the gene expression profile of EB on day 14. 201B7 or PFX#9 cells were cultured feeder cell-free in single cells on VTN-N-coated dish. The copy number of CHD7 mRNA was reduced by deliberately creating an overgrowth state. The medium used was Es8. The copy number of CHD7 mRNA before EB formation was detected by digital PCR. The gene expression profile of EB derived from each cell on day 14 was determined by qRT-PCR scorecard panel and shown in a bar graph (P: passage numbers).

Fig. 19 shows the expression level of CDH7 protein in PSCs cultured under various culture conditions. P1 is H9 cultured for 17 passages with Es8, P2 is KhES-1 cultured for 10 passages with Es8, and N is KhES-1 cultured for 11 passages with RFF2. N2 (negative control) is a concentrated lysate of H9 cells cultured for 17 passages with RFF2. All cells were cultured feeder cell-free in single cells on a VTN-N-coated dish, then lysate was prepared, with the standard (concentrated lysate of H9 cells cultured for 17 passages with Es8) as 1000 units/mL, the concentrations of the primary antibody and the secondary antibody were changed and the relative values were shown in a bar graph.

Fig. 20 is an asymptote determined from the protein concentration and the number of mRNA copies.

[Description of Embodiments]

The present invention provides a method for predicting a differentiation potential of a pluripotent stem cell (hPSC) including measuring an expression level of CHD7 of the human pluripotent stem cell (hereinafter to be also referred to as "the prediction method of the present invention").

In the present specification, the "differentiation potential" of the pluripotent stem cell means a potential of a pluripotent stem cell to differentiate into a three germ layer lineage or particular cell line spontaneously or in response to a particular differentiation stimulus. In a preferable embodiment of the present invention, a human pluripotent stem cell having a potential to differentiate into a cell line corresponding to a certain particular differentiation stimulus in response to the differentiation stimulus is predicted and selected. Such differentiation stimulus is not particularly limited as long as it is a stimulus that causes escape of PSC from an undifferentiated state and induction thereof into any differentiated cell, and the direction of the differentiation is known. For example, the culture conditions used for EB formation assay or cytokine-induced differentiation assay in the below-mentioned Examples and the like can be mentioned.

In the present specification, unless particularly indicated, the "expression level of CHD7" may mean any of the expression level of CHD7 gene and the expression level of CHD protein.

The present invention is based on, at least partially, the finding that the level of CHD7 expression required to maintain hPSC in an undifferentiated state has an upper limit but no lower limit, and the upper limit varies depending on the culture conditions. When the expression level of CHD7 of hPSC exceeds the upper limit, hPSC spontaneously initiates differentiation even in a maintenance medium of PSC and the culture system can no longer support proliferation of the differentiated cells. On the other hand, when the expression level of CHD7 of hPSC is lower than the threshold value, hPSC cannot show a sufficient differentiation potential in response to a differentiation stimulus and is maintained in an undifferentiated state. For hPSC to show a differentiation potential in response to a differentiation stimulus, therefore, it is necessary that the expression level of CHD7 is moderate in the PSC (i.e., not more than the above-mentioned upper limit and not less than the above-mentioned threshold value).

As mentioned above, the upper limit of the CHD7 expression level necessary for maintaining hPSC in an undifferentiated state is considered to vary depending on the culture conditions. When the expression level of CHD7 exceeds the upper limit under respective culture conditions, hPSC spontaneously initiates differentiation and maintenance and proliferation of the PSC becomes impossible under such culture conditions. If hPSC does not differentiate spontaneously, it means that the expression level has not exceeded the upper limit under the culture conditions. Therefore, it is not necessary to set the upper limit of the CHD7 expression level necessary for maintaining hPSC in an undifferentiated state under various culture conditions.

On the other hand, the threshold value of CHD7 expression level necessary for hPSC to show a differentiation potential in response to a differentiation stimulus when, for example, measuring the expression level of CHD7 gene by quantitative digital PCR analysis is, for example, not less than 2710 copies in 5 ng of the total RNA. When cultured in single cells, feeder cell-free, on a dish coated with extracellular substrate, it is unlimitatively, for example, not less than 1502 copies or not less than 1500 copies in 5 ng of the total RNA. When hPSC is ESC and cultured with feeder cells by the Small Cell Clumps method, it is unlimitatively, for example, not less than 2710 copies or not less than 2120 copies; when cultured in single cells, feeder cell-free, on a dish coated with extracellular substrate, it is unlimitatively, for example, not less than 3280 copies; when hPSC is iPSC and cultured with feeder cells by the Small Cell Clumps method, it is unlimitatively, for example, not less than 3080 copies or not less than 2280 copies; and when cultured in single cells, feeder cell-free, on a dish coated with extracellular substrate, it is unlimitatively, for example, not less than 1502 copies or not less than 1500 copies.

When the expression level of CHD7 protein is measured, the threshold value is, for example, not less than 2.0 times, not less than 3.0 times, not less than 4.0 times, not less than 5.0 times, not less than 6.0 times, not less than 7.0 times, not less than 7.7 times, not less than 8.0 times, not less than 8.5 times, not less than 9.0 times, not less than 9.2 times, not less than 10 times, not less than 10.2 times or not less than 10.3 times, compared to CHD7 protein concentration of human pluripotent stem cell showing differentiation resistance. Examples of the human pluripotent stem cell showing differentiation resistance include human pluripotent stem cell cultured for not less than 5 passages with ReproFF2 medium. Furthermore, when similar to the above-mentioned measurement, the threshold value is, for example, not more than 90.3%, not more than 90.2%, not more than 90%, not more than 89.1%, not more than 88.3%, not more than 87.0%, not more than 85.8%, not more than 85%, not more than 83.4%, not more than 80.2%, not more than 80%, not more than 75%, not more than 70%, not more than 50%, compared to CHD7 protein concentration of human pluripotent stem cell showing normal differentiation potential. Examples of the human pluripotent stem cell showing normal differentiation potential include human pluripotent stem cell cultured for not less than 5 passages with Es8 or SPM medium.

For example, the threshold value may be determined in consideration of the expression level of CHD7 gene measured by quantitative digital PCR analysis. Examples of the threshold value in consideration of the expression level of CHD7 gene include not less than 2 times, not less than 3 times, not less than 4 times, not less than 5 times and not less than 10 times, compared to CHD7 protein concentration of human pluripotent stem cell showing differentiation resistance. Examples of the human pluripotent stem cell showing differentiation resistance include human pluripotent stem cell cultured for not less than 5 passages with ReproFF2 medium. Furthermore, when similar to the above-mentioned case, the threshold value is, for example, not more than 50%, not more than 70%, not more than 75%, not more than 80% or not more than 90%, compared to CHD7 protein concentration of human pluripotent stem cell showing normal differentiation potential. Examples of the human pluripotent stem cell showing normal differentiation potential include human pluripotent stem cell cultured for not less than 5 passages with Es8 or SPM medium.

The CHD7 protein concentration may be a value obtained by directly measuring the CHD7 protein concentration of the lysate of the cultured cells, or may be a value relative to the lysate of the cultured cells as the standard. The cultured cell may be a stem cell showing differentiation resistance or a stem cell showing normal differentiation potential, and the lysate may or may not be concentrated.

In the prediction method of the present invention, a human pluripotent stem cell whose differentiation potential is predictable is not particularly limited as long as it is an undifferentiated cell having "self-proliferation ability" that enables proliferation while maintaining an undifferentiated state, and "differentiation potential" that enables differentiation into all three germ layer lineages. Examples include embryonic stem cell (ES cell), induced pluripotent stem cell (iPS cell), embryonic germ (EG) cell derived from a primordial germ cell, multipotent germline stem (mGS) cell isolated in the process of establishment and culture of GS cell from testis tissue and the like. The ES cell may be ES cell produced from a somatic cell by nuclear reprogramming (nt ES cell). Preferred is ES cell or iPS cell. The prediction method of the present invention is particularly preferably used in human pluripotent stem cell, and is applicable to any mammal in which pluripotent stem cell has been established or can be established. As the non-human mammal, for example, mouse, monkey, swine, rat, dog and the like can be mentioned.

Pluripotent stem cell can be produced by a method known per se. For example, for iPS cell, the methods described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO 2010/056831, WO2010/068955, WO2010/098419, WO2010/102267, WO 2010/111409, WO 2010/111422, WO2010/115050, WO2010/124290, WO2010/147395, WO2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26:2467-2474, Huangfu D, et al. (2008), Nat Biotechnol. 26:1269-1275, Shi Y, et al. (2008), Cell Stem Cell, 3, 568-574, Zhao Y, et al. (2008), Cell Stem Cell, 3:475-479, Marson A, (2008), Cell Stem Cell, 3, 132-135, Feng B, et al. (2009), Nat Cell Biol. 11:197-203, R.L. Judson et al., (2009), Nat. Biotech., 27:459-461, Lyssiotis CA, et al. (2009), Proc Natl Acad Sci U S A. 106:8912-8917, Kim JB, et al. (2009), Nature. 461:649-643, Ichida JK, et al. (2009), Cell Stem Cell. 5:491-503, Heng JC, et al. (2010), Cell Stem Cell. 6:167-74, Han J, et al.(2010), Nature. 463:1096-100, Mali P, et al. (2010), Stem Cells. 28:713-720, Maekawa M, et al. (2011), Nature. 474:225-9 and the like can be mentioned.

ES cell can be established by removing an inner cell mass from the blastocyst of a fertilized egg of a target animal, and culturing the inner cell mass on fibroblast feeder cells. In addition, the cell can be maintained by passage culture with a culture medium added with substances such as leukemia inhibitory factor (LIF), basic fibroblast growth factor (bFGF) and the like. The methods of establishment and maintenance of human and monkey ES cells are described in, for example, U.S. Pat. No. 5,843,780; Thomson J A, et al. (1995), Proc Natl. Acad. Sci. USA. 92:7844-7848; Thomson J A, et al. (1998), Science. 282:1145-1147; H. Suemori et al. (2006), Biochem. Biophys. Res. Commun., 345:926-932; M. Ueno et al. (2006), Proc. Natl. Acad. Sci. USA, 103:9554-9559; H. Suemori et al. (2001), Dev. Dyn., 222:273-279; H. Kawasaki et al. (2002), Proc. Natl. Acad. Sci. USA, 99:1580-1585; Klimanskaya I, et al. (2006), Nature. 444:481-485 and the like. As for human ES cell line, for example, WA01(H1) and WA09(H9) are available from WiCell Research Institute, and KhES-1, KhES-2 and KhES-3 are available from Institute for Frontier Medical Sciences, Kyoto University (Kyoto, Japan).

EG cell can be established by culturing a primordial germ cell in the presence of a substance such as LIF, bFGF, a stem cell factor and the like (Y. Matsui et al. (1992), Cell, 70:841-847; J. L. Resnick et al. (1992), Nature, 359:550-551).

For production of an nt ES cell, a combination of the nuclear transplantation technique (J. B. Cibelli et al. (1998), Nature Biotechnol., 16:642-646) and the ES cell production technique (mentioned above) is used (Kiyoka Wakayama et al., (2008), Experimental Medicine, Vol. 26, No. 5 (Suppl.), pp. 47-52). In nuclear transplantation, reprogramming can be performed by injecting the nucleus of a somatic cell to an enucleated unfertilized egg of a mammal, and culturing for a few hours.

The mGS cell can be produced from a testis cell according to the method described in WO 2005/100548.

PSC produced as described above, preferably hPSC, may preferably contain a step of maintaining and culturing PSC prior to the measurement of the expression level of CHD7. The culture may be suspension culture or adhesive culture using a coated culture dish. In this step, adhesive culture is preferably used. PSC can be dissociated, for example, physically, or dissociated using a dissociation solution having protease activity and collagenase activity (e.g., Accutase (TM), Accumax (TM) and the like) or a dissociation solution having collagenase activity alone, or Trypsin/EDTA. Preferably, a method of dissociating cells by using Trypsin/EDTA is used.

When detaching PSC, it is preferable to contain a Rho-associated protein kinase (ROCK) inhibitor in the medium. The ROCK inhibitor is not particularly limited as long as it can suppress the function of ROCK. For example, Y-27632 can be preferably used in the present invention. The concentration of Y-27632 in the medium is not particularly limited and is preferably 1 μM - 50 μM, for example, 1 μM, 2 μM, 3 μM, 4 μM, 5 μM, 6 μM, 7 μM, 8 μM, 9 μM, 10 μM, 11 μM, 12 μM, 13 μM, 14 μM, 15 μM, 16 μM, 17 μM, 18 μM, 19 μM, 20 μM, 25 μM, 30 μM, 35 μM, 40 μM, 45 μM or 50 μM, but it is not limited to these.

The period during which the ROCK inhibitor is added to the medium may be the culture period in the step of culturing PSC, or any period that suppresses cell death at the time of single dispersion, for example, at least one day.

In the present specification, the suspension culture is forming an embryoid body by culturing cells in a non-adhesive state on a culture dish, and is not particularly limited. It can be performed using a culture dish free from an artificial treatment to improve adhesiveness to cells (e.g., coating treatment with an extracellular substrate or the like) or a culture dish that has been artificially treated to inhibit adhesion (e.g., coating treatment with polyhydroxyethylmethacrylic acid (poly-HEMA)).

In the present specification, adhesive culture can be performed by culturing on feeder cells or using a culture vessel coating treated with an extracellular substrate. The coating treatment can be performed by placing a solution containing an extracellular substrate in a culture vessel and thereafter removing the solution as appropriate.

In the present specification, the feeder cell means other cell that plays an auxiliary role and is used for adjusting the culture conditions of the target cell. In the present invention, the extracellular substrate is a supramolecular structure existing outside the cell and may be naturally derived or an artifact (recombinant). For example, substances such as vitronectin, collagen, proteoglycan, fibronectin, hyaluronic acid, tenascin, entactin, elastin, fibrillin, laminin and fragments thereof can be mentioned, and vitronectin or a fragment thereof is preferable.

PSC produced as described above, preferably hPSC, can be maintained, for example, by adhesive culture using a culture vessel coated with an extracellular substrate. The culture medium used in the step of culturing PSC can be prepared using a medium used for culturing animal cells as a basal medium. Examples of the basal medium include IMDM medium, Medium 199, Eagle's Minimum Essential Medium (EMEM), αMEM medium, Dulbecco's modified Eagle's Medium (DMEM), Ham's F12 medium, RPMI 1640 medium, Fischer's medium and a mixed medium of these and the like. As a commercially available PSC maintenance medium, for example, the aforementioned Essential 8 medium (Es8, Thermo Fisher Scientific), SPM (Stem-Partner (registered trade mark) Human iPS/ES cells medium, KYOKUTO PHARMACEUTICAL INDUSTRIAL CO., LTD.), ReproFF2 medium (RFF2, REPROCELL), StemPro34 (invitrogen), RPMI-base medium, mTeSR1 (STEMCELL Technologies) and the like can also be used. In this step, Es8, SPM and the like are preferably used as a maintenance medium to predict and select a PSC clone that shows a differentiation potential in response to a differentiation stimulus. When cultured in a medium that confers differentiation resistance to PSC, such as RFF2 medium, PSC loses the ability to differentiate in response to differentiation stimulus. However, such differentiation resistance is not an inherent property of PSC clone, and differentiation potential may sometimes be reversibly recovered by changing the culture conditions. Therefore, when verifying the quality of PSC clone (whether differentiation resistance is an inherent property of the clone or reversible depending on the culture conditions), it is efficient to culture PSC with a medium that has been confirmed to maintain the differentiation potential of PSC in the present invention, such as Es8, SPM and the like. The medium may contain serum or may be serum-free. Where necessary, as long as the differentiation potential is not affected, the medium may contain one or more serum replacements such as albumin, transferrin, Knockout Serum Replacement (KSR) (serum substitute for FBS during ES cell culture), N2 supplement (Invitrogen), B27 supplement (Invitrogen), fatty acid, insulin, collagen precursor, trace element, 2-mercaptoethanol (2 ME), thiolglycerol and the like, and may also contain one or more substances such as lipid, amino acid, L-glutamine, Glutamax (Invitrogen), non-essential amino acid, vitamin, growth factor, low-molecular-weight compound, antibiotic, antioxidant, pyruvic acid, buffering agent, inorganic salts and the like.

In the step of culturing PSC, PSC may be cultured in single cells. For example, PSC is made into single cells by pipetting, trypsin treatment, and the like (to be also referred to as "single cell suspension"), seeded on a dish coated with VTN-N, and cultured feeder cell-free, whereby PSC can be cultured in single cells. In the step of culturing PSC, PSC may be cultured in a small cell clump. For example, PSC can be cultured in a small cell clump by culturing PSC on feeder cells (to be also referred to as "Small Cell Clumps method").

In the step of culturing PSC, the culture temperature is not limited to the following, and is, for example, about 30 - 40°C, preferably about 37°C, and the culture is performed in an atmosphere of $CO_2$-containing air. The $CO_2$ concentration is about 2 - 10%, preferably 5%.

In the step of culturing PSC, the medium can be changed during the culture period. The medium used for the medium change may be a medium having the same components as the medium before the medium change or a medium having different components. Preferably, a medium having the same components is used. The timing of the medium change is not particularly limited, but may be performed, for example, every day, every two days, every three days, every four days or every five days after starting culture with a fresh medium. In this step, the medium change is preferably performed every day.

Measurement of the expression level of CHD7 of hPSC can be performed using any RNA or protein measurement method known per se. For example, when measuring the expression of CHD7 at the RNA level, Northern hybridization, RT-PCR, digital PCR and the like can be performed using a nucleic acid (probe) that can hybridize with the CHD7 mRNA under stringent conditions, or an oligonucleotide set that can function as a primer that amplifies a part or all of the mRNA. The nucleic acid to be used as the probe may be DNA or RNA, or DNA/RNA chimera. Preferably, DNA is used. The nucleic acid may be double-stranded or single-stranded. In the case of double-stranded, it may be a double-stranded DNA, a double-stranded RNA or a DNA:RNA hybrid. The length of the nucleic acid is not particularly limited as long as it can specifically hybridize with the target mRNA, and is, for example, about 15 bases or more, preferably about 20 bases or more. The nucleic acid is preferably labeled with a labeling agent to enable detection and quantification of the target mRNA. As the labeling agent, for example, radioisotope, enzyme, fluorescent substance, luminescent substance, and the like are used. As the radioisotope, for example, [$^{32}P$], [$^{3}H$], [$^{14}C$] and the like are used. As the enzyme, a stable enzyme having high specific activity is preferable. For example, β-galactosidase, β-glucosidase, alkaline phosphatase, peroxidase, malate dehydrogenase and the like are used. As the fluorescent substance, for example, fluorescamine, fluorescein isothiocyanate and the like are used. As the luminescent substance, for example, luminol, luminol derivative, luciferin, lucigenin and the like are used. Furthermore, biotin-(strept)avidin can also be used for binding the probe and the labeling agent.

The oligonucleotide set to be used as a primer is not particularly limited as long as it can be annealed specifically to each of the base sequence of mRNA encoding CHD7 (sense strand) and a base sequence complementary thereto (antisense strand), and can amplify the DNA fragment sandwiched between them. For example, a set of oligonucleotides each designed to have a length of about 15 to about 100 bases, preferably about 15 to about 50 bases, and to amplify a DNA fragment of about 100 bp to several kbp can be mentioned. In particular, a primer set capable of specifically amplifying mRNA of CHD7 isoform 1 (NCBI database (GenBank accession number NM_017780.3) also referred to as CHD7L (base sequence is shown in SEQ ID NO: 1 and amino acid sequence is shown in SEQ ID NO: 2) and capable of annealing to a base sequence of mRNA encoding the amino acids 672 to 2620 of CHD7 isoform 1 and a base sequence complementary thereto can be preferably used.

To quantitatively analyze the gene expression of CHD7 by using a trace amount of RNA sample, it is preferable to use competitive RT-PCR, real-time RT-PCR or digital PCR analysis. When competitive RT-PCR is used, a nucleic acid that affords an amplification product that is amplified by the above-mentioned primer set and can be distinguished from the target DNA (for example, an amplification product having a different size from the target DNA, an amplification product showing a different migration pattern by a restriction enzyme treatment and the like) can be further contained in addition to the primer set. The competitor nucleic acid may be DNA or RNA. In the case of DNA, PCR may be performed by adding a competitor after synthesizing cDNA from an RNA sample by a reverse transcription reaction. In the case of RNA, RT-PCR can be performed by adding a competitor to an RNA sample from the beginning. In the latter case, the efficiency of the reverse transcription reaction is also taken into consideration, and thus the absolute amount of the original mRNA can be estimated.

On the other hand, since real-time RT-PCR can monitor the amount of PCR amplification in real time, electrophoresis is not necessary and CHD7 gene expression can be analyzed more quickly. Generally, monitoring is performed using various fluorescent reagents. Among these are reagents that emit fluorescence by binding to double-stranded DNA (intercalators) such as SYBR Green I, ethidium bromide and the like, a nucleic acid that can be used as the above-mentioned probe (note that, the nucleic acid hybridizes to the target nucleic acid in the amplification region), and has both ends modified with a fluorescent substance (e.g., FAM, HEX, TET, FITC etc.) and a quenching substance (e.g., TAMRA, DABCYL etc.), and the like.

Digital PCR analysis is an analysis method that absolutely measures the expression level of a target gene in a sample by synthesizing cDNA from the extracted mRNA, diluting and dispersing the cDNA in the water droplets of ultra-fine compartment or water-in-oil (W/O) emulsion so that 1 or 0 cDNA will be contained, performing PCR amplification, and directly measuring the expression level of the target gene in the sample by directly counting the number of micro-compartments with positive amplification signals and the number of water droplets. This method can be used particularly preferably. For digital PCR analysis, a commercially available digital PCR analyzer can be used, the QuantStudio 3D digital PCR system (trade name of Thermo Fisher Scientific), BioMark HD (trade name of Fluidigm) and the products of Bio-Rad Laboratories adopting Droplet Digital PCR method, and the like can be used, and analysis can be performed according to the instruction manuals and protocols of each device.

The nucleic acid used as the above-mentioned probe may be cDNA encoding CHD7 or a fragment thereof, or may be obtained by chemical synthesis using a commercially available DNA/RNA automatic synthesizer and the like, based on the base sequence information (e.g., in the case of human CHD7, reference can be made to the base sequence registered under accession number NM_017780.3 (SEQ ID NO: 1) in the NCBI database (GenBank)). The oligonucleotide set to be used as the above-mentioned primer can be obtained by chemically synthesizing the base sequence and a part of the complementary chain sequence thereof based on the above-mentioned base sequence information and using a commercially available DNA/RNA automatic synthesizer or the like.

On the other hand, when the expression of CHD7 is measured at the protein level, it can be performed using various immunological methods, for example, various immunoassays such as Western blotting, ELISA, RIA, FIA and the like by using an anti-CHD7 antibody. The anti-CHD7 antibody to be used may be either a polyclonal antibody or a monoclonal antibody, may be prepared by a well-known immunological method, or a commercially available antibody may also be used. The antibody includes not only a complete antibody molecule but also a fragment thereof, and examples thereof include Fab, F(ab')2, ScFv, minibody and the like. For the detail of the above-mentioned immunological measurement methods, reference can be made to, for example, Hiroshi Irie ed., "Radioimmunoassay" (Kodansha, published 1974), Hiroshi Irie ed., "Supplementary volume of Radioimmunoassay" (Kodansha, published 1979), Eiji Ishikawa et al. ed., "enzyme immunoassay" (Igaku-Shoin, published 1978), Eiji Ishikawa et al. ed., "enzyme immunoassay" (2nd edition) (Igaku-Shoin, published 1979), Eiji Ishikawa et al. ed., "enzyme immunoassay" (third ed.) (Igaku-Shoin, published 1987), "Methods in ENZYMOLOGY" Vol. 70 (Immunochemical Techniques (Part A)), ibidem Vol. 73 (Immunochemical Techniques (Part B)), ibidem Vol. 74 (Immunochemical Techniques (Part C)), ibidem Vol. 84 (Immunochemical Techniques (Part D: Selected Immunoassays)), ibidem Vol. 92 (Immunochemical Techniques (Part E: Monoclonal Antibodies and General Immunoassay Methods)), ibidem Vol. 121 (Immunochemical Techniques (Part I: Hybridoma Technology and Monoclonal Antibodies)) (all above published by Academic Press) and the like.

When the expression of CHD7 is measured at the protein level by sandwich ELISA, the selection of the combination of capture antibody and detection antibody is not particularly limited as long as CHD7 can be detected. Specifically, for example, a monoclonal antibody (mouse IgG1) is obtained using an antigen, a polypeptide in which Ala 263-Gln 457 of human CHD7 (SEQ ID NO: 2) are expressed in Escherichia coli, purifying with Protein A or Protein G, and can be used as a capture antibody. Specifically, for example, anti-human CHD7 rabbit IgG is obtained by immunizing a rabbit with Gly 25-Met 200 of human CHD7 (SEQ ID NO: 2) expressed in Escherichia coli, affinity purified using the antigen, and can be used as a primary antibody.

For the measurement of the expression level of CHD7, the expression level of isoform of CHD7 can be measured. Examples of the isoform include isoform 1 (NCBI database (GenBank accession number NM_017780.3) (base sequence is shown in SEQ ID NO: 1 and amino acid sequence is shown in SEQ ID NO: 2)), isoform 2 (i.q., accession number NM_001316690.1 (base sequence is shown in SEQ ID NO: 3 and amino acid sequence is shown in SEQ ID NO: 4)), isoform X4 (i.q., accession number XM_011517560.2 (base sequence is shown in SEQ ID NO: 5 and amino acid sequence is shown in SEQ ID NO: 6)) and the like. Preferred is isoform 1.

Using any of the above-mentioned methods or other method known per se, the expression level of CHD7 of PSC is measured, and the measurement value is compared with the threshold value of CHD7 expression level necessary for PSC to show a differentiation potential in response to a differentiation stimulus. The threshold value varies depending on the measurement method of the CHD7 expression level to be used. When, for example, measuring the expression level of CHD7 gene by quantitative digital PCR analysis is, for example, not less than 2710 copies in 5 ng of the total RNA. When cultured in single cells without feeder cell on a dish coated with extracellular substrate, it is unlimitatively, for example, not less than 1502 or not less than 1500 copies in 5 ng of the total RNA. When hPSC is ESC and cultured with feeder cells by the Small Cell Clumps method, it is unlimitatively, for example, not less than 2710 copies or not less than 2120 copies; when cultured in single cells without feeder cellon a dish coated with extracellular substrate, it is unlimitatively, for example, not less than 3280 copies; when hPSC is iPSC and cultured with feeder cells by the Small Cell Clumps method, it is unlimitatively, for example, not less than 3080 copies or not less than 2280 copies; and when cultured in single cells without feeder cell on a dish coated with extracellular substrate, it is unlimitatively, for example, not less than 1502 copies or not less than 1500 copies.

When the expression level of CHD7 protein is measured, the threshold value is, for example, not less than 2.0 times, not less than 3.0 times, not less than 4.0 times, not less than 5.0 times, not less than 6.0 times, not less than 7.0 times, not less than 7.7 times, not less than 8.0 times, not less than 8.5 times, not less than 9.0 times, not less than 9.2 times, not less than 10 times, not less than 10.2 times or not less than 10.3 times, compared to CHD7 protein concentration of human pluripotent stem cell showing differentiation resistance. Examples of the human pluripotent stem cell showing differentiation resistance include human pluripotent stem cell cultured for not less than 5 passages with ReproFF2 medium. Furthermore, when similar to the above-mentioned measurement, the threshold value is, for example, not more than 90.3%, not more than 90.2%, not more than 90%, not more than 89.1%, not more than 88.3%, not more than 87.0%, not more than 85.8%, not more than 85%, not more than 83.4%, not more than 80.2%, not more than 80%, not more than 75%, not more than 70%, not more than 50%, compared to CHD7 protein concentration of human pluripotent stem cell showing normal differentiation potential. Examples of the human pluripotent stem cell showing normal differentiation potential include human pluripotent stem cell cultured for not less than 5 passages with Es8 or SPM medium.

For example, the threshold value may be determined in consideration of the expression level of CHD7 gene measured by quantitative digital PCR analysis. Examples of the threshold value in consideration of the expression level of CHD7 gene may be not less than 2 times, not less than 3 times, not less than 4 times, not less than 5 times and not less than 10 times, compared to CHD7 protein concentration of human pluripotent stem cell showing differentiation resistance. Examples of the human pluripotent stem cell showing differentiation resistance may be human pluripotent stem cell cultured for not less than 5 passages with ReproFF2 medium. Furthermore, when similar to the above-mentioned case, the threshold value is, for example, not more than 50%, not more than 70%, not more than 75%, not more than 80% or not more than 90%, compared to CHD7 protein concentration of human pluripotent stem cell showing normal differentiation potential. Examples of the human pluripotent stem cell showing normal differentiation potential may be human pluripotent stem cell cultured for not less than 5 passages with Es8 or SPM medium.

The CHD7 protein concentration may be a value obtained by directly measuring the CHD7 protein concentration of the lysate of the cultured cells, or may be a value relative to the lysate of the cultured cells as the standard. The cultured cell may be a stem cell showing differentiation resistance or a stem cell showing normal differentiation potential, and the lysate may or may not be concentrated.

Even when other measurement method is used, PSC clone (e.g., hESC clones such as H1, H9, KhES1 and the like) confirmed by EB formation assay or cytokine-induced differentiation assay to show differentiation potential by culturing with Es8 or SPM medium is cultured with Es8 or SPM, specifically, for example, when cultured in single cells without feeder cell on a dish coated with extracellular substrate, the expression level of CHD7 when preferably cultured for not less than 5 passages is measured by plural experiments and, for example, the maximum value or mean of the measurement value can be determined as a threshold value.

As a result of comparison, when the expression level of CHD7 is not less than the threshold value of CHD7 expression level necessary for PSC to show a differentiation potential in response to a differentiation stimulus, the PSC shows a differentiation potential in response to a differentiation stimulus. Therefore, it can be predicted that there is no risk of remaining undifferentiated cells (which cause tumorigenesis) when differentiation is induced or that the cells have a low risk. Conversely, if the expression level of CHD7 is less than the threshold value, the PSC shows differentiation resistance to differentiation stimulus. Therefore, when differentiation is induced, it can be predicted that there is a risk of remaining undifferentiated cells (which cause tumorigenesis) or that the cells have a high risk. As a result of the prediction, if the risk remains or the cells have a high risk, the differentiation resistance can be reduced or eliminated by introducing a nucleic acid encoding CHD7 or changing the medium with one confirmed to be able to maintain the differentiation potential of PSC (e.g., Es8, SPM) and culturing for at least 5 passages. In addition, as a result of the prediction, if iPSC cultured together with feeder cells has a risk or the cells have a high risk, differentiation resistance can be reduced or eliminated by culturing in single cells without feeder cell.

As described above, whether or not PSC shows differentiation potential in response to differentiation stimulus is not necessarily an inherent property of PSC clone, but rather, highly probably alters depending on the PSC maintenance culture conditions. That is, even if the PSC clone is the same, when the culture conditions are different, it may differentiate in response to a differentiation stimulus (e.g., when cultured with Es8 or SPM), or it may often show differentiation resistance (when cultured with RFF2).

Therefore, the present invention also provides a method for evaluating a medium for a human pluripotent stem cell comprising measuring an expression level of CHD7 of PSC (hereinafter to be also referred to as the "evaluation method of the present invention"). Examples of the test medium include various media that can be used for maintenance culture of PSC prior to the step of measuring the above-mentioned expression level of CHD7.

As described in the below-mentioned Examples, 5 passages (about 15 days) are preferable for completely changing the epigenetic state of CHD7 gene in PSC by changing the medium to a different medium. Therefore, in the evaluation method of the present invention, PSC is preferably cultured with a test medium for 5 passages or more prior to the step of measuring the expression level of CHD7. The culture of PSC with the test medium can be performed by a method similar to that in the above-mentioned prediction method of the present invention.

The measurement of the expression level of CHD7 in the above-mentioned evaluation method of the present invention can also be performed by a method similar to that in the above-mentioned prediction method of the present invention. The obtained measurement value is compared with the threshold value of the CHD7 expression level which is necessary for PSC to show differentiation potential in response to a differentiation stimulus in the above-mentioned prediction method of the present invention. When, for example, measuring the expression level of CHD7 gene by quantitative digital PCR analysis, the threshold value is, for example, not less than 2710 copies in 5 ng of the total RNA. When cultured in single cells without feeder cell on a dish coated with extracellular substrate, it is unlimitatively, for example, not less than 1502 or not less than 1500 copies in 5 ng of the total RNA. When hPSC is ESC and cultured with feeder cells by the Small Cell Clumps method, it is unlimitatively, for example, not less than 2710 copies or not less than 2120 copies; when cultured in single cells without feeder cell on a dish coated with extracellular substrate, it is unlimitatively, for example, not less than 3280 copies; when hPSC is iPSC and cultured with feeder cells by the Small Cell Clumps method, it is unlimitatively, for example, not less than 3080 copies or not less than 2280 copies; and when cultured in single cells without feeder cell on a dish coated with extracellular substrate, it is unlimitatively, for example, not less than 1502 copies or not less than 1500 copies.

When the expression level of CHD7 protein is measured, the threshold value is, for example, not less than 2.0 times, not less than 3.0 times, not less than 4.0 times, not less than 5.0 times, not less than 6.0 times, not less than 7.0 times, not less than 7.7 times, not less than 8.0 times, not less than 8.5 times, not less than 9.0 times, not less than 9.2 times, not less than 10 times, not less than 10.2 times or not less than 10.3 times, compared to CHD7 protein concentration of human pluripotent stem cell showing differentiation resistance. Examples of the human pluripotent stem cell showing differentiation resistance include human pluripotent stem cell cultured for not less than 5 passages with ReproFF2 medium. Furthermore, when similar to the above-mentioned measurement, the threshold value is, for example, not more than 90.3%, not more than 90.2%, not more than 90%, not more than 89.1%, not more than 88.3%, not more than 87.0%, not more than 85.8%, not more than 85%, not more than 83.4%, not more than 80.2%, not more than 80%, not more than 75%, not more than 70%, not more than 50%, compared to CHD7 protein concentration of human pluripotent stem cell showing normal differentiation potential. Examples of the human pluripotent stem cell showing normal differentiation potential include human pluripotent stem cell cultured for not less than 5 passages with Es8 or SPM medium.

In addition, the threshold value may be determined, for example, in consideration of the expression level of CHD7 gene measured by quantitative digital PCR analysis. The threshold value in consideration of the expression level of CHD7 gene is, for example, not less than 2 times, not less than 3 times, not less than 4 times, not less than 5 times or not less than 10 times, compared to CHD7 protein concentration of human pluripotent stem cell showing differentiation resistance. Examples of the human pluripotent stem cell showing differentiation resistance include human pluripotent stem cell cultured for not less than 5 passages with ReproFF2 medium. Furthermore, when similar to the above-mentioned case, the threshold value is, for example, not more than 50%, not more than 70%, not more than 75%, not more than 80%, not more than 90%, compared to CHD7 protein concentration of human pluripotent stem cell showing normal differentiation potential. Examples of the human pluripotent stem cell showing normal differentiation potential include human pluripotent stem cell cultured for not less than 5 passages with Es8 or SPM medium.

The CHD7 protein concentration may be a value obtained by directly measuring the CHD7 protein concentration of the lysate of the cultured cells, or may be a value relative to the lysate of the cultured cells as the standard. The cultured cell may be a stem cell showing differentiation resistance or a stem cell showing normal differentiation potential, and the lysate may or may not be concentrated.

As a result of comparison, when the expression level of CHD7 is not less than the threshold value of CHD7 expression level necessary for hPSC to show a differentiation potential in response to a differentiation stimulus, the test medium can perform maintenance culture of PSC so that PSC shows a differentiation potential in response to a differentiation stimulus. Therefore, when differentiation is induced, it can be evaluated that there is no risk of leaving undifferentiated cells (which cause tumorigenesis) or that the medium has a low risk. Conversely, if the expression level of CHD7 is less than the threshold value, the test medium performs maintenance culture of PSC so that PSC shows a differentiation potential in response to a differentiation stimulus. Therefore, when differentiation is induced, it can be evaluated that there is a risk of leaving undifferentiated cells (which cause tumorigenesis) or that the medium has a high risk.

When maintenance culture of PSC is performed using the medium evaluated in the evaluation method of the present invention that the medium performs maintenance culture of PSC such that PSC shows a differentiation potential in response to a differentiation stimulus, the medium is changed to a medium evaluated by the same evaluation method to be able to perform maintenance culture of PSC such that PSC shows a differentiation potential in response to a differentiation stimulus, and PSC is cultured, preferably not less than 5 passages, whereby the PSC can be altered to show a differentiation potential in response to a differentiation stimulus.

As demonstrated in the below-mentioned Examples, spontaneous differentiation can be induced in PSC without applying a differentiation stimulus by introducing a nucleic acid encoding CHD7 into PSC instead of changing the medium with a medium capable of maintenance culture of PSC such that PSC shows a differentiation potential in response to a differentiation stimulus. For example, after transplanting hPSC-derived differentiation cell population, when an undifferentiated cell remains in the graft cells, the risk of tumorigenesis may be reduced by injecting a nucleic acid (mRNA, plasmid DNA etc.) encoding CHD7 into the transplantation site and introducing the nucleic acid into the remaining undifferentiated cells, thus causing spontaneous differentiation regardless of the environment of the transplantation site.

Therefore, the present invention also provides a differentiation-inducing factor for PSC, particularly hPSC, containing a nucleic acid encoding CHD7, and a method for inducing differentiation of PSC including introducing the nucleic acid into PSC showing differentiation resistance.

The present invention also provides a reagent for use in the above-mentioned prediction method of the present invention and the evaluation method of the present invention. The reagent contains a substance capable of detecting the expression of CHD7. Such substance is not particularly limited. For example, when the expression of CHD7 is detected at the RNA level, the nucleic acid and the like exemplified as the probe or primer in the above-mentioned prediction method of the present invention can be recited. When the expression of CHD7 is detected at the protein level, anti-CHD7 antibody and the like can be mentioned. The reagents can also be provided as a kit further containing various reagents necessary for carrying out the various methods for measuring the aforementioned expression level of CHD7.

While the present invention is further specifically explained in the following by showing Examples, the present invention is not at all limited by these Examples.

[Example]

Material and method

In the present specification, all the experiments conducted in this study used human ESCs and human iPSCs. The study was approved by the ethical committee of the Foundation for Biomedical Research and Innovation at Kobe (FBRI).

(Cell culture)

Any of human ESC lines KhES-1 (Riken BRC) (Navarro-Alvarez et al., Cell Transplant 2008; 17(1-2): 111-119) and H9 (WiCell Research Institute, Levenstein et al., Stem Cell; 24(3):568-74, 2006 Mar.), and the human iPSC lines PFX#9 (Nishishita et al. PLoS One. 2012; 7(6): e38389), 201B7 (Riken RBC) and SHh#2 (Nishishita et al., PLoS One. 2012; 7(6)) were cultured with hPSCs culture media (Takenaka et al., PLoS ONE 2015; 10(6): e0129855) on mitomycin C-treated SNL76/7 cells (SIM strain embryonic fibroblast, ECACC; European Collection of Authenticated Cell Culture) or with Essential 8 medium (Es8, Thermo Fisher Scientific), (Chen et al., Nat Methods 2011; 8(5): 424-429), SPM (StemPartner (registered trade mark) Human iPS/ES cells medium) (Takenaka et al., PLoS ONE 2015; 10(6): e0129855) (Kyokuto Pharmaceutical Co. Ltd.) or RiproFF2 medium (RFF2, ReproCELL) on rhVitronectin-N (recombinant human Vitronectin-N) (Thermo Fisher Scientific)-coated dishes. Cells were passaged in clumps using Gentle Cell Dissociation Reagent (GCDR; Stem Cell Technologies) and split at a ratio of 1:3 for iPSCs or 1:3.5 for ESCs. Alternatively, cells were passaged by seeding a single-cell suspension using TrypLE Select (Life Technologies) (Takenaka et al., PLoS ONE 2015; 10(6): e0129855). Cells in single-cell suspensions were seeded at $3 \times 10^5$ cells/well in 6-well plates when cultured with Es8 medium and $1 \times 10^5$ cells/well with RFF2 medium. Cells were cultured in an atmosphere containing 5% $CO_2$ in an incubator (MCO-19AIC; Panasonic) at 37°C. Karyotypes of KhES-1, H9, and PFX#9 cells were examined by multicolor fluorescence in situ hybridization (mFISH) every 5 passages and by G-banding every 10 passages; PSCs with a normal karyotype were used in this study.

(siRNA reagents and transfection)

All reagents were purchased from Thermo Fisher Scientific, unless otherwise specified. Silencer Select Predesigned human CHD7 siRNA (catalog No. 4392420; ID: s31142) and Silencer Select Negative Control No. 1 (catalog No. 4404021) were used for siCHD7 or control siRNA (mock) transfection experiments, respectively. Cells were transfected as follows.

ESCs were seeded at $2\times10^5$ cells/well on VTN-N-coated 6-well plates and cultured with 4 mL of Es8, SPM or RFF2 medium. The following day, the medium was changed, and siCHD7 or control siRNA was transfected into the cells with Lipofectamine RNAiMAX in accordance with the manufacturer's instructions (transfection amount 50 pmol, 30 pmol or 10 pmol). Briefly with regard to introduction of 50 pmol siCHD7, cocktail A (4 μL Lipofectamine RNAiMAX Reagent and 150 μL Opti-MEM Medium) was mixed with cocktail B (1 μL of 50 μM siCHD7 (50 pmol) or control siRNA (50 pmol) and 150 μL Opti-MEM Medium), and the mixture was then incubated for 5 min at room temperature. The mixed cocktail (240 μL) was used for transfection of ESCs with siCHD7 (final concentration 10 nM) or control siRNA (final concentration 10 nM), and cells were incubated for 48 h. The transfection efficiency of the reagent was assessed by qRT-PCT for detection of CHD7 mRNA in the transfected cells at the designated time points.

(Synthetic mRNA reagents and transfection)

The T7 promoter and T7 terminator were fused into the 5' and 3' coding DNA sequences for CHD7 isoform 2 (NM_001316690.1 (SEQ ID NO: 3)), respectively and cloned into the pMX vector. Synthetic mRNA for CHD7 isoform 2 was then generated using an mMESSAGE mMACHINE T7 ULTRA Transcription Kit after digesting the pMX vector with SfiI. Synthetic mRNA covering both chromodomain and SNF2-like ATPase/helicase domain (CHD7 DN1) was generated by the same manner. The region of the SANT-SLIDE domain in CHD7 was determined based on the homology with the published CHD1 sequence (Ryan et al., Embo j 2011; 30(13):2596-2609), and synthetic mRNA (CHD7 DN2) was generated in the same manner. The quantity of the resulting mRNAs was measured with an ND-1000 (Nano Drop). mRNA for enhanced green fluorescent protein (eGFP) obtained from the same vector backbone was used as the control (control mRNA, mock).

(mRNA transfection)

ESCs ($3 \times 10^5$) were seeded in each well of a VTN-N-coated 6-well plate with RFF2 supplemented with 5 ng/mL fibroblast growth factor 2 (FGF2; Peprotech). mCHD7 or control mRNA (mock) was transfected into the cells with Lipofectamine Messenger MAX in accordance with the manufacturer's instructions. Briefly, cocktail A (3.75 μL Lipofectamine Messenger MAX transfection reagent and 125 μL Opti-MEM Medium) was incubated for 10 min at room temperature. Then, cocktail B (2.5 μg mCHD7 or control mRNA and 125 μL Opti-MEM Medium) was prepared and mixed with cocktail A, followed by incubated for 5 min at room temperature. The mixed cocktail (240 μL) was added to 4 mL of RFF2 supplemented with FGF2 to a final concentration of 5 ng/mL, and the cells were cultured with this medium for 24 h at 37°C. The transfection efficiency of the reagent was assessed by determining the expression of CHD7 mRNA by qRT-PCR in the transfected cells at the designated time points.

(Methylation and GeneChip analysis)

The methylation state of cultured ESCs or iPSCs was determined with the Infinium HumanMethylation450 BeadChip (Illumina). The methylation pattern in the promoter region was hierarchically clustered using Cluster 3.0 and visualized in Java Tree View. The methylation status of the respective genes in the promoter regions was assessed by a comparison with the gene expression signal obtained by GeneChip (Human Genome U133 Plus 2.0 Array; Affymetrix) array data to extract the candidate genes.

(EB formation assays after transfection with siCHD7)

Cells cultured on VTN-N-coated wells with 4 mL of Es8 medium were washed once with PBS (-) 48 h after transfection with either siCHD7 (50 pmol) or control siRNA (mock). Cells were then scraped with a cell scraper (Iwaki), dissociated by pipetting, transferred into a low-attachment 6-well plate (Corning), and cultured with Essential 6 medium (Es6) with 10 mM ROCK inhibitor (Y-27632, Wako) for 1 day and with Es6 medium alone for 13 days for EB formation. Medium was changed every 2 days. The numbers and morphologies of EBs were observed microscopically (Olympus IX71; Olympus). Gene expression for CHD7 was determined by qRT-PCR and gene expression profiles were determined by TaqMan (registered trade mark) Scorecard Panel (A15870) using a qRT-PCR device (QuantStudio 12 K Flex). Primer sequences for CHD7 are shown in Table 1.

[Table 1-1]

primer

| Gene | F/ R | seq (5'-3') | SEQ ID NO |
|---|---|---|---|
| POU5F1 | F | GAA ACC CAC ACT GCA GCA GA | SEQ ID NO:7 |
| POU5F1 | R | TCG CTT GCC CTT CTG GCG | SEQ ID NO:8 |
| SOX2 | F | GGG AAA TGG GAG GGG TGC AAA AGA GG | SEQ ID NO:9 |
| SOX2 | R | TTG CGT GAG TGT GGA TGG GAT TGG TG | SEQ ID NO:10 |
| NANOG | F | CTC AGC TAC AAA CAG GTG AAG AC | SEQ ID NO:11 |
| NANOG | R | TCC CTG GTG GTA GGA AGA GTA AA | SEQ ID NO:12 |
| PE300 | F | TTG AAT GTA CAG AGT GCG GAA GA | SEQ ID NO:13 |
| PE300 | R | AAC AGC CAT CAC AGA CGA ATC C | SEQ ID NO:14 |
| CHD7 | F | GGT TCC CAC ACT CGT GCA TA | SEQ ID NO:15 |
| CHD7 | R | TGC GCC TCG GGA CAG A | SEQ ID NO:16 |
| CHD7 isoform 2 | F | CCC ATG AAA GCA ATG AGT AAT CC | SEQ ID NO:17 |
| CHD7 isoform 2 | R | TCC ATT GGT ATC CCA GCA CTT C | SEQ ID NO:18 |
| CHD7 Chromodomain | F | TGA TGG ACT TGG AAC ACA AAG TG | SEQ ID NO:19 |
| CHD7 Chromodomain | R | TGA AGG GAA GCG ACT TGG TT | SEQ ID NO:20 |
| CHD7 sant slide | F | CAA ACA TGG CTA TGA GAA GTA CAA CTC | SEQ ID NO:21 |
| CHD7 sant slide | R | CCG ACT CGT TCC AGA AAG CA | SEQ ID NO:22 |
| GAPDH | F | CCA CTC CTC CAC CTT TGA CG | SEQ ID NO:23 |
| GAPDH | R | ATG AGG TCC ACC ACC CTG TT | SEQ ID NO:24 |

[Table 1-2]

Taq man PCR primer set 1

| Refseq No. | Gene | Taqman probe |
|---|---|---|
| NM_017780<br>XM_011517553.2<br>XM_011517555.2<br>XM_017013613.1<br>XM_011517560<br>XM_011517554.2 | CHD7 | Hs00215010_m1 |

[Table 1-3]

Taq man PCR primer set 2

| Refseq No. | Gene | P/F/R | seq (5'-3') | SEQ ID NO |
|---|---|---|---|---|
| NM_001316690 | CHD7 | P | TATGACTCAGAAACCGAAACAGAAACGACA | SEQ ID NO:25 |
| | CHD7 | F | GCCCTTTCTAGAGAAACCAGTG | SEQ ID NO:26 |
| | CHD7 | R | AGGCACCCTTTCTTCTCCTG | SEQ ID NO:27 |

[Table 1-4]

Taq man PCR primer set 3

| Refseq No. | Gene | P/F/R | seq (5'-3') | SEQ ID NO |
|---|---|---|---|---|
| NM_017780<br>XM_011517553.2<br>XM_011517555.2<br>XM_017013613.1<br>XM_011517554.2 | CHD7 | P | CACGGACGCTATAAACGCCAACTCACTG | SEQ ID NO:28 |
| | CHD7 | F | GAATCTGCTTGTCTATGGTTGGG | SEQ ID NO:29 |
| | CHD7 | R | AGGATGGTTCTGCAGATGGT | SEQ ID NO:30 |

(EB formation assays after transfection with mCHD7)

Cells cultured on VTN-N-coated wells and RFF2 supplemented with FGF2 to a final concentration of 5 ng/mL were washed once with PBS (-) 24 h after introduction of either mCHD7 or control mRNA. Then the cells were scraped with a cell scraper (Iwaki), dissociated by pipetting, transferred into a low-attachment 6-well plate (Corning), cultured with RFF2 containing 10 mM ROCK inhibitor (Y-27632, Wako) and not containing FGF2 for 1 day, and cultured with RFF2 not containing FGF2 to form EB while changing the medium every day. The number and morphology of EBs were observed microscopically (Olympus IX71, Olympus). Gene expression for CHD7 was determined by qRT-PCR and gene expression profiles were determined by TaqMan (registered trade mark) Scorecard Panel (A15870) using a qRT-PCR device (QuantStudio 12 K Flex).

(Quantitative RT-PCR)

According to the manufacturer's instructions, total RNA was extracted using the RNeasy micro kit (74004, QIAGEN). To synthesize cDNA by using the QuantiTect Reverse Transcription Kit (205311, QIAGEN), 1 $\mu$g of total RNA was used. Using TaqMan (registered trade mark) hPSC Scorecard Panel (A15870), quantitative PCR (qPCR) for profiling gene expression relating to the three germ layers and self-proliferation was performed. Using SYBR (registered trade mark) Select Master Mix and StepOnePlus, cDNA was synthesized from 500 ng of total RNA by reverse transcription reaction. The reaction was carried out under the conditions of 95°C for 10 min, and 40 cycles at 95°C for 15 sec, at 60°C for 1 min and at 72°C for 15 sec. The primers used are listed in Table 1. Relative quantification was calculated using the $2^{-\Delta\Delta Ct}$ method after normalization with glyceraldehyde-3-phosphate dehydrogenase (GAPDH).

(Determination of copy number of CHD7 transcript)

The copy number of the CHD7 transcript was determined by a Droplet Digital PCR system (Bio-Rad Laboratories). Specifically, cDNA was generated from 5 ng of total RNA extracted from KhES-1 cultured with Es8 or RFF2 using a probe of TaqMan (registered trade mark) Gene Expression Assay (Hs00215010_m1, Thermo Fisher Scientific). An emulsion of the RT-PCR reaction mixture was generated using a QX100 system (Bio-Rad Laboratories) according to the instruction manual. Thereafter, cDNA was respectively amplified from Es8 culture and RFF2 culture using an Applied Biosystems GeneAmp 9700 Thermalcycler. Each reaction mixture was composed of a 20 μL solution containing 10 μL of ddPCR probe Supermix, 1000 nM primer, 250 nM probe and template cDNA. The reaction was performed under the conditions of a treatment at 95°C for 10 min, after which 40 cycles of denaturation at 94°C for 30 sec and elongation reaction at 53°C for 60 sec, and finally, at 98°C for 10 min. After the reaction, raw fluorescence data of each well was analyzed with software QuantaSoft ver. 1.2 (Bio-Rad Laboratories).

(Western Blotting for detection of CHD7)

Cell lysates for Western blotting were prepared 72 h after seeding. Proteins were extracted using Complete Lysis-M (Roche), supplemented with protease inhibitor cocktail tablets (Complete Mini; Roche). Polyclonal sheep IgG anti-CHD7 antibody (AF7350; R&D Systems) and rabbit anti-sheep IgG (H + L) antibody labeled with horseradish peroxidase were respectively used as primary antibody and secondary antibody. The signal was detected with Chemi-Lumi One Super reagents (Nacalai Tesque). Total protein was measured with a bicinchoninic acid total protein assay kit (Nacalai Tesque) prior to application to the lane.

(Preparation of standard and sample for sandwich ELISA of CHD7)

ESC was cultured under various conditions. Specifically, H9 was cultured for 17 passages with Es8 (P1), KhES-1 was cultured for 10 passages with Es8 (P2), and KhES-1 was cultured for 11 passages with RFF2 (N). All cells were cultured in single cells on a VTN-N coated dish without feeder cell. Each cultured ESC was lysed according to the protocol of cOmplete Lysys-M (Merck KGaA, product number: 04719956001), the protein concentration was measured, dispensed by 500 μL, and rapidly frozen in liquid nitrogen and stored at -80°C until use. The total protein concentration of P1, P2 and N was 1.25 mg/mL, 1.27 mg/mL and 0.84 mg/mL respectively. H9 cells were cultured with Es8 or RFF2, lysed according to the protocol of cOmplete Lysys-M, then Amicon Ultra-4, PLTK Ultracel-PL membrane, 30 kDa (Amicon (registered trade mark) Ultra-4 PLTK Ultracel-PL membrane, 30 kDa, catalog number: UFC803024, Merck KGaA) were used to concentrate at 4000 X g, 4°C and the concentrated products were respectively used as a standard and a negative control (N2). The standard and the negative control (N2) were dispensed by 500 μL, rapidly frozen in liquid nitrogen, and stored at -80°C until use. The total protein concentration of the standard and the negative control (N2) was respectively 4.06 mg/mL and 10.23 mg/mL.

(Antibody for sandwich ELISA)

For Sandwich ELISA, as a capture (solid phase) antibody, an antibody obtained by purifying, with Protein A or Protein G, a monoclonal antibody (mouse IgG1) obtained using, as an antigen, a polypeptide in which Ala 263-Gln 457 of human CHD7 (SEQ ID NO: 2) are expressed in Escherichia coli was used; as a primary antibody, anti-human CHD7 rabbit IgG obtained by immunizing the rabbit with Gly 25-Met 200 of human CHD7 (SEQ ID NO: 2) expressed in Escherichia coli and affinity purifying the rabbit with the antigen was used; and as the secondary antibody, anti-rabbit IgG-HRP (SouthernBiotech, 4090-05) was used.

(Sandwich ELISA)

Sandwich ELISA was performed by the following method. To determine the optimal conditions for sandwich ELISA, the concentrations of the primary antibody and the secondary antibody were examined under the conditions shown in the following Table. 100 μL of the capture antibody solution diluted to 3 μg/mL with D-PBS(-) was added to the well of a 96-well plate (MaxiSorp (registered trade mark), Nunc, 44-2404-21), sealed with a plate seal, and stored at 4°C overnight. Thereafter, the capture antibody solution was removed by decantation, 200 μL of D-PBS(-) containing 0.05% Tween (registered trade mark) 20 (hereinafter to be also referred to as "washing solution") was added, and the mixture was gently stirred, allowed to stand for 5 min, removed and washed. This washing step was repeated 3 times. Blocking was performed by adding a blocking solution obtained by adding 1% BSA to the washing solution to the well after removal of the washing solution and incubating the mixture at room temperature for 1 hr. Then, the standard diluted with the blocking solution, the object sample, was added at 100 μL/well. To the Blank well was added the same amount of the blocking solution. A plate seal was adhered tightly to prevent evaporation of the solution and the mixture was incubated at 4°C overnight. Thereafter, the well was washed three times with the washing solution, 100 μL of the primary antibody diluted to 1 μg/mL or 3 μg/mL with the blocking solution was added, and the mixture was incubated at room temperature for 1 hr. Thereafter, the primary antibody was removed and the well was washed 3 times with the washing solution. Then, 100 μL of the secondary antibody diluted 5000-fold or 10000-fold was added to the well and the mixture was incubated at room temperature for 1 hr. Thereafter, the mixture was washed 3 times with the washing solution, 100 μL of the substrate solution (TMB, ScyTek Laboratories, TM4500) was added, and the mixture was incubated for 20-30 min in the dark and 0.5 M $H_2SO_4$ was added at 100 μL/well to stop the reaction. The absorbance at 450 nm (Abs 450 nm) and 650 nm (reference wavelength, Abs 650 nm) was measured with a microplate reader wherein the CHD7 concentration of the stock solution of the standard was used as 1000 units, not less than 8 points in the 2-fold diluted dilution series or not less than 6 points in the 3-fold diluted dilution series from 500 U/mL were produced and measured.

[Table 2]

| Condition | Capture antibody | First Antibody | Secondary Antibody |
|---|---|---|---|
| F1/S5k | 3 μg/mL | 1 μg/ml | 1/5000 dilution |
| F1/S10k | 3 μg/mL | 1 μg/mL | 1/10000 dilution |
| F3/S5k | 3 μg/mL | 3 μg/mL | 1/5000 dilution |
| F3/S10k | 3 μg/mL | 3 μg/mL | 1/10000 dilution |

(Calculation of CHD7 protein concentration)

The concentration of CHD7 was calculated by the following steps.

1) ΔAbs is calculated by the formula 1 from the measured absorbance:

$$\Delta Abs = [\![Abs]\!]\ 450\ nm - [\![Abs]\!]\ 650\ nm \cdots (1)$$

2) Blank is subtracted from ΔAbs of each sample by the next formula 2 to calculate ΔAbs-blk:

$$\Delta Abs\text{-}blk = [\![\Delta Abs]\!]\ Sample - [\![\Delta Abs]\!]\ Blank \cdots (2)$$

3) An analytical curve is drawn by the next formula 3 as a similar 4 parameter logistic curve:

$$f(x) = (a-d)/(1+(x/c)^b)+d \ldots (3)$$

f(x): ΔAbs-blk
x: CHD7 concentration (U/mL) after dilution
a: lower asymptote
b: inflection point inclination
c: inflection point
d: upper asymptote

4) ΔAbs-blk of the measurement sample is substituted into the equation of the analytical curve in the following formula 4 to calculate CHD7 concentration after dilution.

$$x = c \times \sqrt[b]{\frac{a-d}{f(x)-d} - 1} \cdots (4)$$

5) CHD7 concentration after dilution of the measurement sample is multiplied by dilution factor to calculate CHD7 concentration before dilution.

Results

1. Differentiation potential of ESCs alters by changing culture conditions:

When KhES-1 cells showing a normal karyotype were cultured with Essential 8 medium (Es8) on an hrVitronectin-N(VTN-N)-coated dish, the KhES-1 cells maintained both self-proliferation ability and differentiation potential. KhES-1 cells lost differentiation potential after culturing for 5 passages with ReproFF2 (RFF2), but recovered the differentiation potential after culturing with Es8 (Fig. 1). While KhES-1 cells cultured with SPM (Takenaka et al., PLoS ONE 2015; 10(6): e0129855) maintained differentiation potential, the cells lost differentiation potential after culturing for 5 passages with RFF2. Furthermore, when PFX#9 iPSC (Nishishita et al., PLoS One 2012; 7(6): e38389) showing normal karyotype was cultured with Es8 and then with RFF2 on a VTN-N-coated dish, the same results were obtained (Fig. 2). These results indicate that the differentiation potential of PSC can reversibly alter depending on the culture conditions. It was considered that alterations in the epigenetic status of the cell relate to these responses.

A comparison study of the methylation status of PSCs cultured with RFF2, Es8 or SPM was conducted using a methylation beads assay and characteristic methylation status that can lead to "loss of differentiation potential" of ESCs was identified. The comparative study results of the number of methylated genes in RFF2 culture and SPM and Es8 cultures are shown in Fig. 3A, and the clustering of methylation patterns in the promoter region is shown in Fig. 3B. Furthermore, the methylation status of promoters of major genes classified into self-proliferation, ectoderm, mesoderm, mesendoderm and endoderm displayed on scorecard panel (Thermo Fisher Scientific) was examined. The average methylation status in 6 PSC samples cultured with RFF2 (3 samples of iPSC and 3 samples of ESC) (RFF2), and 6 PSC samples cultured with SPM or Es8 (1 sample of iPSC and 2 samples of ESC cultured with SPM and 1 sample of iPS and 2 samples of ESC cultured with Es8) (SPM&Es8) is shown in Table (Table 3). All cells were maintained in an undifferentiated state, but there was no significant difference in the methylation pattern in the promoter region of these genes even when different medium was used. In Table 3, a numerical value of less than 0.2 means a low methylation status, a numerical value of 0.2 or more and less than 0.5 means a moderate methylation status, and a numerical value of 0.5 or more means a high methylation status.

[Table 3-1]

self-renewal

| GENE_SYMBOL | RFF2 | SPM&Es8 | Ratio |
|---|---|---|---|
| KLF4 | 0.02 | 0.02 | 1.02 |
| KLF2 | 0.21 | 0.19 | 1.12 |
| TFCP2L1 | 0.06 | 0.06 | 0.97 |
| SOX2 | 0.02 | 0.02 | 0.94 |
| EP300(p300) | 0.04 | 0.04 | 0.85 |
| ZFP42(REX1) | 0.03 | 0.07 | 0.43 |
| NANOG | 0.36 | 0.23 | 1.58 |
| POU5F1 | 0.26 | 0.36 | 0.71 |
| HESX1 | 0.64 | 0.59 | 1.08 |
| LCK | 0.73 | 0.69 | 1.07 |
| DNMT3B | 0.69 | 0.71 | 0.97 |
| TRIM22 | 0.88 | 0.89 | 0.98 |
| IDO1 | 0.92 | 0.93 | 0.99 |

ectoderm

| GENE_SYMBOL | RFF2 | SPM&Es8 | Ratio |
|---|---|---|---|
| COL2A1 | 0.04 | 0.04 | 0.98 |
| DRD4 | 0.04 | 0.06 | 0.76 |
| EN1 | 0.07 | 0.05 | 1.39 |
| LMX1A | 0.03 | 0.03 | 1.09 |
| NR2F1 | 0.04 | 0.03 | 1.29 |
| NR2F2 | 0.08 | 0.06 | 1.32 |
| OLFM3 | 0.08 | 0.07 | 1.14 |
| PAPLN | 0.10 | 0.11 | 0.91 |
| PAX3 | 0.05 | 0.04 | 1.45 |
| PAX6 | 0.03 | 0.03 | 1.00 |
| POU4F1 | 0.03 | 0.03 | 1.08 |
| PRKCA | 0.03 | 0.03 | 0.97 |
| SDC2 | 0.04 | 0.03 | 1.17 |
| SOX1 | 0.06 | 0.04 | 1.47 |
| WNT1 | 0.11 | 0.09 | 1.27 |
| ZBTB16 | 0.06 | 0.05 | 1.22 |
| CDH9 | 0.35 | 0.25 | 1.42 |
| LMX1A | 0.84 | 0.83 | 1.01 |
| DMBX1 | 0.82 | 0.83 | 0.99 |
| TRPM8 | 0.89 | 0.89 | 1.01 |
| NOS2 | 0.89 | 0.90 | 1.00 |
| MYO3B | 0.92 | 0.93 | 0.99 |

mesoderm

| GENE_SYMBOL | RFF2 | SPM&Es8 | Ratio |
|---|---|---|---|
| ALOX15 | 0.12 | 0.11 | 1.10 |
| CDX2 | 0.03 | 0.02 | 1.14 |
| FOXF1 | 0.04 | 0.03 | 1.28 |
| HAND1 | 0.03 | 0.03 | 1.06 |
| HAND2 | 0.05 | 0.03 | 1.56 |
| HEY1 | 0.01 | 0.02 | 0.86 |
| IL6ST | 0.04 | 0.04 | 1.02 |
| NKX2-5 | 0.10 | 0.04 | 2.75 |
| PDGFRA | 0.04 | 0.03 | 1.09 |
| SNAI2 | 0.05 | 0.03 | 1.35 |
| TBX3 | 0.03 | 0.03 | 0.98 |
| RGS4 | 0.33 | 0.19 | 1.71 |
| HOPX | 0.22 | 0.22 | 1.02 |
| ESM1 | 0.79 | 0.82 | 0.97 |
| CDH5 | 0.85 | 0.84 | 1.02 |
| TM4SF1 | 0.85 | 0.86 | 0.99 |
| PLVAP | 0.87 | 0.90 | 0.98 |
| ABCA4 | 0.89 | 0.90 | 0.98 |
| FCN3 | 0.90 | 0.91 | 0.98 |
| BMP10 | 0.92 | 0.92 | 1.00 |
| COLEC10 | 0.94 | 0.94 | 1.00 |

mesendoderm

| GENE_SYMBOL | RFF2 | SPM&Es8 | Ratio |
|---|---|---|---|
| FGF4 | 0.05 | 0.05 | 0.89 |
| NPPB | 0.14 | 0.09 | 1.44 |
| PTHLH | 0.03 | 0.03 | 1.18 |
| T | 0.03 | 0.03 | 1.18 |
| GDF3 | 0.80 | 0.83 | 0.97 |
| NR5A2 | 0.91 | 0.92 | 0.99 |

[Table 3-2]

endoderm

| GENE SYMBOL | RFF2 | SPM&Es8 | Ratio |
|---|---|---|---|
| CABP7 | 0.07 | 0.08 | 0.85 |
| CLDN1 | 0.03 | 0.03 | 1.01 |
| CPLX2 | 0.13 | 0.10 | 1.29 |
| EOMES | 0.07 | 0.04 | 1.66 |
| FOXA1 | 0.03 | 0.03 | 1.06 |
| FOXA2 | 0.04 | 0.04 | 0.98 |
| GATA4 | 0.04 | 0.03 | 1.13 |
| GATA6 | 0.02 | 0.02 | 0.91 |
| HHEX | 0.03 | 0.04 | 0.86 |
| HMP19 | 0.09 | 0.07 | 1.38 |
| HNF1B | 0.03 | 0.03 | 1.22 |
| KLF5 | 0.03 | 0.03 | 1.04 |
| NODAL | 0.06 | 0.07 | 0.83 |
| PHOX2B | 0.09 | 0.05 | 1.75 |
| POU3F3 | 0.03 | 0.03 | 1.04 |
| PRDM1 | 0.03 | 0.03 | 0.94 |
| SOX17 | 0.04 | 0.03 | 1.35 |
| ELAVL3 | 0.25 | 0.21 | 1.16 |
| SST | 0.42 | 0.40 | 1.05 |
| LEFTY1 | 0.70 | 0.74 | 0.96 |
| FOXP2 | 0.83 | 0.78 | 1.06 |
| LEFTY2 | 0.84 | 0.81 | 1.03 |
| HNF4A | 0.91 | 0.90 | 1.01 |
| CDH20 | 0.91 | 0.93 | 0.98 |
| AFP | 0.92 | 0.94 | 0.98 |

Then, using GeneChip data (Affymetrix) of the same sample, a gene that showed high methylation status and low gene expression in RFF2 culture, and a gene that showed low methylation status and high gene expression in SPM or Es8 culture were investigated. By principal component analysis (PCA) and GeneChip analysis, four candidates were identified as methylation sites in the promoter region of candidate genes that could be markers for loss of differentiation potential (Table 4). Among the identified genes, chromodomain helicase DNA binding protein 7 (CHD7) was examined noting the function thereof.

[Table 4]

| Description | Methylation status | Gene expression signal |
|---|---|---|
| | RFF2/SPM/Es8 | RFF2/SPM/Es8 |
| DISP1 Dispatched homologue 1 | 0.45/0.26/0.18 | 148/559/500 |
| RIMS3 Regulating Synaptic membrane exocytosis 3 | 0.33/0.21/0.17 | 566/1132/1369 |
| NTS neurotensin | 0.38/0.26/0.23 | 380/512/1721 |
| CHD7 Chromodomain Helicase DNA binding Protein 7 | 0.25/0.17 /0.13 | 688/2553/3844 |

The effect of cell culture with RFF2 on the expression of self-proliferation-related genes such as NANOG, POU5F1, SOX2, EP300 and the like was determine using quantitative reverse transcription polymerase chain reaction (qRT-PCR). As a result, there was no significant effect on the expression level of self-proliferation-related genes. However, it was demonstrated by qRT-PCR that when primers were designed to target the 3' non-translated region of CHD7 for detection of all CHD7 isoforms, the expression of the CHD7 gene was significantly suppressed by culturing ESCs with RFF2 (Fig. 4). The structures of isoform 1, isoform 2 and isoform X4 which are the three major isoforms of CHD7 (Schnetz et al., Genome Res 2009; 19(4): 590-601, Colin et al., BMC Res Notes 2010; 3: 252) and the positions of the primers are shown in Fig. 5A.

Next, the expression of three CHD7 isoforms was examined by Western blotting. As a result, CHD7 isoform 2 in cell lysate derived from Es8 and RFF2 cultures, and CHD7 isoform X4 in the both cell lysates were respectively detected at the same level by antibody recognizing the N-terminal of CHD7. However, CHD7 isoform 1 was not clearly detected in cell lysate derived from RFF2 culture (Fig. 6). The signal intensity of the CHD7 isoform cannot be compared and evaluated correctly by Western blotting when the molecular size of the target protein is different.

Based on the above, each isoform was quantified by digital PCR using an isoform-specific TaqMan primer (Fig. 5A). The copy number of CHD7 isoform 1, isoform 2 or isoform X4 determined by digital PCR is shown (Table 5). For quantification, total RNA (5 ng) obtained from KhES-1 cells cultured with Es8 or RFF2 medium was used as a template. Using primer set 3, the copy number of isoform 1 in each RNA sample was calculated. The copy number of isoform X4 was determined by subtracting the number of copies generated by primer set 3 from the number of copies generated by primer set 1 (Fig. 5A). Consistent with the results of Western blotting, isoform 1 was found to be the major isoform and initial target affected by culture conditions. Based on the results of Western blotting, the copy number of isoform 2 was expected to be the same as or less than the copy number of isoform X4. However, Taq man primer set 2 sandwiching the spliced sequence did not function properly due to primer design issues. Structural analysis of the CHD7 isoforms showed that isoform 1 contains a regulatory region extending from 527 to 2576 amino acids in the middle of the protein. This region contains ATPase/DNA helicase domains, a chromosome binding domain, a DNA binding domain, and a BRK domain (Allen et al., J Mol Biol 2007; 371(5): 1135-1140, Colin et al., BMC Res Notes 2010; 3: 252, Ryan et al., Embo j 2011; 30(13): 2596-2609). Isoform 2 lacks regulatory regions of isoform 1. Isoform X4 (Fig. 5A), which is another splicing variant of isoform 1, also altered gene expression thereof depending on the culture conditions.

[Table 5]

| | copy number in 5ng total RNA | | | |
|---|---|---|---|---|
| Isoform type | Es8 P8 | Es8 P10 | RFF2 P5 | RFF2 P10 |
| Isoform 1 | 6380 | 7460 | 688 | 698 |
| isoform X4 | 220 | 320 | 52 | 34 |

Since it was shown that differentiation potential is related to CHD7 expression, whether KhES-1 cells cultured with Es8 lose differentiation potential when the expression level of mCHD7 downregulates by introduction of siRNA (Fig. 5B) was investigated. In addition, it was investigated whether KhES-1 cells cultured with RFF2 are differentiated when CHD7 mRNA was introduced. However, due to the length of the translation region, a full-length CHD7 isoform 1 mRNA could not be designed. Instead, CHD7 isoform 2 mRNA lacking the regulatory region (Fig. 5B) was prepared and mRNA was introduced into KhES-1 cells cultured with RFF2. Furthermore, to inhibit the activity by being competitive with the functional region of CHD7 isoform 1, mRNA encoding a DNA binding domain (SANT-SLIDE domain) assumed to be a regulatory region and mRNA producing a dominant negative protein encoding a chromatin interaction domain (chromodomain) and SNF2-like ATPase/helicase domain were respectively designed and introduced into KhES-1 cells cultured with Es8.

2. Down-regulation of CHD7 perturbs differentiation:

KhES-1 cells cultured with Es8 maintain differentiation potential and form EB. First, whether downregulation of CHD7 occurs in KhES-1 cells when siCHD7 is transfected was examined. 10 pmol, 30 pmol or 50 pmol of siCHD7 was transfected per well of a 6-well dish, and the expression level of CHD7 was examined. As a result, the expression level of CHD7 decreased as the introduced amount of siCHD7 increased, and the dependency of the introduction amount was observed (Fig. 7B). Next, the differentiation of siCHD7-transfected KhES-1 cells into three germ layers was examined. In EBs, perturbation of mesoderm differentiation was observed on day 5 and development of mesoderm and endoderm was suppressed to a moderate level on day 14 (Fig. 8). Downregulation of CHD7 mRNA with a single introduction of siCHD7 was not complete. A single introduction of siCHD7 failed to prevent initiation of ectoderm differentiation but perturbed development of mesoderm and endoderm after 14 days of culture. These data suggested that, to promote development of the three germ layers, the expression level of CHD7 needs to be maintained at a constant level throughout EB differentiation.

PSCs can be effectively cultured with high glucose (3.1 g/L) Es8 on a VTN-N-coated dish. When PSCs are cultured with nutrient-depleted Es8 (i.e., omitting daily medium change), differentiation of PSCs may be triggered (Vander Heiden et al., Science 2015; 324(5930): 1029-1033, Yanes et al., Nat Chem Biol 2010; 6(6): 411-417; Moussaieff et al.,

Cell Metab 2015; 21(3): 392-402). Mock-transfected KhES-1 cells cultured with nutrient-depleted Es8 were differentiated and were not maintained with Es8 on a VNT-N-coated dish. On the other hand, siCHD7-transfected KhES-1 cells remained on the VNT-N-coated dish 4 days after introduction (Fig. 9C), and exhibited a relatively undifferentiated genetic profile (Fig. 10). The cells did not proliferate in nutrient-depleted Es8, but downregulation of CHD7 expression via siCHD7 prevented differentiation caused by depletion of the nutrient (Fig. 10). Non-transfected KhES-1 cells with daily change of Es8 were used as normal culture control.

3. Introduction of CHD7 mRNA induces three germ layer differentiation:

Introduction of CHD7 isoform 2 mRNA induced "spontaneous" differentiation of KhES-1 cells cultured with RFF2 without any additional differentiation stimulus (Fig. 12). KhES-1 cells cultured with RFF2 medium were transfected with CHD7 isoform 2, and the expression level of 94 genes in KhES-1 cells after 1, 2 and 3 days was determined by qRT-PCR scorecard panel (Table 6). In the Table, when fold change (fc) is not less than 2.0, it means upregulation, and when it is not more than 0.1, it means downregulation. The PSC culture system is designed to maintain undifferentiated cells rather than differentiated cells. When differentiated, KhES-1 cells could not be cultured on a VTN-N-coated dish, and the number of KhES-1 cells in the RFF2 culture decreased as the cells differentiated (Fig. 11C and Fig. 12). In particular, overexpression of CHD7 isoform 2 simultaneously induced three germ layer differentiation without following a continuous differentiation process.

[Table 6-1]

| Target Name | Category | KhES-1 RFF2 culture | | | | | | | | |
|---|---|---|---|---|---|---|---|---|---|---|
| | | Day1 | | | Day2 | | | Day3 | | |
| | | Nontrans | mock | mCHD7 | Nontrans | mock | mCHD7 | Nontrans | mock | mCHD7 |
| CXCL5 | | 12.60 | 10.05 | 7.18 | 8.51 | 6.78 | 5.12 | 6.31 | 6.68 | 4.87 |
| DNMT3B | | 1.23 | 1.01 | 0.82 | 1.17 | 1.46 | 0.84 | 1.80 | 1.64 | 1.39 |
| HESX1 | | 0.41 | 0.28 | 0.24 | 0.26 | 0.21 | 0.21 | 0.37 | 0.39 | 0.32 |
| IDO1 | | 0.55 | 0.31 | 0.38 | 0.76 | 0.48 | 0.41 | 0.90 | 0.83 | 0.68 |
| LCK | Self-renewal | 1.28 | 0.99 | 1.07 | 1.70 | 1.54 | 0.92 | 2.01 | 1.76 | 1.10 |
| NANOG | | 3.09 | 2.94 | 2.19 | 4.67 | 3.17 | 2.42 | 3.92 | 3.24 | 2.62 |
| POU5F1 | | 0.98 | 0.64 | 0.55 | 1.80 | 1.30 | 1.35 | 1.63 | 1.30 | 0.77 |
| SOX2 | | 0.76 | 0.56 | 0.65 | 0.71 | 0.61 | 0.71 | 0.74 | 0.67 | 0.78 |
| TRIM22 | | 21.76 | 19.35 | 34.22 | 28.64 | 22.26 | 17.95 | 17.20 | 21.28 | 14.86 |
| CDH9 | | 0.01 | 0.02 | 0.36 | 0.04 | 0.01 | 0.08 | 0.01 | 0.03 | 0.05 |
| COL2A1 | | 0.21 | 0.19 | 1.75 | 0.77 | 0.43 | 3.09 | 0.35 | 0.33 | 1.03 |
| DMBX1 | | 0.42 | 0.34 | 0.40 | 0.16 | 0.22 | 0.51 | 0.13 | 0.10 | 0.38 |
| DRD4 | | 0.22 | 0.24 | 0.50 | 0.32 | 0.22 | 0.46 | 0.33 | 0.33 | 0.56 |
| EN1 | | 0.65 | 1.23 | 69.19 | 0.87 | 2.96 | 8.41 | 0.44 | 0.36 | 2.43 |
| LMX1A | | 0.00 | 0.08 | 2.64 | 0.01 | 0.29 | 2.46 | 0.00 | 0.02 | 0.48 |
| MAP2 | | 7.24 | 11.63 | 10.18 | 14.79 | 16.34 | 14.96 | 2.66 | 3.86 | 5.35 |
| MYO3B | | 0.43 | 0.29 | 0.35 | 0.76 | 2.36 | 0.32 | 0.54 | 0.81 | 0.65 |
| NOS2 | | 0.82 | 0.75 | 1.31 | 0.70 | 1.08 | 1.68 | 0.77 | 0.90 | 0.68 |
| NR2F1/NR2F2 | | 0.00 | 0.03 | 21.43 | 0.01 | 0.31 | 28.47 | 0.01 | 0.06 | 7.33 |
| NR2F2 | | 0.30 | 0.23 | 3.44 | 0.63 | 1.10 | 1.56 | 0.32 | 0.41 | 1.38 |
| OLFM3 | Ectoderm | 0.68 | 0.57 | 2.00 | 0.39 | 0.44 | 1.23 | 0.27 | 0.46 | 0.77 |
| PAPLN | | 0.19 | 0.21 | 0.41 | 0.44 | 0.34 | 0.43 | 0.36 | 0.39 | 0.35 |
| PAX3 | | 0.58 | 0.33 | 59.94 | 1.94 | 0.50 | 12.68 | 0.07 | 0.10 | 5.65 |
| PAX6 | | 0.13 | 0.18 | 16.25 | 0.11 | 0.13 | 10.65 | 0.04 | 0.11 | 2.26 |
| POU4F1 | | 0.40 | 0.04 | 42.21 | 0.04 | 0.10 | 33.30 | 0.49 | 0.32 | 7.77 |
| PRKCA | | 0.77 | 0.75 | 0.57 | 0.79 | 0.72 | 0.84 | 0.65 | 0.72 | 0.78 |
| SDC2 | | 29.34 | 19.66 | 20.10 | 26.51 | 18.98 | 18.81 | 17.11 | 16.78 | 15.36 |
| SOX1 | | 0.05 | 0.07 | 0.22 | 0.11 | 0.02 | 0.19 | 0.19 | 0.10 | 0.14 |
| TRPM8 | | 0.57 | 0.67 | 0.37 | 0.28 | 0.78 | 0.63 | 1.59 | 0.93 | 1.53 |
| WNT1 | | 1.73 | 4.36 | 3.56 | 1.45 | 1.49 | 4.66 | 2.25 | 1.82 | 10.01 |
| ZBTB16 | | 0.24 | 0.27 | 0.58 | 0.27 | 0.36 | 0.74 | 0.47 | 0.27 | 0.62 |
| ABCA4 | | 0.59 | 0.75 | 0.87 | 0.99 | 0.92 | 2.17 | 1.17 | 1.80 | 2.41 |
| ALOX15 | | 2.62 | 2.32 | 2.67 | 3.03 | 2.74 | 2.18 | 3.13 | 3.47 | 2.35 |
| BMP10 | | 1.29 | 1.24 | 3.39 | 3.85 | 2.46 | 3.14 | 0.32 | 1.29 | 0.06 |
| CDH5 | | 6.21 | 4.44 | 3.01 | 4.59 | 11.32 | 7.23 | 1.09 | 3.76 | 8.08 |
| CDX2 | | 0.15 | 0.16 | 8.22 | 0.05 | 0.32 | 10.09 | 0.27 | 0.66 | 6.01 |
| COLEC10 | | 0.49 | 1.09 | 0.72 | 1.65 | 2.72 | 2.39 | 1.95 | 2.17 | 1.41 |
| ESM1 | | 1.83 | 1.58 | 1.74 | 0.03 | 0.56 | 0.51 | 2.82 | 2.68 | 3.67 |
| FCN3 | | 0.69 | 0.58 | 0.66 | 1.46 | 1.02 | 0.95 | 1.76 | 1.17 | 0.47 |
| FOXF1 | | 0.30 | 0.51 | 13.58 | 0.69 | 1.58 | 17.82 | 0.18 | 0.92 | 6.37 |
| HAND1 | | 0.18 | 0.26 | 1.57 | 0.14 | 0.70 | 1.66 | 0.10 | 0.89 | 5.67 |
| HAND2 | | 0.37 | 0.63 | 273.66 | 0.02 | 1.04 | 317.00 | 0.24 | 0.72 | 60.26 |
| HEY1 | Mesoderm | 0.74 | 0.65 | 1.33 | 0.59 | 0.77 | 1.01 | 0.44 | 0.51 | 0.72 |
| HOPX | | 1.90 | 0.40 | 15.67 | 2.09 | 0.50 | 2.38 | 0.28 | 0.44 | 1.46 |
| IL6ST | | 1.15 | 1.51 | 1.85 | 1.05 | 2.05 | 3.57 | 0.81 | 1.36 | 2.49 |
| NKX2-5 | | 2.60 | 2.56 | 33.32 | 2.65 | 3.16 | 25.85 | 2.11 | 2.34 | 6.08 |
| ODAM | | 0.96 | 1.71 | 15.69 | 1.30 | 1.48 | 4.54 | 0.53 | 1.40 | 1.87 |
| PDGFRA | | 1.25 | 0.84 | 0.57 | 0.70 | 0.84 | 0.36 | 0.51 | 0.81 | 0.73 |
| PLVAP | | 1.23 | 0.78 | 1.32 | 1.70 | 1.32 | 1.85 | 2.12 | 1.53 | 1.19 |
| RGS4 | | 0.77 | 0.32 | 1.53 | 0.30 | 0.37 | 3.24 | 1.27 | 1.48 | 2.02 |
| SNAI2 | | 0.60 | 0.72 | 0.73 | 0.39 | 0.59 | 0.75 | 0.32 | 0.46 | 0.97 |
| TBX3 | | 1.42 | 1.83 | 3.02 | 0.59 | 1.24 | 3.39 | 0.85 | 1.92 | 2.82 |
| TM4SF1 | | 0.38 | 0.22 | 0.46 | 0.37 | 0.39 | 0.46 | 0.39 | 0.42 | 0.99 |

[Table 6-2]

| Target Name | Category | KhES-1 RFF2 culture | | | | | | | | |
|---|---|---|---|---|---|---|---|---|---|---|
| | | Day1 | | | Day2 | | | Day3 | | |
| | | Nontrans | mock | mCHD7 | Nontrans | mock | mCHD7 | Nontrans | mock | mCHD7 |
| FGF4 | Mesendoderm | 10.49 | 5.56 | 5.04 | 12.41 | 11.18 | 5.41 | 12.77 | 16.21 | 18.31 |
| GDF3 | | 23.80 | 15.71 | 9.35 | 26.97 | 22.59 | 12.60 | 42.30 | 37.12 | 26.71 |
| NPPB | | 3.15 | 7.14 | 12.79 | 2.16 | 4.78 | 16.30 | 0.79 | 2.09 | 7.36 |
| NR5A2 | | 128.86 | 122.08 | 259.54 | 161.63 | 161.89 | 313.07 | 184.43 | 179.10 | 174.98 |
| PTHLH | | 1.02 | 1.24 | 45.09 | 0.52 | 1.36 | 32.69 | 0.28 | 0.73 | 9.52 |
| T | | 0.00 | 0.00 | 0.01 | 0.00 | 0.00 | 0.01 | 0.00 | 0.00 | 0.02 |
| AFP | Endoderm | 0.04 | 0.03 | 0.32 | 0.00 | 0.01 | 0.10 | 0.00 | 0.00 | 0.06 |
| CABP7 | | 2.02 | 2.05 | 5.18 | 2.70 | 2.48 | 3.55 | 3.66 | 5.14 | 8.92 |
| CDH20 | | 0.75 | 0.39 | 3.28 | 0.36 | 1.01 | 3.05 | 0.34 | 0.45 | 1.26 |
| CLDN1 | | 1.84 | 2.30 | 3.95 | 2.79 | 2.95 | 4.35 | 1.78 | 2.16 | 2.40 |
| CPLX2 | | 2.34 | 2.17 | 4.33 | 1.64 | 2.27 | 2.91 | 2.45 | 2.99 | 3.19 |
| ELAVL3 | | 0.81 | 0.88 | 3.33 | 3.67 | 2.07 | 2.33 | 2.45 | 1.70 | 0.85 |
| EOMES | | 0.02 | 0.07 | 13.59 | 0.05 | 0.05 | 5.22 | 0.03 | 0.03 | 1.94 |
| FOXA1 | | 0.10 | 0.07 | 21.37 | 0.07 | 0.19 | 14.07 | 0.02 | 0.09 | 4.44 |
| FOXA2 | | 0.01 | 0.03 | 0.11 | 0.04 | 0.05 | 0.27 | 0.04 | 0.05 | 0.29 |
| FOXP2 | | 0.24 | 0.12 | 3.96 | 0.22 | 0.32 | 7.37 | 0.12 | 0.16 | 2.35 |
| GATA4 | | 0.02 | 0.09 | 4.26 | 0.03 | 0.10 | 3.65 | 0.03 | 0.06 | 1.21 |
| GATA6 | | 0.10 | 0.27 | 2.67 | 0.08 | 0.48 | 2.40 | 0.03 | 0.25 | 1.28 |
| HHEX | | 0.10 | 0.11 | 4.36 | 0.16 | 0.12 | 2.62 | 0.13 | 0.21 | 1.01 |
| HMP19 | | 0.44 | 0.52 | 0.52 | 0.80 | 0.72 | 1.31 | 1.33 | 0.76 | 0.75 |
| HNF1B | | 0.02 | 0.02 | 0.36 | 0.03 | 0.05 | 0.32 | 0.01 | 0.05 | 0.22 |
| HNF4A | | 0.03 | 0.12 | 0.87 | 0.02 | 0.26 | 0.89 | 0.02 | 0.14 | 0.31 |
| KLF5 | | 4.02 | 6.91 | 19.30 | 4.14 | 5.08 | 11.28 | 1.97 | 2.96 | 6.25 |
| LEFTY1 | | 0.44 | 0.36 | 0.31 | 0.35 | 0.43 | 0.30 | 0.26 | 0.28 | 0.23 |
| LEFTY2 | | 0.26 | 0.23 | 0.14 | 0.11 | 0.16 | 0.23 | 0.05 | 0.09 | 0.14 |
| NODAL | | 1.11 | 1.06 | 0.95 | 1.19 | 1.57 | 1.40 | 0.71 | 0.70 | 1.15 |
| PHOX2B | | 0.03 | 0.03 | 2.55 | 0.03 | 0.03 | 1.73 | 0.03 | 0.04 | 0.40 |
| POU3F3 | | 0.05 | 0.08 | 3.46 | 0.05 | 0.06 | 1.53 | 0.08 | 0.07 | 0.44 |
| PRDM1 | | 0.21 | 0.17 | 1.74 | 0.31 | 0.49 | 3.66 | 0.34 | 0.44 | 1.70 |
| RXRG | | 0.05 | 0.98 | 2.07 | 0.01 | 0.86 | 6.17 | 0.05 | 0.35 | 2.02 |
| SOX17 | | 0.07 | 0.07 | 54.39 | 0.12 | 0.11 | 4.12 | 0.01 | 0.25 | 1.76 |
| SST | | 12.92 | 10.67 | 12.19 | 10.84 | 14.97 | 17.65 | 19.21 | 16.72 | 23.81 |

To inhibit or decrease CHD7 isoform 1 activity by being competitive with the functional region of CHD7 isoform 1, mRNA that generates a dominant negative protein covering chromodomain that recognizes the binding site of histone with a specific methylation status and the SNF2-like ATPase/helicase domain (CHD7 DN1) was introduced into KhES-1 cells (Fig. 5B and Fig. 13 A, B). By the introduction of CHD7 DN1, inhibition or depression of both differentiation potential and cell proliferation was observed in the EB formation assay (Fig. 13C). Furthermore, when mRNA that generates a dominant negative protein of SANT-SLID domain (CHD7 DN2), which is a putative DNA binding site of CHD7, was introduced into KhES-1 cells (Fig. 5B and Fig. 13 A, B), differentiation potential was also inhibited or depressed in the EB formation assay, thus showing decreased cell proliferation (Fig. 13C).

Introduction of CHD7 isoform 2 mRNA also suggested a possibility of the presence of the upper limit of CHD7 expression when ESCs are maintained in an undifferentiated state. The upper limit may sometimes vary depending on the culture conditions. Indeed, introduction of CHD7 isoform 2 mRNA into cultured KhES-1 cells with Es8 did not upregulate CHD7 isoform 2 in KhES-1 cells in Es8 culture (Fig. 14). It is considered that introduction of CHD7 isoform 2 mRNA into KhES-1 cells induces "spontaneous" differentiation of KhES-1 cells in Es8 and the Es8 culture system cannot support differentiated cells.

4. CHD7 expression level regulates proliferation rate of cells:

Another function of CHD7 is to support proliferation of ESCs. $1\times10^5$ KhES-1 cells were seeded per well of a 6-well plate, the cells were cultured with Es8, and $8\times10^5$ cells were harvested after 3 days. The proliferation rate of KhES-1 cells in RFES2 medium was 1/3 of that using Es8; however, when Es8 was used, KhES-1 cells were observed to expand 8 times after 3 days of culture. To examine whether the CHD7 expression level can modulate the proliferation rate of ESC in a concentration-dependent manner, CHD7 was downregulated by transfection of siCHD7 into KhES-1 cells, the cells were cultured with Es8 and the number of cells was counted (Fig. 15 A, B). As a result, the proliferation rate of KhES-1 cells was regulated by the expression level of CHD7 mRNA (Fig. 15 C, D). Furthermore, introduction of mRNA into KhES-1 cells that produces a dominant-negative protein of CHD7 covering the chromodomain and/or the SANT-SLIDE domain dramatically reduced the cell proliferation rate (Fig. 13).

5. Expression level of CHD7 mediates differentiation potential of PSCs:

Whether the CHD7 mRNA level correlates with the differentiation potential of ESCs was examined by measuring the expression level of CHD7 mRNA of PSCs cultured under various conditions. First, using the GeneChip database, the expression level of CHD7 mRNA in embryo at various embryo formation stages before implantation, and the expression level of CHD7 mRNA of PSCs and EBs were examined (Fig. 16). CHD7 mRNA corresponding to isoform 1 was expressed at a relatively high level in the 2- and 4-cell stages and thereafter expressed at a low level in the morula stage and blastocyst stage (Fig. 16, "2 cell", "4 cell", "morula" and "blastocyst"). During the embryo formation, a fertilized egg is differentiated and proliferates. Embryos reach the blastocyst stage, which is composed of proliferation of the same cell mass without a developmental axis, which is called an inner cell mass. In the measurement of a GeneChip expression signal, PSCs showed a gene expression profile similar to the gene expression profile of mouse epiblast after implantation and various levels of CHD7 mRNA according to the culture conditions. PSCs showing low expression of CHD7 (Fig. 16, "KhES-1 RFF2/N" and "PFX#9 RFF2/N") maintained a proliferation ability in an undifferentiated state but lost differentiation potential.

Since GeneChip expression signal may not be suitable for quantification, the copy number of CHD7 isoform 1 mRNA in 5 ng of the total RNA was quantified by digital PCR in various PSCs cultured under various conditions. Specifically, the cells used were H9 or KhES-1 (each ESC), or PFX#9, 201B7 or SHh#2 (each iPSC). The cells were cultured by the Small Cell Clumps method with hPSC medium on feeder cells. Alternatively, they were cultured in single cells on a VTN-N-coated dish with Es8, RFF2 or SPM. The copy number of CHD7 isoform 1 mRNA in 5 ng of the total RNA extracted from the cultured cells was determined by droplet digital PCR. PSCs cultured with RFF2 showed a low copy number of CHD7 isoform 1 in 5 ng of the total RNA and did not show differentiation potential; however, differentiation potential was confirmed in the cells cultured under other conditions. The copy number and passage numbers (P) thereof are shown in Table 7. The results of specifically analyzed differentiation potential of some of the cells cultured under these culture conditions are shown in Fig. 17.

[Table 7]

| | H9 | KhES-1 | PFX#9 | 201B7 | SHh#2 | Passage Method |
|---|---|---|---|---|---|---|
| on feeder | 5220 (P29)<br>5100 (P36)<br>4900 (P38) | 2120 (P32)<br>2338 (P32)<br>2710 (P32)<br>3340 (P35) | 3080 (P25)<br>3320 (P35)<br>2855 (P37) | 4200 (P29)<br>2280 (P35)<br>2520 (P40) | 630 (P19)<br>542 (P24) | Small Cell Clumps |
| Es8/VNT-N | 6560 (P5)<br>7340 (P17)<br>8900 (P20) | 9320 (P7)<br>7460 (P10)<br>6380 (P10)<br>6540 (P13) | 4620 (P5)<br>5740 (P11)<br>4060 (P10)<br>4260 (P16) | 6060 (P9)<br>5360 (P10) | 5120 (P5)<br>5980 (P6) | Single Cell Suspension |
| SPM/VNT -N | 3280 (P10) | 4520 (P5)<br>3940 (P10)<br>6540 (P15) | 4580 (P10)<br>4600 (P15) | 4540 (P15) | ND | |
| RFF2/VNT-N | 352 (P11)<br>266 (P8) | 688 (P5)<br>698 (P9)<br>490 (P10) | 652 (P20)<br>274 (P30)<br>246 (P45) | ND | 732 (P5) | |

With regard to PSCs having differentiation potential, the threshold value of the copy number of CHD7 isoform 1 mRNA was further examined. Specifically, the differentiation potential of the cells made to have a lower copy number of CHD7 mRNA by culturing under conditions of overgrowth than normal (201B7 or PFX#9) was analyzed. The results of the copy number of CHD7 mRNA by digital PCR are shown in Table 8, and the results of differentiation potential are shown in Fig. 18.

[Table 8]

| | PFX#9 | 201B7 | Passage Method |
|---|---|---|---|
| Es8/VNT -N | 1760 (P5)<br>2720 (P40) | 1502 (P5)<br>2580 (P23) | Single Cell Suspension |

It was shown that 201B7 and PFX#9 have differentiation potential even when the copy number of CHD7 isoform 1 mRNA in 5 ng of total RNA was 1502 copies (201B7) or and 1760 copies (PFX#9) (Fig. 18). Based on the data of Table 7 and Table 8, and Fig. 17 and Fig. 18, a numerical criterion is proposed that when cultured at least feeder cell-free in single cells, in 5 ng of the total RNA, PSCs having a copy number of not more than 732 does not differentiate but PSCs having a copy number of not more than 1500 maintains differentiation potential. Therefore, the copy number of CHD7 isoform 1 of PSC may be a good numerical value marker for predicting the degree of differentiation property while maintaining PSCs in an undifferentiated state.

6. Expression level of CHD7 protein is also a prediction marker of differentiation potential of PSCs:

To study not only the copy number of CHD7 mRNA but also the relationship between the expression level of CHD7 protein and the differentiation potential of PSCs, the measurement of the expression level of CHD7 protein was performed.

The culture conditions, the results of copy number of mRNA of CHD7 isoform 1 and differentiation potential of each cell are shown in Table 9, and the results of sandwich ELISA are shown in Fig. 19.

[Table 9]

| | Cell | Medium | Passage Method | CHD7 mRNA Copy No. | Differentiation |
|---|---|---|---|---|---|
| P1 | H9 | Essential 8 | Single Cell Suspension | 7340 (P17) | + |
| P2 | KhES | Essential 8 | Single Cell Suspension | 6460 (p10) | + |
| N | KhES | RFF2 | Single Cell Suspension | 232 (P11) | + |
| N2 | H9 | RFF2 | Single Cell Suspension | 316 (P17) | + |

The graph shows the values relative to the CHD7 protein concentration of a standard (same protein solution as P1 and concentrated to a concentration of 4.06 mg/mL using Amicon (registered trade mark) Ultra-4, PLTK Ultracel-PL membrane at 30 kDa (Millipor, UFC803024)) as 1000 Units/mL. In the case of F1/S5K, CHD7 protein concentrations of P1 and P2 were 9.2 times (N is 10.9% of P1) and 7.0 times (N is 14.2% of P2) as compared to the protein concentration of N. Similarly, in the case of F1/S10K, it was respectively 10.2 times (N is 9.8% of P1) and 7.7 times (N is 13.0% of P2), in the case of F3/S5K, it was respectively 6.0 times (N is 16.6% of P1) and 5.0 times (N is 19.8% of P2), and in the case of F3/S10K, it was respectively 10.3 times (N is 9.7% of P1) and 8.5 times (N is 11.7% of P2). As shown in Fig. 19, CHD7 protein concentration and the copy number of mRNA of CHD7 isoform 1 are low in differentiation-resistant PSCs: PSCs that show good differentiation potential in response to differentiation stimulus were confirmed to show high CHD7 protein concentration and high copy number of mRNA of CHD7 isoform 1, and a correlation was found between the expression level of CHD7 protein, and the copy number and differentiation potential of mRNA of CHD7 isoform 1 in PSCs. Not only the copy number of mRNA but also the expression level of CHD7 protein could be a good numerical marker for predicting the degree of differentiation property during PSCs are maintained in an undifferentiated state.

Analysis using CHD7 protein level and expression level of CHD7 gene:

In consideration of the CHD7 protein level and gene expression level and using the above-mentioned results, the threshold value was determined.

[Table 10]

| ELISA measured values | | | | | |
|---|---|---|---|---|---|
| label | sample | concentration (U/mL) | sample/N (times) | N/sample (%) | 100-N/sample (%) |
| F1/S5k | P1 | 295.4 | 9.2 | 10.9 | 89.1 |
| | P2 | 226.9 | 7.0 | 14.2 | 85.8 |
| | N | 32.2 | | | |
| | N2 | 28.2 | | | |
| F1/S10k | P1 | 267.4 | 10.2 | 9.8 | 90.2 |
| | P2 | 203.0 | 7.7 | 13.0 | 87.0 |
| | N | 26.3 | | | |
| | N2 | 24.2 | | | |
| F3/S5k | P1 | 294.3 | 6.0 | 16.6 | 83.4 |
| | P2 | 245.8 | 5.0 | 19.8 | 80.2 |
| | N | 48.8 | | | |
| | N2 | 25.5 | | | |
| F3/S10k | P1 | 283.0 | 10.3 | 9.7 | 90.3 |
| | P2 | 233.7 | 8.5 | 11.7 | 88.3 |
| | N | 27.4 | | | |
| | N2 | 21.2 | | | |

[Table 11]

| Data of F3/S10k and mRNA copy No. | | |
|---|---|---|
| | units/mL (X axis) | mRNA Copy No. (Y axis) |
| | 0 | 0 |
| N | 27 | 232 |
| P2 | 234 | 6460 |
| P1 | 283 | 7340 |

Asymptote was drawn from the mRNA copy number and the protein concentration (units/mL) obtained from the ELISA results (Fig. 20). When calculated using the asymptote, the concentration (x) was 56.6 units/mL when the copy number (y) in 5 ng of total RNA was 1500 copies. This was 2.1 times the concentration of N (N was 52% of the obtained concentration). Similarly, the concentration (x) was 102.2 units/mL when the copy number (y) in 5 ng of total RNA was 2710 copies. This was 3.8 times the concentration of N (N was 74% of the obtained concentration). In the DESCRIPTION, it is appreciated that a necessary appropriate threshold value can be obtained by using the asymptote also in copy number (y) which is other than those mentioned above and predicted to show differentiation potential in response to a differentiation stimulus.

[Industrial Applicability]

Whether PSC shows a differentiation potential in response to a differentiation stimulus can be predicted in an undifferentiated state before applying a differentiation stimulus by measuring the expression level of CHD7 in PSC. In addition, whether a medium used for maintenance culture of PSC is suitable for maintaining PSC in a state holding a property showing a differentiation potential in response to a differentiation stimulus can also be evaluated. Therefore, the present invention can be utilized for providing hPSC that shows no differentiation resistance during differentiation induction and has a reduced risk of tumorigenesis, and is extremely useful in a transplantation therapy using a hiPSC-derived differentiated cell. The present invention is also useful for searching for a medium and/or culture conditions suitable for maintenance culture of PSC so that differentiation resistance will not appear during differentiation induction.

This application is based on patent application No. 2017-120024 filed in Japan (filing date: June 19, 2017) and patent application No. 2017-237420 filed in Japan (filing date: December 12, 2017), the contents of which are incorporated in full herein.

## Claims

1. A method for predicting a differentiation potential of a pluripotent stem cell comprising measuring an expression level of CHD7 of the human pluripotent stem cell.

2. The method according to claim 1, wherein a human pluripotent stem cell having the expression level of CHD7 of not less than 1500 copies in 5 ng of the total RNA is predicted to show a differentiation potential in response to a differentiation stimulus.

3. The method according to claim 2, wherein the expression level of CHD7 is not less than 2710 copies in 5 ng of the total RNA.

4. The method according to claim 2 or 3, wherein the expression level of CHD7 is an expression level of a human pluripotent stem cell cultured for not less than 5 passages with Essential 8 medium or Stem-Partner (registered trade mark) Human iPS/ES cells medium.

5. The method according to any one of claims 1 to 4, wherein the human pluripotent stem cell is an embryonic stem cell or an induced pluripotent stem cell.

6. A method for evaluating a medium for a human pluripotent stem cell comprising measuring an expression level of CHD7 of the pluripotent stem cell.

7. The method according to claim 6, wherein the human pluripotent stem cell is a human pluripotent stem cell cultured for not less than 5 passages with the test medium.

8. The method according to claim 6 or 7, wherein the test medium is evaluated as being capable of maintaining the human pluripotent stem cell to show a differentiation potential in response to a differentiation stimulus, when the expression level of CHD7 is not less than 1500 copies in 5 ng of the total RNA.

**9.** The method according to claim 8, wherein the expression level of CHD7 is not less than 2710 copies in 5 ng of the total RNA.

**10.** The method according to any one of claims 6 to 9, wherein the human pluripotent stem cell is an embryonic stem cell or an induced pluripotent stem cell.

**11.** A reagent or kit for predicting a differentiation potential of a human pluripotent stem cell and/or evaluating a medium for human pluripotent stem cell, comprising a substance capable of detecting an expression of CHD7.

**12.** A differentiation-inducing agent for a human pluripotent stem cell comprising a nucleic acid encoding CHD7.

**13.** The method according to claim 1, wherein the human pluripotent stem cell is predicted to show a differentiation potential in response to a differentiation stimulus, when the expression level of CHD7 is a protein level not less than two times a CHD7 protein level of a human pluripotent stem cell showing a differentiation resistance.

**14.** The method according to claim 13, wherein the expression level of CHD7 is an expression level of a human pluripotent stem cell cultured for not less than 5 passages with Essential 8 medium or Stem-Partner (registered trade mark) Human iPS/ES cells medium.

**15.** The method according to claim 13 or 14, wherein the human pluripotent stem cell showing the differentiation resistance is a human pluripotent stem cell cultured for not less than 5 passages with ReproFF2 medium.

Fig. 1

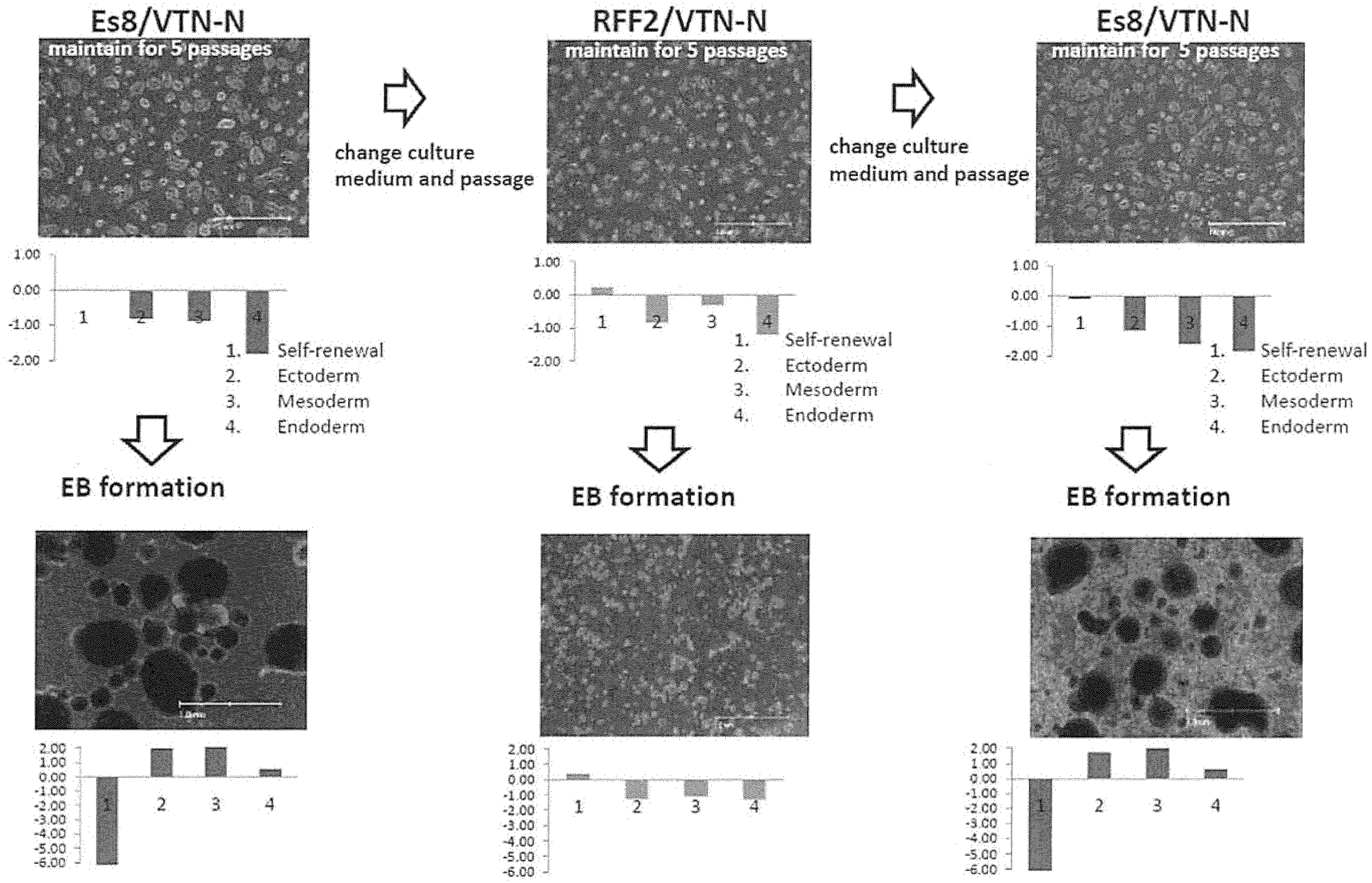
Es8/VTN-N
maintain for 5 passages
change culture
medium and passage
RFF2/VTN-N
maintain for 5 passages
change culture
medium and passage
Es8/VTN-N
maintain for 5 passages
1. Self-renewal
2. Ectoderm
3. Mesoderm
4. Endoderm
EB formation
EB formation
EB formation

Fig. 2

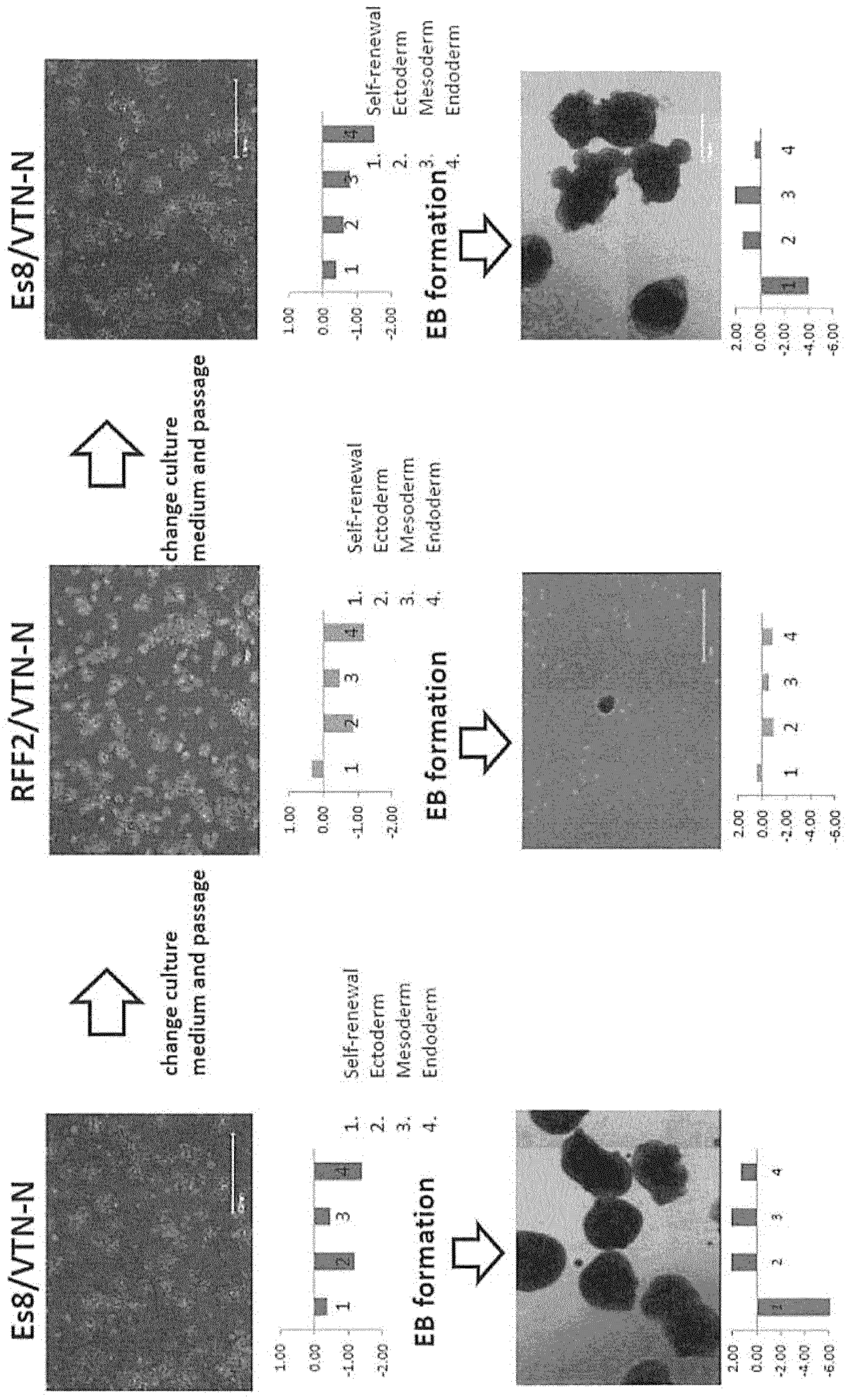
Es8/VTN-N
change culture medium and passage
RFF2/VTN-N
change culture medium and passage
Es8/VTN-N
1. Self-renewal
2. Ectoderm
3. Mesoderm
4. Endoderm
EB formation
1.00
0.00
-1.00
-2.00
2.00
0.00
-2.00
-4.00
-6.00

Fig. 3

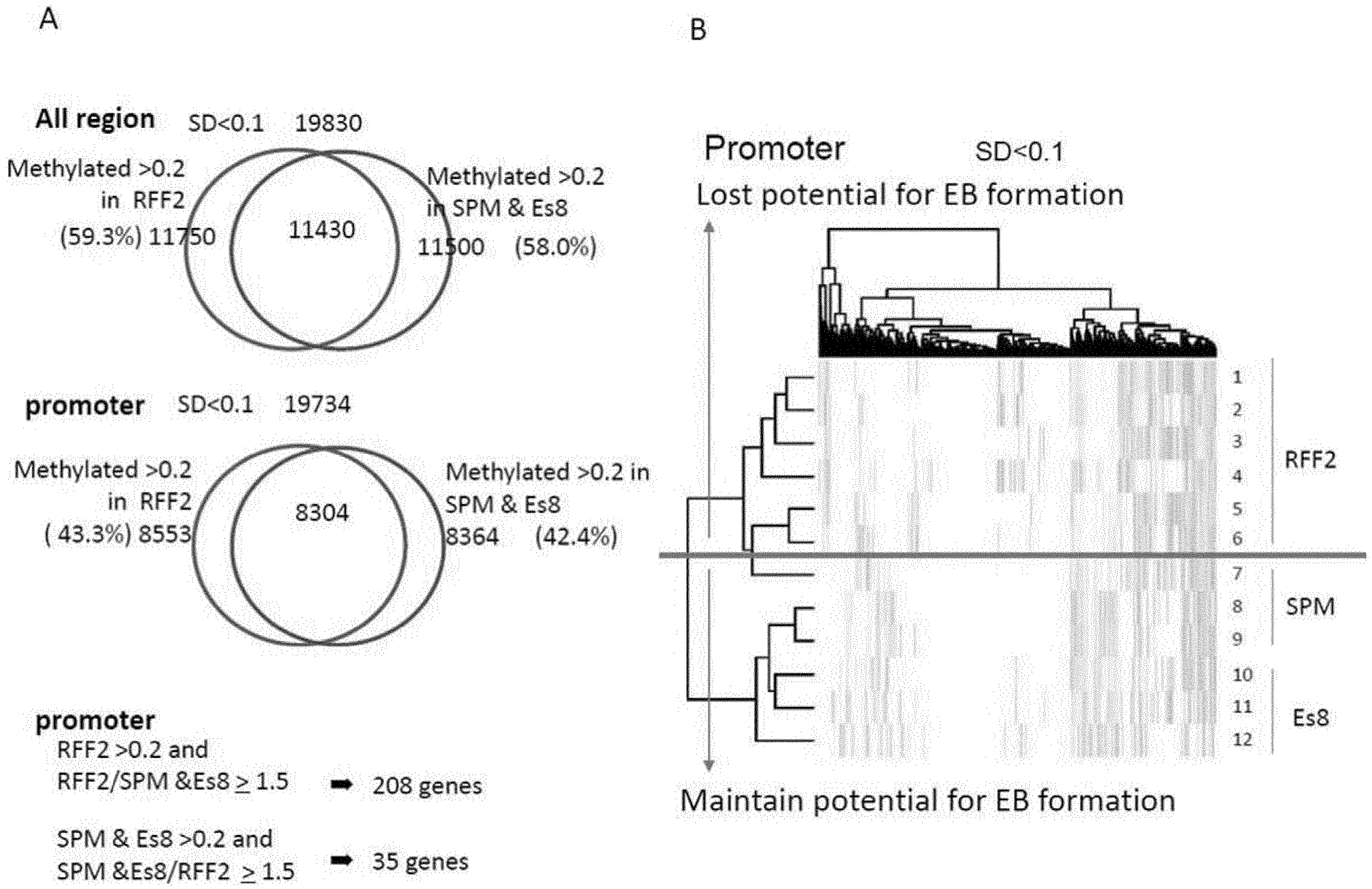
A
All region SD<0.1 19830
Methylated >0.2 in RFF2 (59.3%) 11750
11430
Methylated >0.2 in SPM & Es8 11500 (58.0%)
promoter SD<0.1 19734
Methylated >0.2 in RFF2 ( 43.3%) 8553
8304
Methylated >0.2 in SPM & Es8 8364 (42.4%)
promoter
RFF2 >0.2 and RFF2/SPM &Es8 ≥ 1.5 ➡ 208 genes
SPM & Es8 >0.2 and SPM &Es8/RFF2 ≥ 1.5 ➡ 35 genes
B
Promoter SD<0.1
Lost potential for EB formation
1
2
3
4
5
6
7
8
9
10
11
12
RFF2
SPM
Es8
Maintain potential for EB formation

Fig. 4

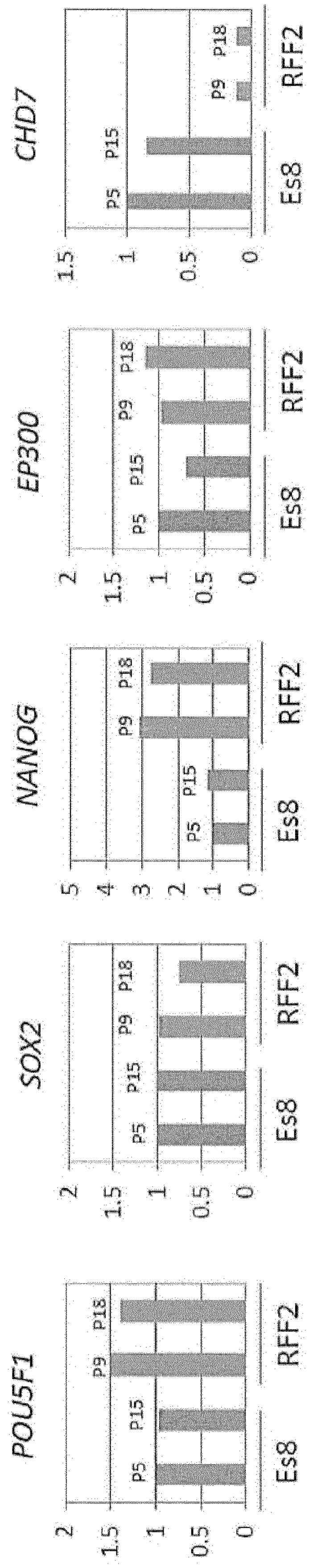
POU5F1
SOX2
NANOG
EP300
CHD7
P5
P15
P9
P18
Es8
RFF2
0
0.5
1
1.5
2
3
4
5

Fig. 5

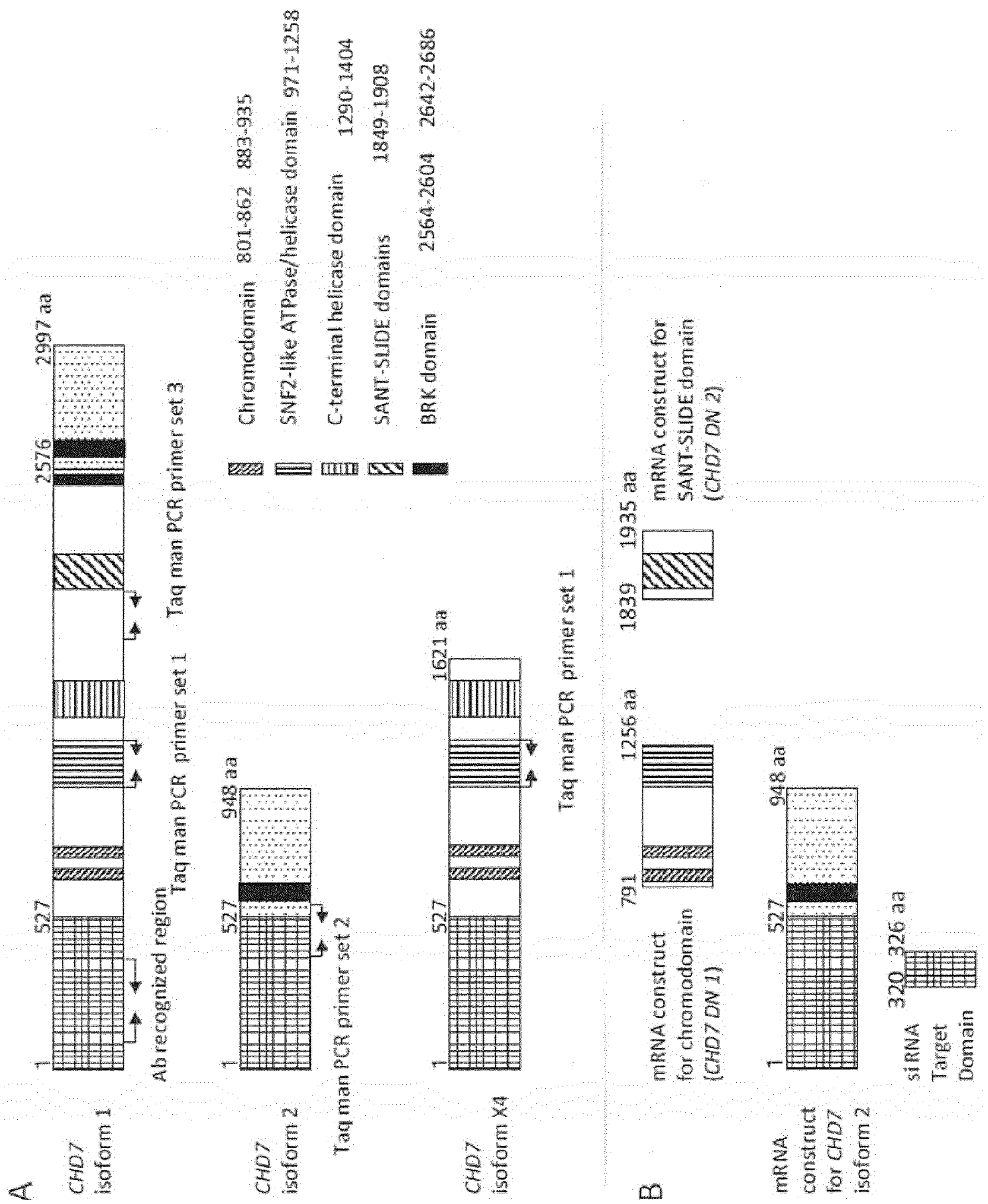
A
CHD7 isoform 1
1
527
2576
2997 aa
Ab recognized region
Taq man PCR primer set 1
Taq man PCR primer set 3
CHD7 isoform 2
948 aa
Taq man PCR primer set 2
CHD7 isoform X4
1621 aa
Chromodomain 801-862 883-935
SNF2-like ATPase/helicase domain 971-1258
C-terminal helicase domain 1290-1404
SANT-SLIDE domains 1849-1908
BRK domain 2564-2604 2642-2686
B
mRNA construct for chromodomain (CHD7 DN 1)
791
1256 aa
1839
1935 aa
mRNA construct for SANT-SLIDE domain (CHD7 DN 2)
mRNA construct for CHD7 isoform 2
si RNA Target Domain
320
326 aa

Fig. 6

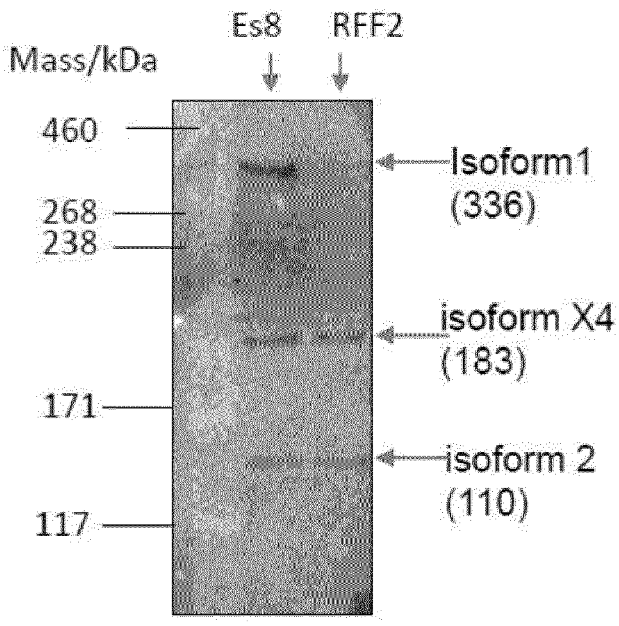
Es8
RFF2
Mass/kDa
460
268
238
171
117
Isoform1
(336)
isoform X4
(183)
isoform 2
(110)

Fig. 7

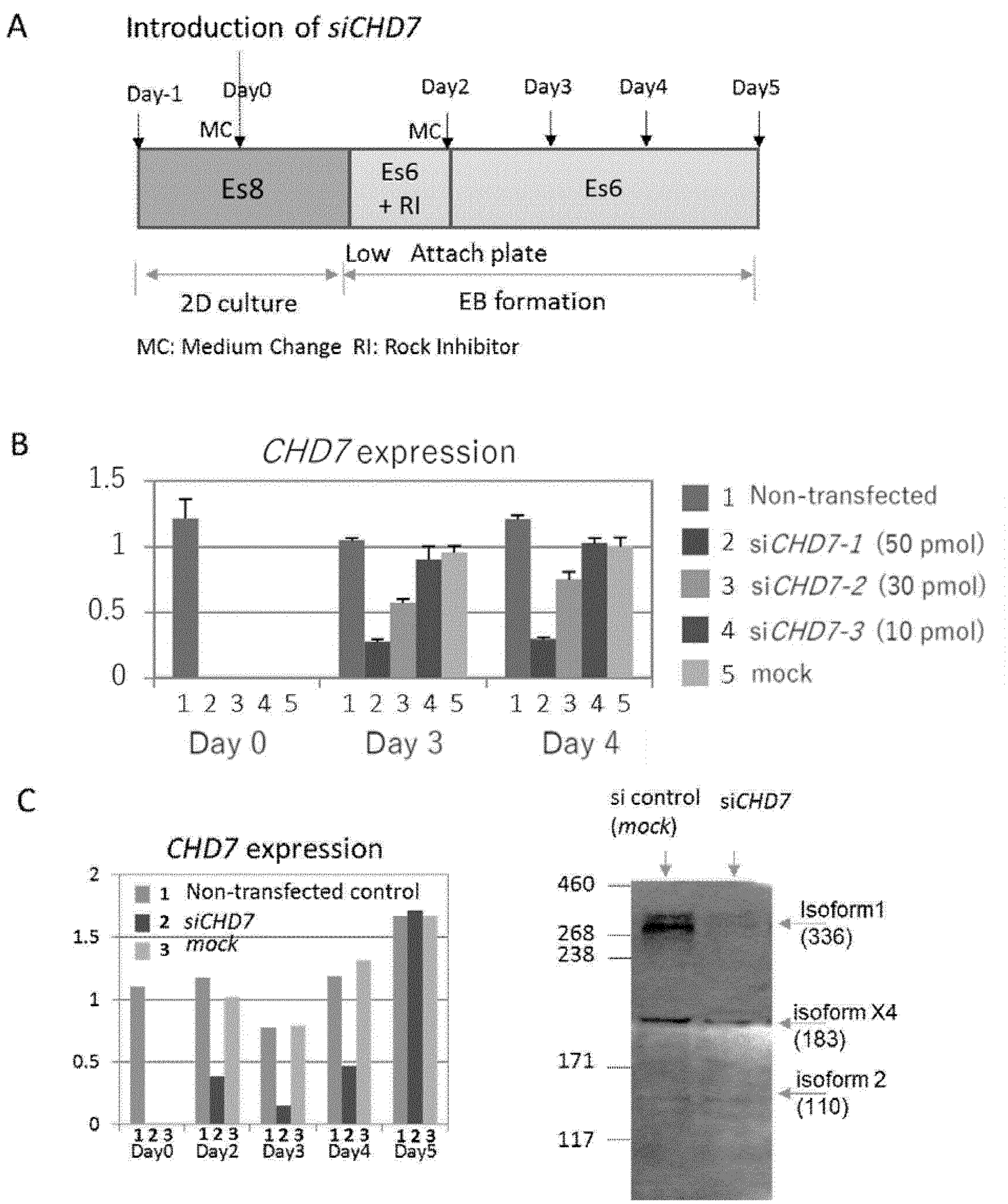
A
Introduction of siCHD7
Day-1
Day0
Day2
Day3
Day4
Day5
MC
MC
Es8
Es6
+ RI
Es6
Low Attach plate
2D culture
EB formation
MC: Medium Change RI: Rock Inhibitor
B
CHD7 expression
1.5
1
0.5
0
1 Non-transfected
2 siCHD7-1 (50 pmol)
3 siCHD7-2 (30 pmol)
4 siCHD7-3 (10 pmol)
5 mock
1 2 3 4 5
Day 0
Day 3
Day 4
C
CHD7 expression
2
1.5
1
0.5
0
1 Non-transfected control
2 siCHD7
3 mock
1 2 3
Day0
Day2
Day3
Day4
Day5
si control (mock)
siCHD7
460
268
238
171
117
Isoform1 (336)
isoform X4 (183)
isoform 2 (110)

Fig. 8

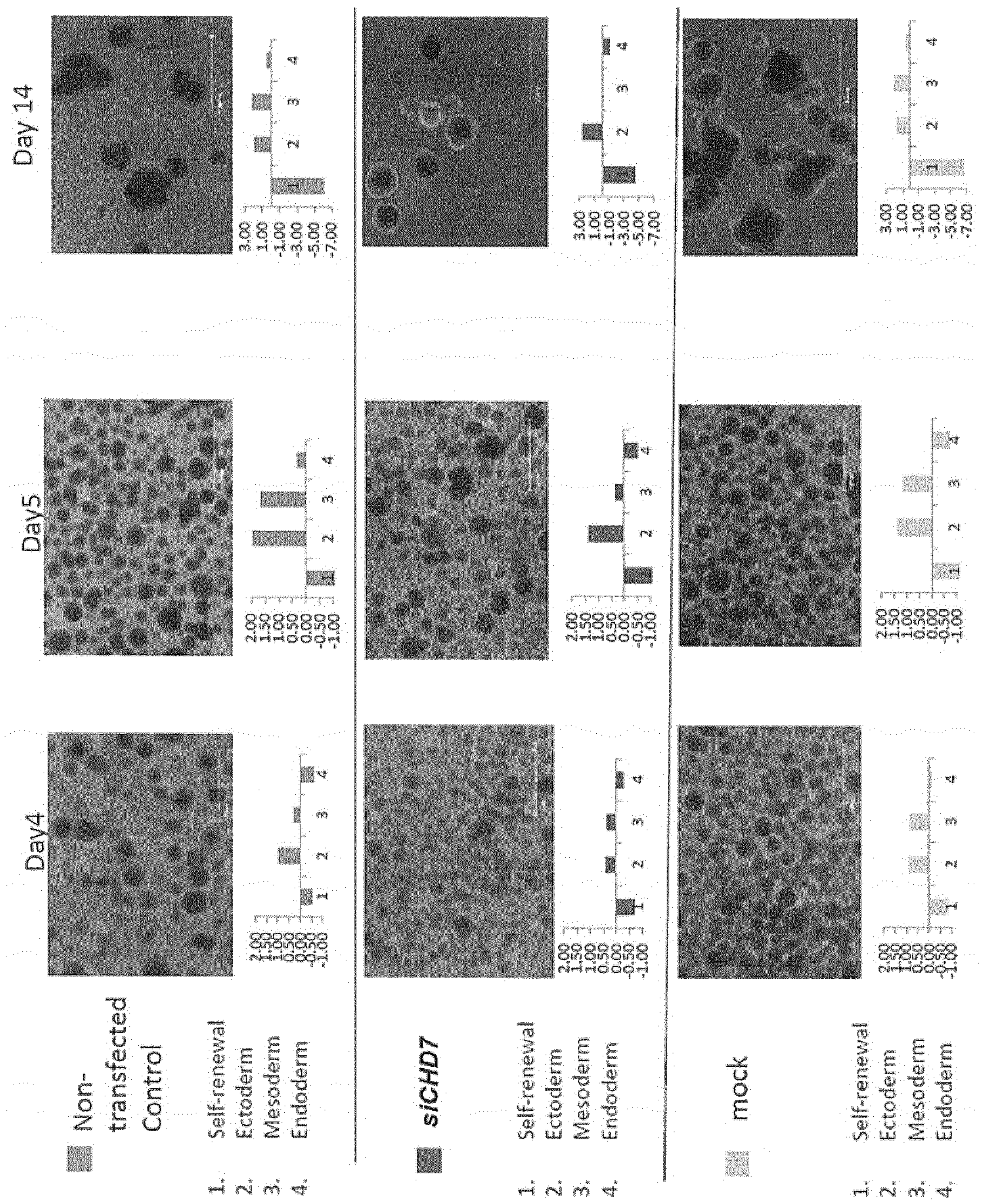
Day4
Day5
Day 14
Non-transfected Control
siCHD7
mock
1. Self-renewal
2. Ectoderm
3. Mesoderm
4. Endoderm
2.00
1.50
1.00
0.50
0.00
-0.50
-1.00
3.00
1.00
-1.00
-3.00
-5.00
-7.00

Fig. 9

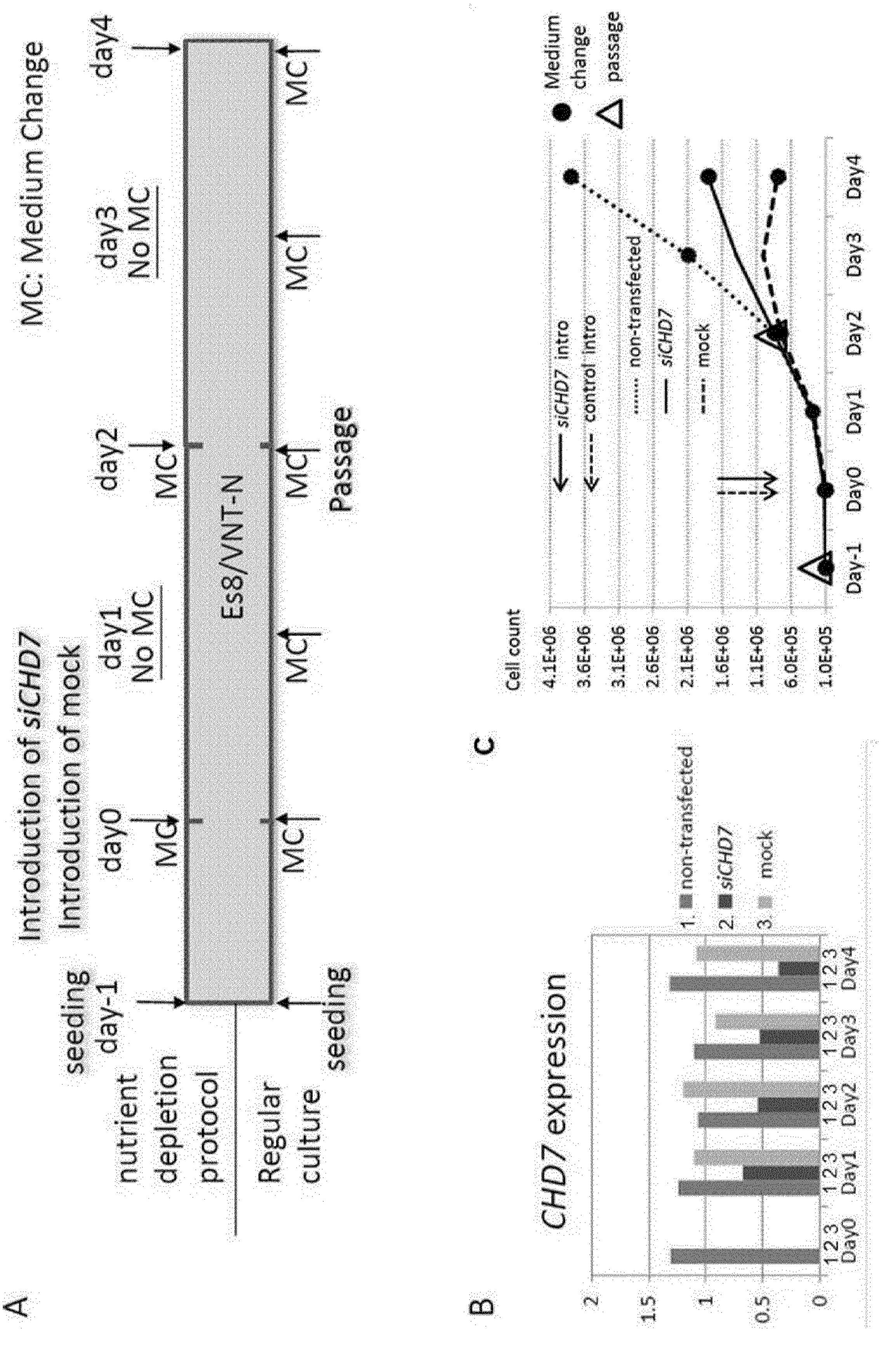
A
nutrient depletion protocol
Regular culture
seeding
day-1
Introduction of siCHD7
Introduction of mock
day0
MC
day1
No MC
day2
day3
No MC
day4
MC: Medium Change
Es8/VNT-N
Passage
B
CHD7 expression
2
1.5
1
0.5
0
Day0
Day1
Day2
Day3
Day4
1. non-transfected
2. siCHD7
3. mock
C
Cell count
4.1E+06
3.6E+06
3.1E+06
2.6E+06
2.1E+06
1.6E+06
1.1E+06
6.0E+05
1.0E+05
Day-1
siCHD7 intro
control intro
non-transfected
siCHD7
mock
Medium change
passage

Fig. 10

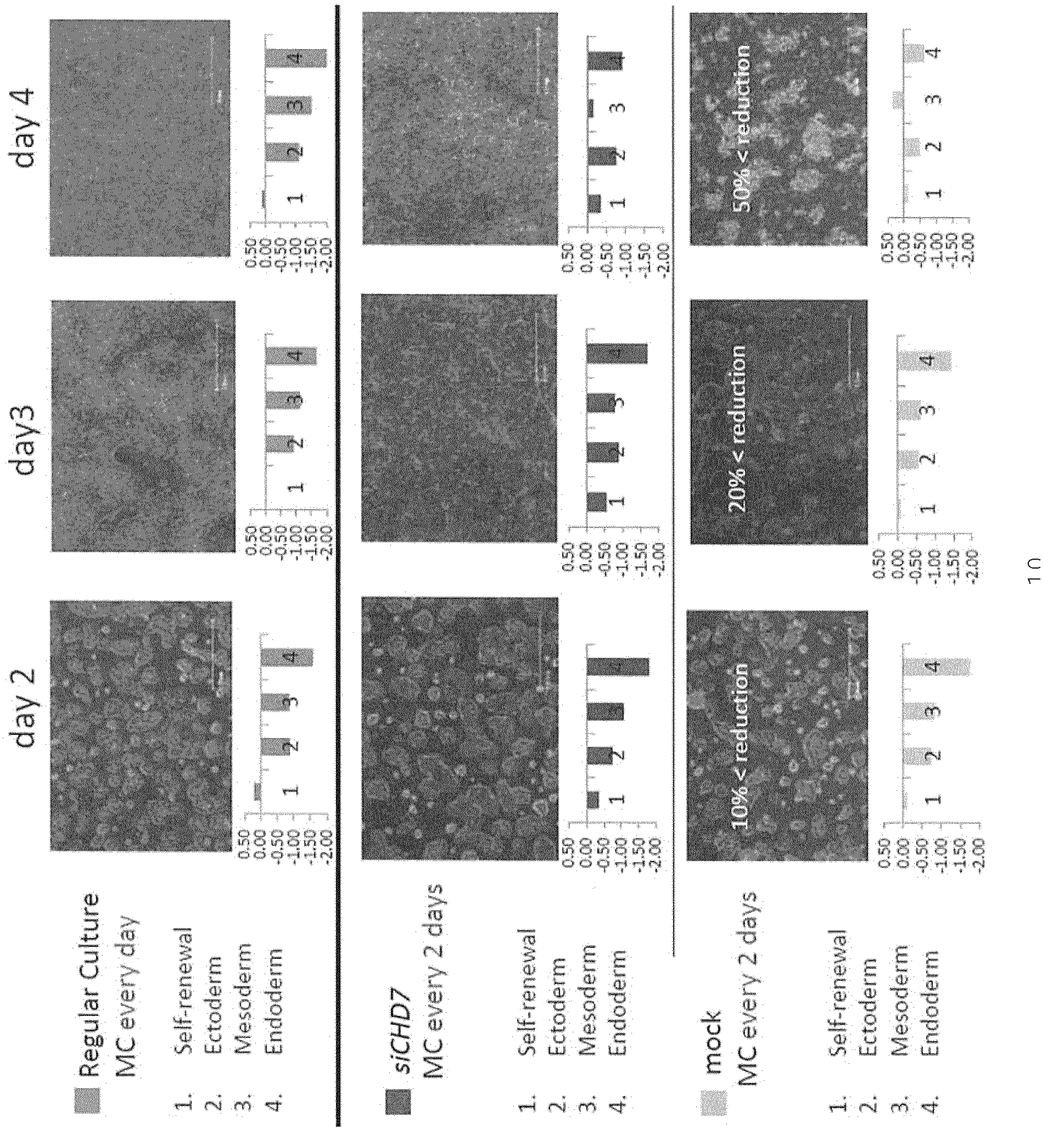
day 2
day3
day 4
Regular Culture
MC every day
1. Self-renewal
2. Ectoderm
3. Mesoderm
4. Endoderm
siCHD7
MC every 2 days
1. Self-renewal
2. Ectoderm
3. Mesoderm
4. Endoderm
mock
MC every 2 days
1. Self-renewal
2. Ectoderm
3. Mesoderm
4. Endoderm
10% < reduction
20% < reduction
50% < reduction

Fig. 11

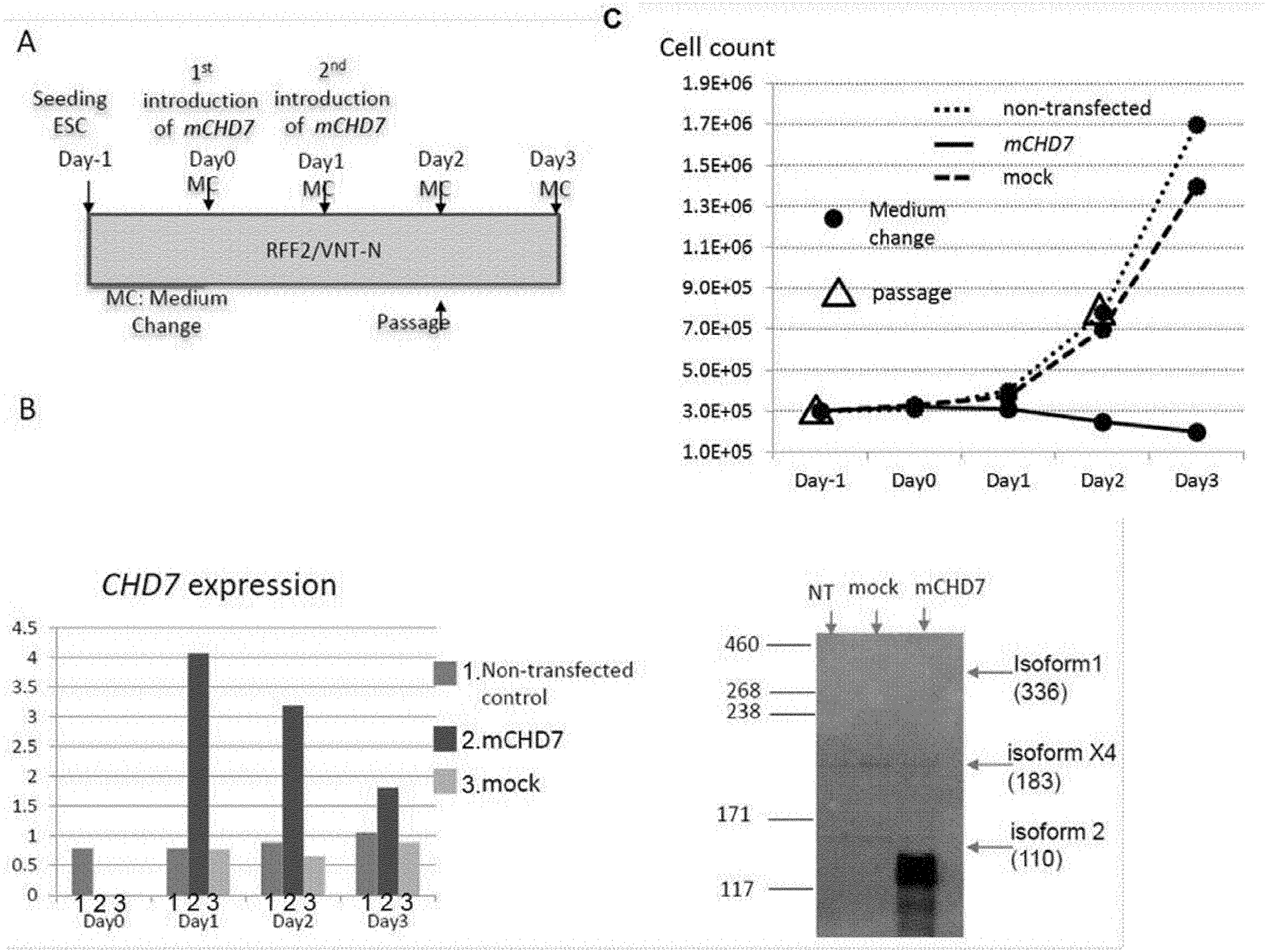
A
Seeding ESC
1st introduction of mCHD7
2nd introduction of mCHD7
Day-1
Day0 MC
Day1 MC
Day2 MC
Day3 MC
RFF2/VNT-N
MC: Medium Change
Passage
B
CHD7 expression
4.5
4
3.5
3
2.5
2
1.5
1
0.5
0
1 2 3 Day0
1 2 3 Day1
1 2 3 Day2
1 2 3 Day3
1.Non-transfected control
2.mCHD7
3.mock
C
Cell count
1.9E+06
1.7E+06
1.5E+06
1.3E+06
1.1E+06
9.0E+05
7.0E+05
5.0E+05
3.0E+05
1.0E+05
Day-1
Day0
Day1
Day2
Day3
non-transfected
mCHD7
mock
Medium change
passage
NT
mock
mCHD7
460
268
238
171
117
Isoform1 (336)
isoform X4 (183)
isoform 2 (110)

Fig. 12

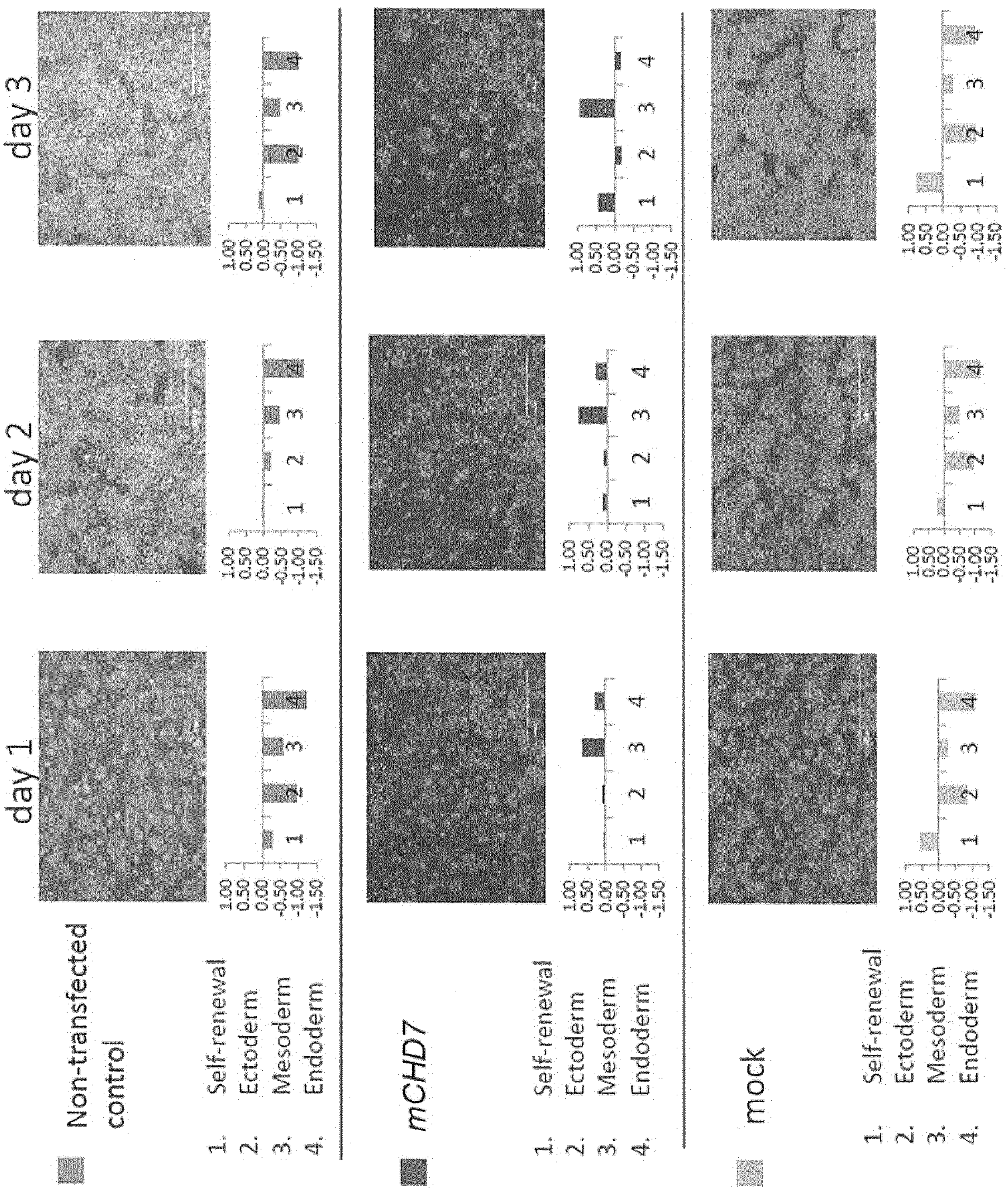
day 1
day 2
day 3
Non-transfected control
mCHD7
mock
1. Self-renewal
2. Ectoderm
3. Mesoderm
4. Endoderm
1.00
0.50
0.00
-0.50
-1.00
-1.50

Fig. 13

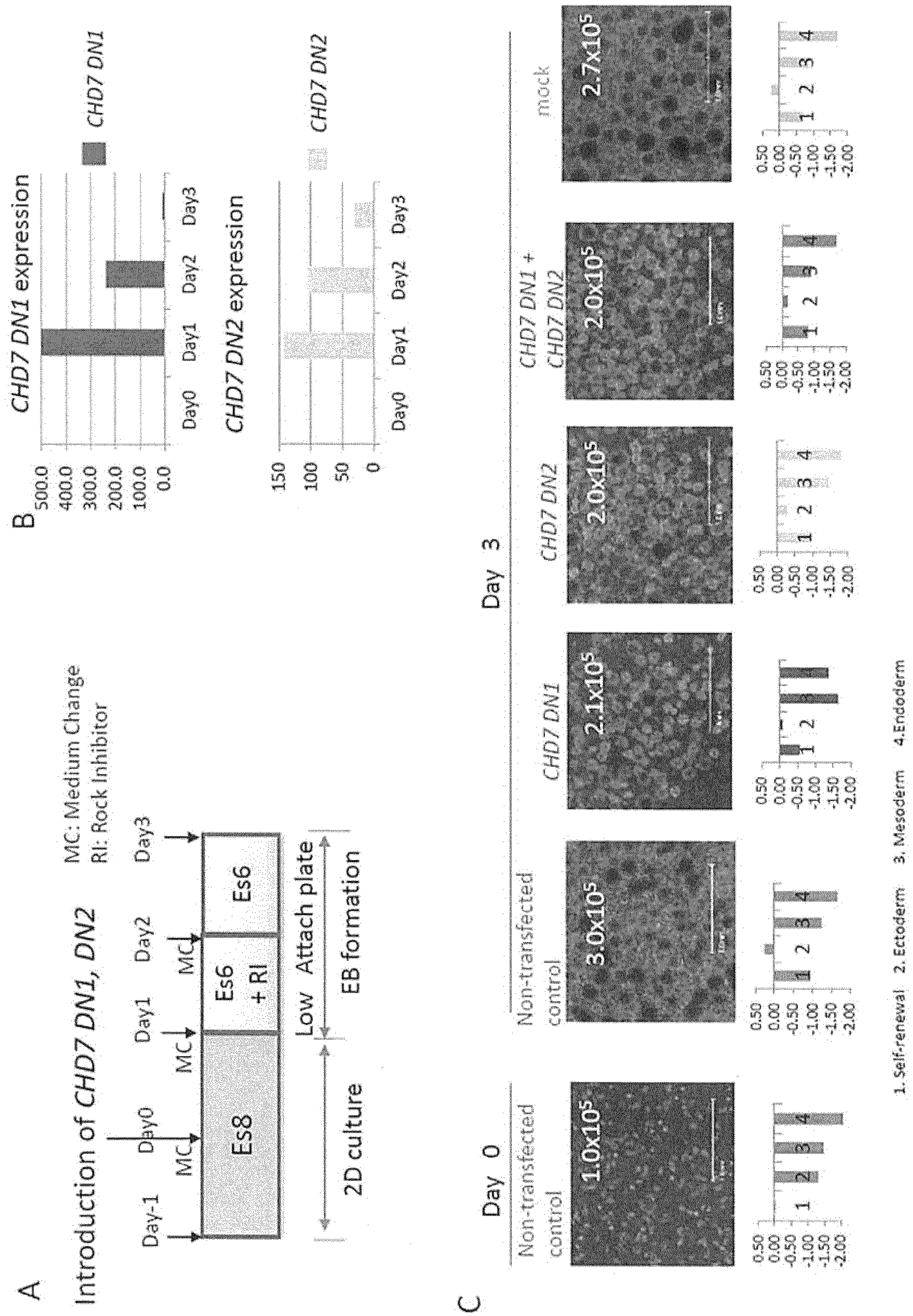
A
Introduction of CHD7 DN1, DN2
MC: Medium Change
RI: Rock Inhibitor
Day-1
Day0
Day1
Day2
Day3
MC
MC
MC
Es8
Es6 + RI
Es6
2D culture
Low Attach plate
EB formation
B
CHD7 DN1 expression
500.0
400.0
300.0
200.0
100.0
0.0
Day0
Day1
Day2
Day3
CHD7 DN1
CHD7 DN2 expression
150
100
50
0
CHD7 DN2
C
Day 0
Day 3
Non-transfected control
1.0x10^5
3.0x10^5
CHD7 DN1
2.1x10^5
CHD7 DN2
2.0x10^5
CHD7 DN1 + CHD7 DN2
2.0x10^5
mock
2.7x10^5
0.50
0.00
-0.50
-1.00
-1.50
-2.00
1
2
3
4
1. Self-renewal
2. Ectoderm
3. Mesoderm
4. Endoderm

Fig. 14

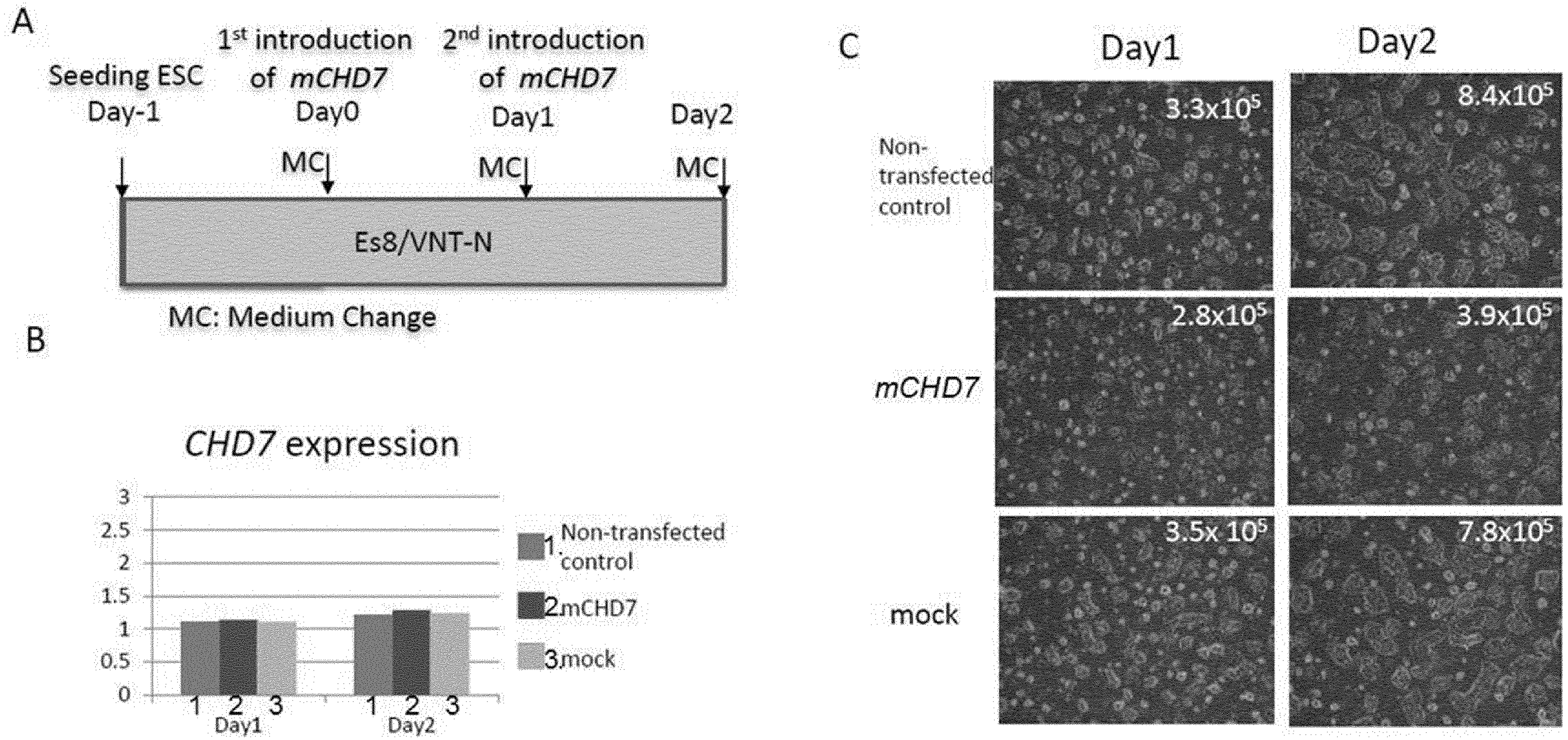
A
Seeding ESC
Day-1
1st introduction
of mCHD7
Day0
2nd introduction
of mCHD7
Day1
Day2
MC
MC
MC
Es8/VNT-N
MC: Medium Change
B
CHD7 expression
3
2.5
2
1.5
1
0.5
0
1 2 3
Day1
1 2 3
Day2
1. Non-transfected control
2.mCHD7
3.mock
C
Day1
Day2
Non-transfected control
mCHD7
mock
3.3x10^5
8.4x10^5
2.8x10^5
3.9x10^5
3.5x 10^5
7.8x10^5

Fig. 15

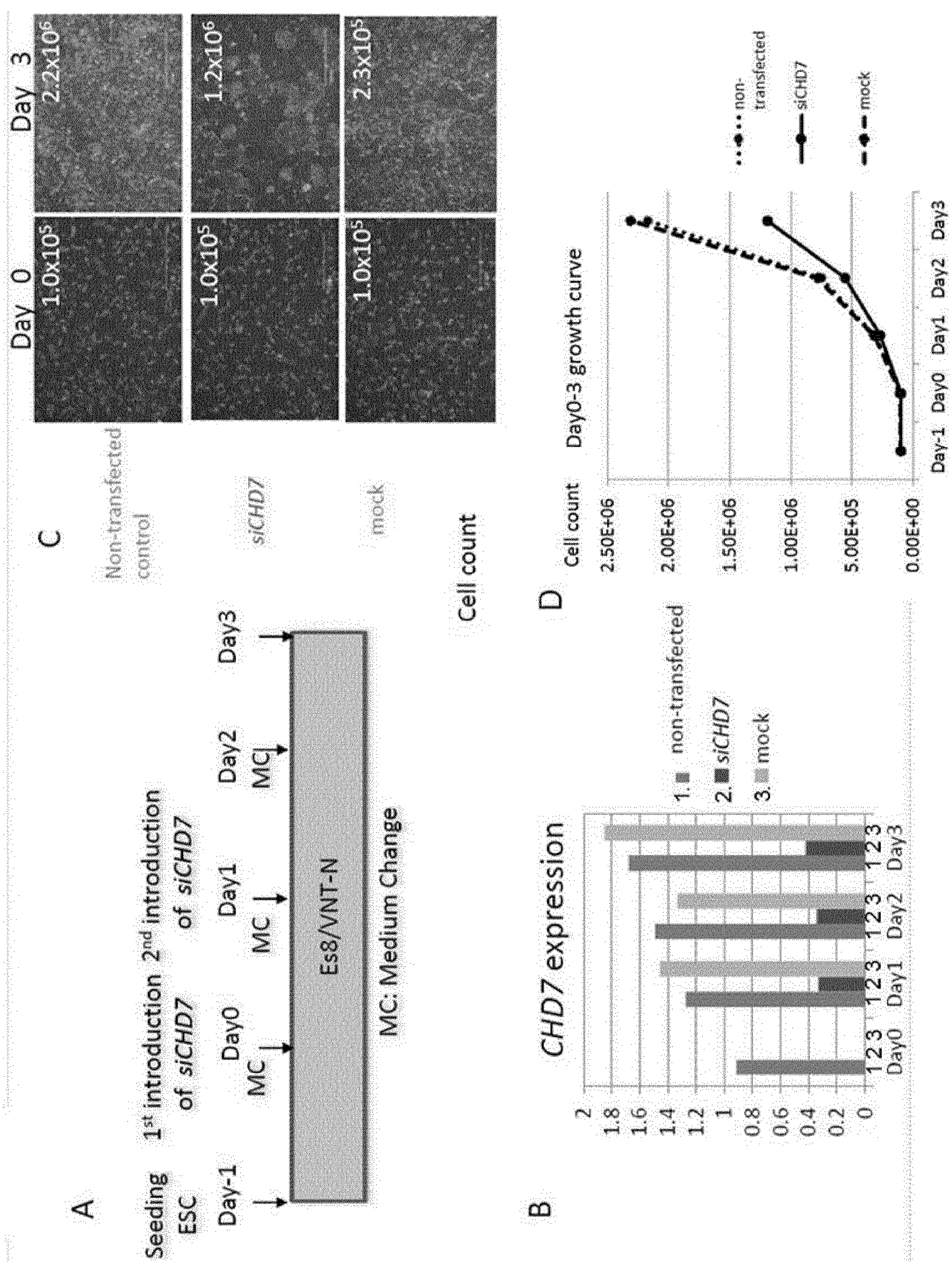
A
Seeding ESC
1st introduction of siCHD7
2nd introduction of siCHD7
Day-1
Day0
Day1
Day2
Day3
MC
Es8/VNT-N
MC: Medium Change
B
CHD7 expression
1. non-transfected
2. siCHD7
3. mock
C
Day 0
Day 3
Non-transfected control
siCHD7
mock
1.0x10^5
2.2x10^6
1.2x10^6
2.3x10^6
Cell count
D
Day0-3 growth curve
Cell count
non-transfected
siCHD7
mock

Fig. 16

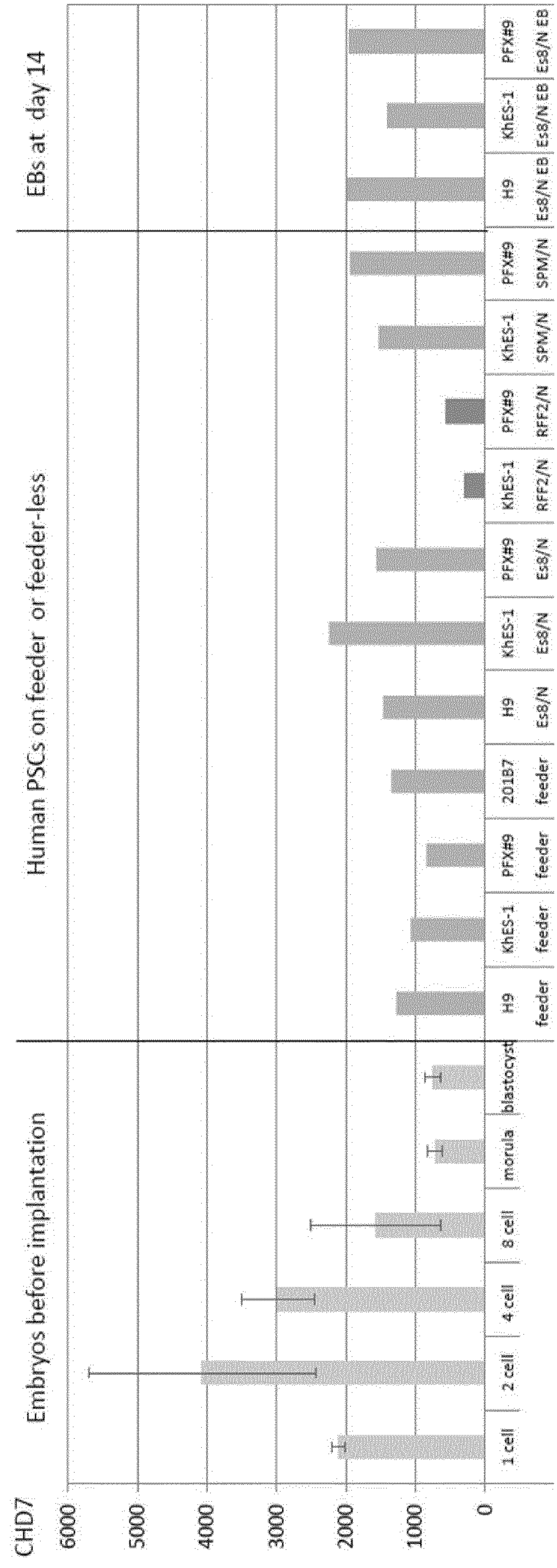
CHD7
6000
5000
4000
3000
2000
1000
0
Embryos before implantation
Human PSCs on feeder or feeder-less
EBs at day 14
1 cell
2 cell
4 cell
8 cell
morula
blastocyst
H9 feeder
KhES-1 feeder
PFX#9 feeder
201B7 feeder
H9 Es8/N
KhES-1 Es8/N
PFX#9 Es8/N
KhES-1 RFF2/N
PFX#9 RFF2/N
KhES-1 SPM/N
PFX#9 SPM/N
H9 Es8/N EB
KhES-1 Es8/N EB
PFX#9 Es8/N EB

Fig. 17

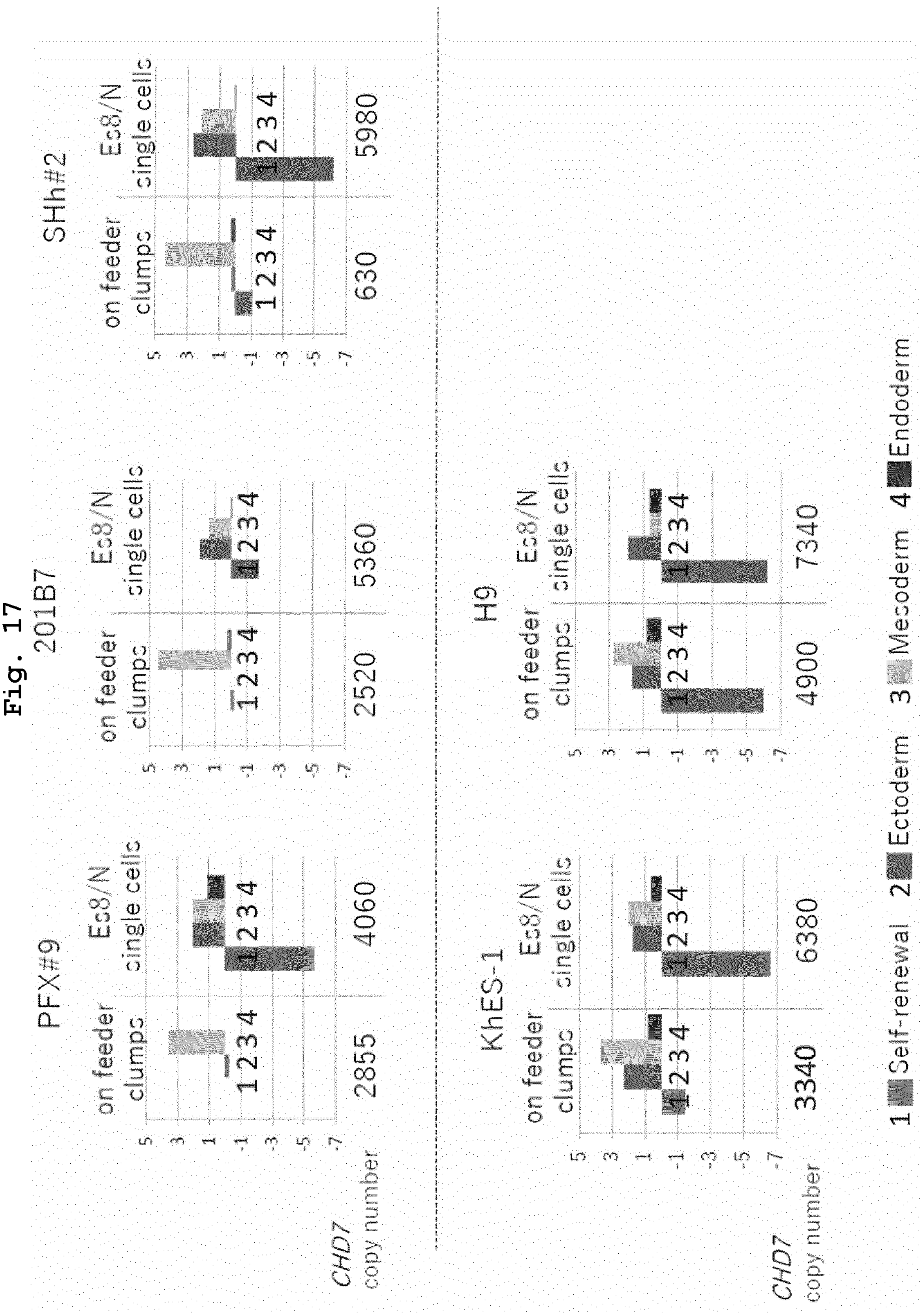

PFX#9
201B7
SHh#2
KhES-1
H9
on feeder clumps
Es8/N single cells
1 2 3 4
5
3
1
-1
-3
-5
-7
*CHD7* copy number
2855
4060
2520
5360
630
5980
3340
6380
4900
7340
1 Self-renewal
2 Ectoderm
3 Mesoderm
4 Endoderm

Fig. 18

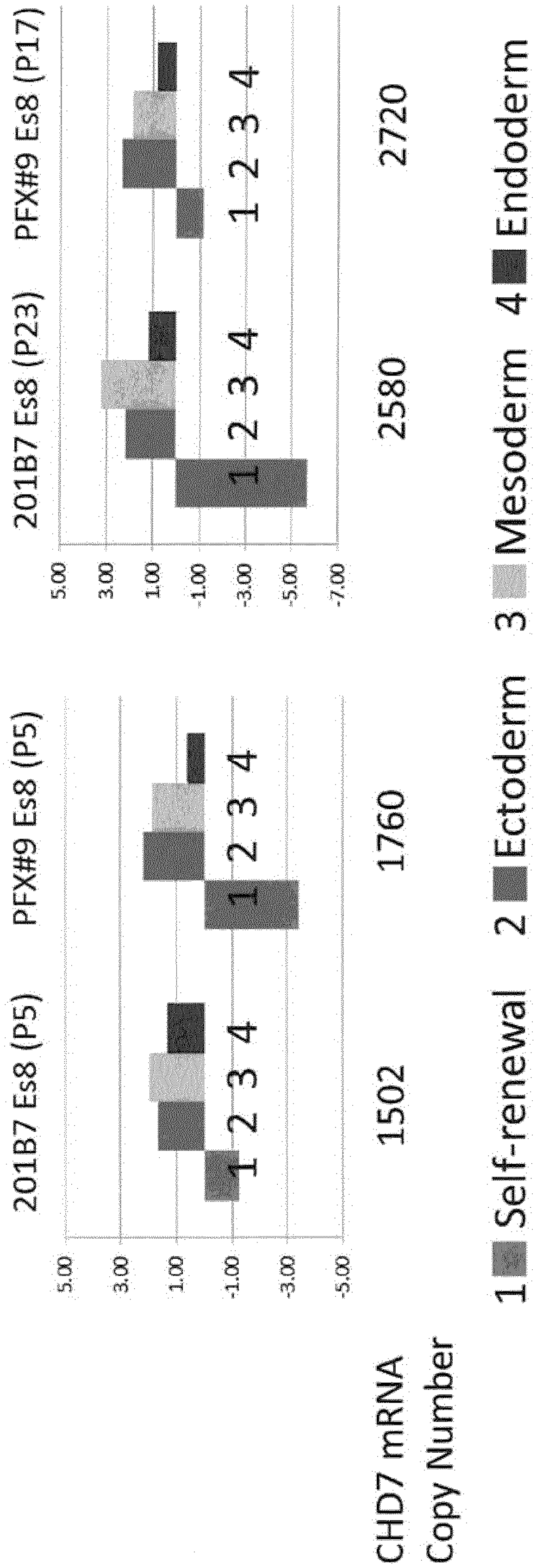
CHD7 mRNA
Copy Number
201B7 Es8 (P5)
PFX#9 Es8 (P5)
1502
1760
201B7 Es8 (P23)
PFX#9 Es8 (P17)
2580
2720
5.00
3.00
1.00
-1.00
-3.00
-5.00
-7.00
1 2 3 4
1 Self-renewal
2 Ectoderm
3 Mesoderm
4 Endoderm

Fig. 19

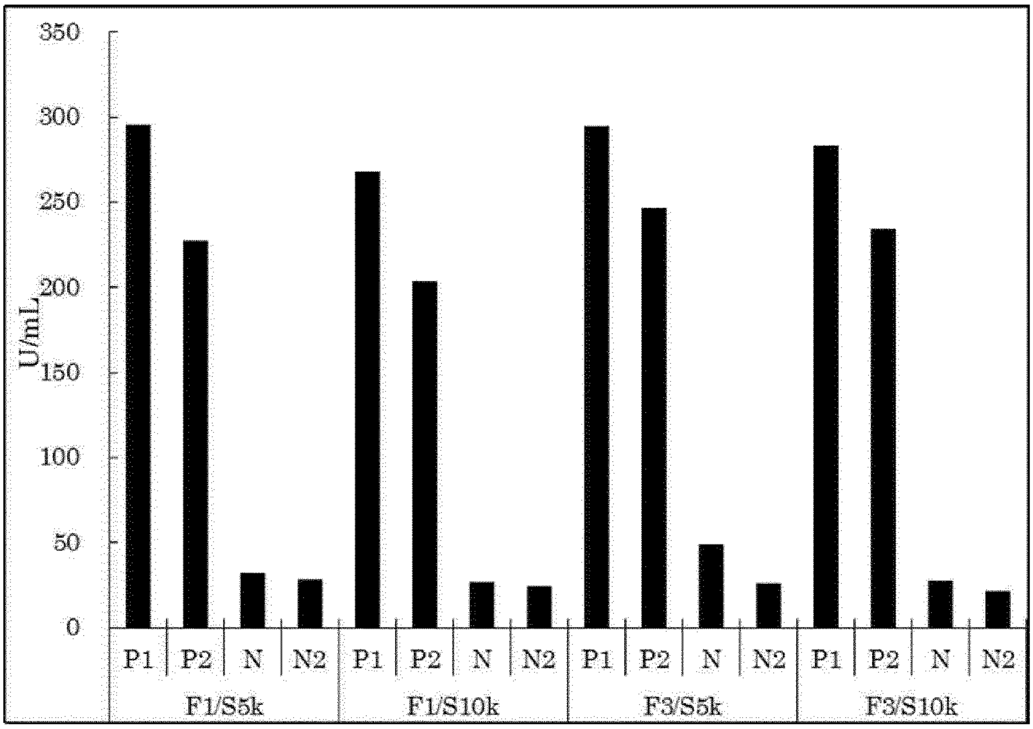
350
300
250
200
150
100
50
0
U/mL
P1
P2
N
N2
F1/S5k
F1/S10k
F3/S5k
F3/S10k

Fig. 20

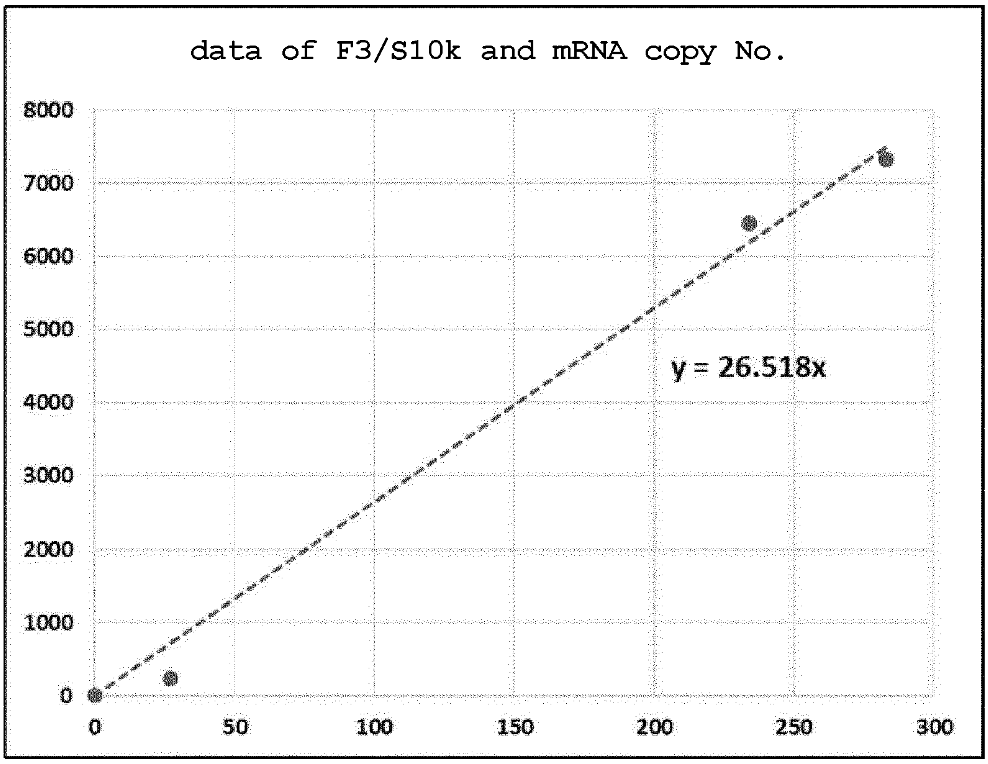
data of F3/S10k and mRNA copy No.
8000
7000
6000
5000
4000
3000
2000
1000
0
50
100
150
200
250
300
y = 26.518x

## INTERNATIONAL SEARCH REPORT

International application No. PCT/JP2018/021430

A. CLASSIFICATION OF SUBJECT MATTER
Int.Cl. C12N15/09(2006.01)i, C12N5/0735(2010.01)i, C12N5/10(2006.01)i, C12Q1/02(2006.01)i

According to International Patent Classification (IPC) or to both national classification and IPC

B. FIELDS SEARCHED

Minimum documentation searched (classification system followed by classification symbols)
Int.Cl. C12N15/09, C12N5/0735, C12N5/10, C12Q1/02

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/MEDLINE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| Y<br>A | JP 2014-523735 A (KYOTO UNIVERSITY) 18 September 2014, claims, examples & US 2014/0309131 A1, claims, examples & WO 2013/014929 A1 & EP 2737064 A1 | 1-5, 11-15<br>6-10 |
| Y<br>A | WO 2012/115270 A1 (KEIO UNIVERSITY) 30 August 2012, claims, examples (Family: none) | 1-5, 11-15<br>6-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 August 2018 (15.08.2018) | 28 August 2018 (28.08.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/021430

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | SCHNETZ, M. P., et al., "CHD7 targets active gene enhancer elements to modulate ES cell-specific gene expression", PLoS Genet., 15 July 2010, vol. 6, no. 7, e1001023, pp. 1-15, in particular, page 7, left column, paragraph [0002] | 1-5, 11-15<br>6-10 |
| P, X | YAMAMOTO, T., et al., "Differentiation potential of Pluripotent Stem Cells correlates to the level of CHD7", Sci. Rep., 10 January 2018, vol. 8, no. 1, 241, pp. 1-12, in particular, abstract | 1-15 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011530273 W
- JP 2012527887 W
- WO 2007069666 A
- WO 2008118820 A
- WO 2009007852 A
- WO 2009032194 A
- WO 2009058413 A
- WO 2009057831 A
- WO 2009075119 A
- WO 2009079007 A
- WO 2009091659 A
- WO 2009101084 A
- WO 2009101407 A
- WO 2009102983 A
- WO 2009114949 A
- WO 2009117439 A
- WO 2009126250 A
- WO 2009126251 A
- WO 2009126655 A
- WO 2009157593 A
- WO 2010009015 A
- WO 2010033906 A
- WO 2010033920 A
- WO 2010042800 A
- WO 2010050626 A
- WO 2010056831 A
- WO 2010068955 A
- WO 2010098419 A
- WO 2010102267 A
- WO 2010111409 A
- WO 2010111422 A
- WO 2010115050 A
- WO 2010124290 A
- WO 2010147395 A
- WO 2010147612 A
- US 5843780 A
- WO 2005100548 A
- JP 2017120024 A
- JP 2017237420 A


### Non-patent literature cited in the description


- **TAKENAKA et al.** *PLoS One,* 2015, vol. 10 (6
- **HUANGFU D et al.** *Nat. Biotechnol.,* 2008, vol. 26, 795-797
- **SHI Y et al.** *Cell Stem Cell,* 2008, vol. 2, 525-528
- **EMINLI S et al.** *Stem Cells,* 2008, vol. 26, 2467-2474
- **HUANGFU D et al.** *Nat Biotechnol.,* 2008, vol. 26, 1269-1275
- **SHI Y et al.** *Cell Stem Cell,* 2008, vol. 3, 568-574
- **ZHAO Y et al.** *Cell Stem Cell,* 2008, vol. 3, 475-479
- **MARSON A.** *Cell Stem Cell,* 2008, vol. 3, 132-135
- **FENG B et al.** *Nat Cell Biol.,* 2009, vol. 11, 197-203
- **R.L. JUDSON et al.** *Nat. Biotech.,* 2009, vol. 27, 459-461
- **LYSSIOTIS CA et al.** *Proc Natl Acad Sci U S A.,* 2009, vol. 106, 8912-8917
- **KIM JB et al.** *Nature,* 2009, vol. 461, 649-643
- **ICHIDA JK et al.** *Cell Stem Cell,* 2009, vol. 5, 491-503
- **HENG JC et al.** *Cell Stem Cell,* 2010, vol. 6, 167-74
- **HAN J et al.** *Nature,* 2010, vol. 463, 1096-100
- **MALI P et al.** *Stem Cells,* 2010, vol. 28, 713-720
- **MAEKAWA M et al.** *Nature,* 2011, vol. 474, 225-9
- **THOMSON J A et al.** *Proc Natl. Acad. Sci. USA.,* 1995, vol. 92, 7844-7848
- **THOMSON J A et al.** *Science,* 1998, vol. 282, 1145-1147
- **H. SUEMORI et al.** *Biochem. Biophys. Res. Commun.,* 2006, vol. 345, 926-932
- **M. UENO et al.** *Proc. Natl. Acad. Sci. USA,* 2006, vol. 103, 9554-9559
- **H. SUEMORI et al.** *Dev. Dyn.,* 2001, vol. 222, 273-279
- **H. KAWASAKI et al.** *Proc. Natl. Acad. Sci. USA,* 2002, vol. 99, 1580-1585
- **KLIMANSKAYA I et al.** *Nature,* 2006, vol. 444, 481-485
- **Y. MATSUI et al.** *Cell,* 1992, vol. 70, 841-847
- **J. L. RESNICK et al.** *Nature,* 1992, vol. 359, 550-551

- **J. B. CIBELLI et al.** *Nature Biotechnol.,* 1998, vol. 16, 642-646
- **KIYOKA WAKAYAMA et al.** *Experimental Medicine,* 2008, vol. 26 (5), 47-52
- Radioimmunoassay. Kodansha, 1974
- Supplementary volume of Radioimmunoassay. Kodansha, 1979
- enzyme immunoassay. Igaku-Shoin, 1978
- **EIJI ISHIKAWA et al.** enzyme immunoassay. Igaku-Shoin, 1979
- enzyme immunoassay. Igaku-Shoin, 1987
- *Methods in ENZYMOLOGY,* vol. 70
- *Immunochemical Techniques,* vol. 73
- *Immunochemical Techniques,* vol. 74
- *Immunochemical Techniques,* vol. 84
- *Immunochemical Techniques (Part D: Selected Immunoassays),* vol. 92
- *Immunochemical Techniques (Part E: Monoclonal Antibodies and General Immunoassay Methods),* vol. 121
- Immunochemical Techniques (Part I: Hybridoma Technology and Monoclonal Antibodies). Academic Press
- **NAVARRO-ALVAREZ et al.** *Cell Transplant,* 2008, vol. 17 (1-2), 111-119
- **LEVENSTEIN et al.** *Stem Cell,* March 2006, vol. 24 (3), 568-74
- **NISHISHITA et al.** *PLoS One,* 2012, vol. 7 (6), e38389
- **NISHISHITA et al.** *PLoS One,* 2012, vol. 7, 6
- **TAKENAKA et al.** *PLoS ONE,* 2015, vol. 10 (6), e0129855
- **CHEN et al.** *Nat Methods,* 2011, vol. 8 (5), 424-429
- **RYAN et al.** *Embo j,* 2011, vol. 30 (13), 2596-2609
- **SCHNETZ et al.** *Genome Res,* 2009, vol. 19 (4), 590-601
- **COLIN et al.** *BMC Res Notes,* 2010, vol. 3, 252
- **ALLEN et al.** *J Mol Biol,* 2007, vol. 371 (5), 1135-1140
- **VANDER HEIDEN et al.** *Science,* 2015, vol. 324 (5930), 1029-1033
- **YANES et al.** *Nat Chem Biol,* 2010, vol. 6 (6), 411-417
- **MOUSSAIEFF et al.** *Cell Metab,* 2015, vol. 21 (3), 392-402

SEQUENCE LISTING

<110>  FOUNDATION FOR BIOMEDICAL RESEARCH AND INNOVATION AT KOBE

<120>  PREDICTION METHOD FOR POTENTIAL FOR DIFFERENTIATION IN PLURIPOTENT STEM CELL AND REAGENT THEREFOR

<130>  092685

<150>  JP 2017-120024
<151>  2017-06-19

<150>  JP 2017-237420
<151>  2017-12-12

<160>  30

<170>  PatentIn version 3.5

<210>  1
<211>  11589
<212>  DNA
<213>  Homo sapiens

<220>
<221>  CDS
<222>  (493)..(9486)

<400>  1
```
gcggcggcgg cggcggcggc ggcggcagcg gcggcggcgg cggcggcgcg ggggttgagt       60

cgtggtggtg cggacgcgct cgtgctcggg aactatcgga ttaaacttga atcgagtgaa      120

attacacaaa ggagcgccgc ggaggaggcg gcccggggac ccggacaccc tgaaactcac      180

cagagacccg ttcgccccccg gccaactccg tgcccgtgga ttcagccccc tggccgcagc      240

tgccgagcca actccggagc ccgctctgcg ttttgttttc ccctcggcac taggcagcgg      300

aggagcccga ccgacccgga cctatatcca gactttgcct gacactgcag ggtccaagag      360

aattaaagaa atatggaatg acatgaagaa gattagttaa ggattatagg ctttgagggc      420

aaacacctca gtgaagtgaa gcacaggcaa gctcctgagc tgtggtttgg aggagccgtg      480

tgttggaaga ag atg gca gat cca gga atg atg agt ctt ttt ggc gag gat      531
              Met Ala Asp Pro Gly Met Met Ser Leu Phe Gly Glu Asp
              1               5                   10

ggg aat att ttc agt gaa ggt ctt gaa ggc ctc gga gaa tgt ggt tac      579
Gly Asn Ile Phe Ser Glu Gly Leu Glu Gly Leu Gly Glu Cys Gly Tyr
    15                  20                  25

ccg gaa aat cca gta aat cct atg ggt cag caa atg cca ata gac caa      627
Pro Glu Asn Pro Val Asn Pro Met Gly Gln Gln Met Pro Ile Asp Gln
30                  35                  40                  45

ggc ttt gcc tct tta cag cca tcc ctt cat cat cct tca act aat caa      675
Gly Phe Ala Ser Leu Gln Pro Ser Leu His His Pro Ser Thr Asn Gln
                50                  55                  60

aat caa aca aag ctg aca cat ttt gat cac tat aat cag tat gaa caa      723
```

```
Asn Gln Thr Lys Leu Thr His Phe Asp His Tyr Asn Gln Tyr Glu Gln
            65                  70                  75

caa aag atg cat ctg atg gat cag ccg aac aga atg atg agc aac acc      771
Gln Lys Met His Leu Met Asp Gln Pro Asn Arg Met Met Ser Asn Thr
        80                  85                  90

cct ggg aac gga ctc gcg tct ccg cac tcg cag tat cac acc cct ccc      819
Pro Gly Asn Gly Leu Ala Ser Pro His Ser Gln Tyr His Thr Pro Pro
    95                  100                 105

gtt cct cag gtg ccc cat ggt ggc agt ggt ggc ggt cag atg ggt gtc      867
Val Pro Gln Val Pro His Gly Gly Ser Gly Gly Gly Gln Met Gly Val
110                 115                 120                 125

tac cct ggc atg cag aat gag agg cat ggg caa tcc ttt gtg gac agc      915
Tyr Pro Gly Met Gln Asn Glu Arg His Gly Gln Ser Phe Val Asp Ser
                130                 135                 140

agc tcc atg tgg ggc ccc agg gct gtt cag gta cca gac cag ata cga      963
Ser Ser Met Trp Gly Pro Arg Ala Val Gln Val Pro Asp Gln Ile Arg
            145                 150                 155

gcc ccc tac cag cag cag cag cca cag ccg cag cca ccg cag ccg gct     1011
Ala Pro Tyr Gln Gln Gln Gln Pro Gln Pro Gln Pro Pro Gln Pro Ala
        160                 165                 170

ccg tcg ggg ccc cct gca cag ggc cac cct cag cac atg cag cag atg     1059
Pro Ser Gly Pro Pro Ala Gln Gly His Pro Gln His Met Gln Gln Met
    175                 180                 185

ggc agc tat atg gca cgt ggg gat ttt tcc atg cag cag cat ggt cag     1107
Gly Ser Tyr Met Ala Arg Gly Asp Phe Ser Met Gln Gln His Gly Gln
190                 195                 200                 205

cca cag cag agg atg agc cag ttt tcc caa ggc caa gag ggc ctc aat     1155
Pro Gln Gln Arg Met Ser Gln Phe Ser Gln Gly Gln Glu Gly Leu Asn
                210                 215                 220

cag gga aat cct ttt att gcc acc tca gga cct ggc cac ttg tcc cac     1203
Gln Gly Asn Pro Phe Ile Ala Thr Ser Gly Pro Gly His Leu Ser His
            225                 230                 235

gtg ccc cag cag agt ccc agc atg gca cct tcc ttg cgt cac tcg gtg     1251
Val Pro Gln Gln Ser Pro Ser Met Ala Pro Ser Leu Arg His Ser Val
        240                 245                 250

cag cag ttc cat cac cac ccc tct act gct ctc cat gga gaa tcc gtt     1299
Gln Gln Phe His His His Pro Ser Thr Ala Leu His Gly Glu Ser Val
    255                 260                 265

gcc cac agt ccc aga ttc tcc ccg aat cct ccc caa caa ggg gct gtt     1347
Ala His Ser Pro Arg Phe Ser Pro Asn Pro Pro Gln Gln Gly Ala Val
270                 275                 280                 285

agg ccg caa acc ctt aac ttt agt tct cgg agc cag aca gtc ccc tct     1395
Arg Pro Gln Thr Leu Asn Phe Ser Ser Arg Ser Gln Thr Val Pro Ser
                290                 295                 300

cct act ata aac aac tca ggg cag tat tct cga tat cct tac agt aac     1443
Pro Thr Ile Asn Asn Ser Gly Gln Tyr Ser Arg Tyr Pro Tyr Ser Asn
            305                 310                 315
```

```
cta aat cag gga tta gtt aac aat aca ggg atg aat caa aat tta ggc        1491
Leu Asn Gln Gly Leu Val Asn Asn Thr Gly Met Asn Gln Asn Leu Gly
        320                 325                 330

ctt aca aat aat act cca atg aat cag tcc gta cca aga tac ccc aat        1539
Leu Thr Asn Asn Thr Pro Met Asn Gln Ser Val Pro Arg Tyr Pro Asn
    335                 340                 345

gct gta gga ttc cca tca aac agt ggt caa gga cta atg cac cag cag        1587
Ala Val Gly Phe Pro Ser Asn Ser Gly Gln Gly Leu Met His Gln Gln
350                 355                 360                 365

ccc atc cac ccc agt ggc tca ctt aac caa atg aac aca caa act atg        1635
Pro Ile His Pro Ser Gly Ser Leu Asn Gln Met Asn Thr Gln Thr Met
                370                 375                 380

cat cct tca cag cct cag gga act tat gcc tct cca cct ccc atg tca        1683
His Pro Ser Gln Pro Gln Gly Thr Tyr Ala Ser Pro Pro Pro Met Ser
            385                 390                 395

ccc atg aaa gca atg agt aat cca gca ggc act cct cct cca caa gtc        1731
Pro Met Lys Ala Met Ser Asn Pro Ala Gly Thr Pro Pro Pro Gln Val
        400                 405                 410

agg ccg gga agt gct ggg ata cca atg gaa gtt ggc agt tat cca aat        1779
Arg Pro Gly Ser Ala Gly Ile Pro Met Glu Val Gly Ser Tyr Pro Asn
    415                 420                 425

atg ccc cat cct cag cca tct cac cag ccc cct ggt gcc atg gga atc        1827
Met Pro His Pro Gln Pro Ser His Gln Pro Pro Gly Ala Met Gly Ile
430                 435                 440                 445

gga cag agg aat atg ggc ccc aga aac atg cag cag tct cgt cca ttt        1875
Gly Gln Arg Asn Met Gly Pro Arg Asn Met Gln Gln Ser Arg Pro Phe
                450                 455                 460

ata ggc atg tcc tcg gca cca agg gaa ttg act ggg cac atg agg cca        1923
Ile Gly Met Ser Ser Ala Pro Arg Glu Leu Thr Gly His Met Arg Pro
            465                 470                 475

aat ggt tgt cct ggt gtt ggc ctt gga gac cca caa gca atc cag gaa        1971
Asn Gly Cys Pro Gly Val Gly Leu Gly Asp Pro Gln Ala Ile Gln Glu
        480                 485                 490

cga ctg ata cct ggc caa caa cat cct ggt caa cag cca tct ttt cag        2019
Arg Leu Ile Pro Gly Gln Gln His Pro Gly Gln Gln Pro Ser Phe Gln
    495                 500                 505

cag ttg cca acc tgt cct cca ctg cag cct cac ccg ggc ttg cac cac        2067
Gln Leu Pro Thr Cys Pro Pro Leu Gln Pro His Pro Gly Leu His His
510                 515                 520                 525

cag tct tca cct cca cac cct cat cac cag cct tgg gca cag ctc cac        2115
Gln Ser Ser Pro Pro His Pro His His Gln Pro Trp Ala Gln Leu His
                530                 535                 540

cca tca ccc cag aac acc ccg cag aaa gtg cct gtg cat cag cat tcc        2163
Pro Ser Pro Gln Asn Thr Pro Gln Lys Val Pro Val His Gln His Ser
            545                 550                 555

ccg tcg gag ccc ttt cta gag aaa cca gtg ccg gat atg act cag gtt        2211
Pro Ser Glu Pro Phe Leu Glu Lys Pro Val Pro Asp Met Thr Gln Val
        560                 565                 570
```

```
agt gga ccg aat gct cag cta gtg aag agt gat gat tac ctg cca tca        2259
Ser Gly Pro Asn Ala Gln Leu Val Lys Ser Asp Asp Tyr Leu Pro Ser
    575                 580                 585

ata gaa cag cag cca caa caa aag aag aag aaa aag aaa aac aac cac        2307
Ile Glu Gln Gln Pro Gln Gln Lys Lys Lys Lys Lys Lys Asn Asn His
590                 595                 600                 605

att gta gca gag gat ccc agt aaa ggt ttt ggt aaa gat gac ttc cct        2355
Ile Val Ala Glu Asp Pro Ser Lys Gly Phe Gly Lys Asp Asp Phe Pro
                610                 615                 620

ggt ggg gta gat aac caa gaa cta aat agg aac tca ctg gat ggg tcc        2403
Gly Gly Val Asp Asn Gln Glu Leu Asn Arg Asn Ser Leu Asp Gly Ser
            625                 630                 635

caa gaa gaa aaa aag aaa aag aaa agg tca aag gca aaa aaa gac ccg        2451
Gln Glu Glu Lys Lys Lys Lys Lys Arg Ser Lys Ala Lys Lys Asp Pro
        640                 645                 650

aag gaa ccg aaa gaa ccc aag gag aaa aaa gag ccc aag gaa ccc aag        2499
Lys Glu Pro Lys Glu Pro Lys Glu Lys Lys Glu Pro Lys Glu Pro Lys
    655                 660                 665

acc ccg aaa gcc cct aag att ccc aaa gag cca aag gaa aag aaa gca        2547
Thr Pro Lys Ala Pro Lys Ile Pro Lys Glu Pro Lys Glu Lys Lys Ala
670                 675                 680                 685

aaa act gcc acg cca aaa ccc aaa tcc agc aaa aag tca agt aat aag        2595
Lys Thr Ala Thr Pro Lys Pro Lys Ser Ser Lys Lys Ser Ser Asn Lys
                690                 695                 700

aaa cct gac tca gaa gca agt gct ttg aag aaa aag gtc aac aag gga        2643
Lys Pro Asp Ser Glu Ala Ser Ala Leu Lys Lys Lys Val Asn Lys Gly
            705                 710                 715

aaa aca gaa ggt tct gaa aat tca gac tta gac aaa aca ccc cca cca        2691
Lys Thr Glu Gly Ser Glu Asn Ser Asp Leu Asp Lys Thr Pro Pro Pro
        720                 725                 730

tct cct cct cct gaa gaa gat gag gac cca ggt gtt cag aag aga cgg        2739
Ser Pro Pro Pro Glu Glu Asp Glu Asp Pro Gly Val Gln Lys Arg Arg
    735                 740                 745

tcc agc aga cag gtg aag aga aag cgc tac act gaa gac ctg gag ttc        2787
Ser Ser Arg Gln Val Lys Arg Lys Arg Tyr Thr Glu Asp Leu Glu Phe
750                 755                 760                 765

aag att tct gat gag gag gca gat gat gca gat gct gct ggg agg gat        2835
Lys Ile Ser Asp Glu Glu Ala Asp Asp Ala Asp Ala Ala Gly Arg Asp
                770                 775                 780

tcc ccc tcc aac acc tcc cag tca gaa cag cag gaa tct gtt gat gca        2883
Ser Pro Ser Asn Thr Ser Gln Ser Glu Gln Gln Glu Ser Val Asp Ala
            785                 790                 795

gaa ggc cca gtg gta gaa aaa att atg agc agt cgt tca gta aaa aag        2931
Glu Gly Pro Val Val Glu Lys Ile Met Ser Ser Arg Ser Val Lys Lys
        800                 805                 810

cag aag gaa tct gga gag gag gta gaa att gag gaa ttc tat gtg aaa        2979
Gln Lys Glu Ser Gly Glu Glu Val Glu Ile Glu Glu Phe Tyr Val Lys
```

```
    815                 820                 825

tac aaa aac ttc tct tat ctt cat tgt cag tgg gca tct ata gaa gat        3027
Tyr Lys Asn Phe Ser Tyr Leu His Cys Gln Trp Ala Ser Ile Glu Asp
830                 835                 840                 845

ctg gaa aaa gat aag aga att cag caa aaa att aaa cga ttt aag gca        3075
Leu Glu Lys Asp Lys Arg Ile Gln Gln Lys Ile Lys Arg Phe Lys Ala
                850                 855                 860

aag cag ggc cag aac aag ttc ctt tca gag att gag gat gag ctt ttt        3123
Lys Gln Gly Gln Asn Lys Phe Leu Ser Glu Ile Glu Asp Glu Leu Phe
            865                 870                 875

aat cca gat tat gtg gag gtt gac cgg ata atg gac ttt gca cgt agc        3171
Asn Pro Asp Tyr Val Glu Val Asp Arg Ile Met Asp Phe Ala Arg Ser
        880                 885                 890

aca gat gac cgg gga gag cct gtg act cac tat ctg gtg aag tgg tgt        3219
Thr Asp Asp Arg Gly Glu Pro Val Thr His Tyr Leu Val Lys Trp Cys
    895                 900                 905

tca ctt cct tat gaa gac agc acg tgg gag cgg agg cag gac ata gat        3267
Ser Leu Pro Tyr Glu Asp Ser Thr Trp Glu Arg Arg Gln Asp Ile Asp
910                 915                 920                 925

caa gca aag atc gag gag ttt gag aaa cta atg tcc agg gag ccg gaa        3315
Gln Ala Lys Ile Glu Glu Phe Glu Lys Leu Met Ser Arg Glu Pro Glu
                930                 935                 940

aca gag cgt gtg gag cga cct cct gct gat gat tgg aag aaa tcg gag        3363
Thr Glu Arg Val Glu Arg Pro Pro Ala Asp Asp Trp Lys Lys Ser Glu
            945                 950                 955

agt tcc agg gag tat aaa aac aat aac aaa ctc agg gaa tac cag ttg        3411
Ser Ser Arg Glu Tyr Lys Asn Asn Asn Lys Leu Arg Glu Tyr Gln Leu
        960                 965                 970

gag gga gta aac tgg cta ctt ttc aat tgg tac aac atg cga aac tgc        3459
Glu Gly Val Asn Trp Leu Leu Phe Asn Trp Tyr Asn Met Arg Asn Cys
    975                 980                 985

att tta gca gat gaa atg ggt ttg gga aaa act  atc cag tcc att aca       3507
Ile Leu Ala Asp Glu Met Gly Leu Gly Lys Thr  Ile Gln Ser Ile Thr
990                 995                 1000                1005

ttt ctc tat gag ata  tat ttg aaa gga atc  cat ggc cct ttt tta          3552
Phe Leu Tyr Glu Ile  Tyr Leu Lys Gly Ile  His Gly Pro Phe Leu
                1010                 1015                 1020

gta att gcc cca ttg  tcc aca atc ccc aac  tgg gaa agg gaa ttc          3597
Val Ile Ala Pro Leu  Ser Thr Ile Pro Asn  Trp Glu Arg Glu Phe
                1025                 1030                 1035

cga acc tgg aca gag  ttg aac gtg gtt gtg  tat cat ggg agt caa          3642
Arg Thr Trp Thr Glu  Leu Asn Val Val Val  Tyr His Gly Ser Gln
                1040                 1045                 1050

gct agt cgt cgg acc  att cag ttg tat gaa  atg tac ttc aaa gat          3687
Ala Ser Arg Arg Thr  Ile Gln Leu Tyr Glu  Met Tyr Phe Lys Asp
                1055                 1060                 1065

ccc cag ggt cga gtg  ata aag ggg tcc tat  aag ttt cat gcc atc          3732
```

```
Pro Gln Gly Arg Val  Ile Lys Gly Ser Tyr  Lys Phe His Ala Ile
                1070                 1075                 1080

atc act aca ttt gag  atg att ttg act gat  tgt cct gag ctg cgg        3777
Ile Thr Thr Phe Glu  Met Ile Leu Thr Asp  Cys Pro Glu Leu Arg
                1085                 1090                 1095

aat att cca tgg cgc  tgt gta gtc att gat  gaa gcc cac agg ctg        3822
Asn Ile Pro Trp Arg  Cys Val Val Ile Asp  Glu Ala His Arg Leu
                1100                 1105                 1110

aag aac agg aac tgc  aag ctg ttg gag gga  ctc aag atg atg gac        3867
Lys Asn Arg Asn Cys  Lys Leu Leu Glu Gly  Leu Lys Met Met Asp
                1115                 1120                 1125

ttg gaa cac aaa gtg  ctg ctg acg gga acc  cca ctc cag aac act        3912
Leu Glu His Lys Val  Leu Leu Thr Gly Thr  Pro Leu Gln Asn Thr
                1130                 1135                 1140

gtg gaa gaa ctc ttc  agc ttg ctt cat ttc  ttg gaa cca agt cgc        3957
Val Glu Glu Leu Phe  Ser Leu Leu His Phe  Leu Glu Pro Ser Arg
                1145                 1150                 1155

ttc cct tca gaa acc  aca ttt atg caa gaa  ttt ggt gat cta aaa        4002
Phe Pro Ser Glu Thr  Thr Phe Met Gln Glu  Phe Gly Asp Leu Lys
                1160                 1165                 1170

aca gaa gag cag gtg  caa aaa ctt caa gct  att cta aag cca atg        4047
Thr Glu Glu Gln Val  Gln Lys Leu Gln Ala  Ile Leu Lys Pro Met
                1175                 1180                 1185

atg ttg aga cgt ctc  aaa gag gat gta gaa  aag aac ttg gcc ccc        4092
Met Leu Arg Arg Leu  Lys Glu Asp Val Glu  Lys Asn Leu Ala Pro
                1190                 1195                 1200

aaa gaa gaa act att  att gaa gtt gag cta  aca aac att cag aag        4137
Lys Glu Glu Thr Ile  Ile Glu Val Glu Leu  Thr Asn Ile Gln Lys
                1205                 1210                 1215

aaa tat tac cga gcc  atc ctt gag aag aat  ttc aca ttt ctt tcc        4182
Lys Tyr Tyr Arg Ala  Ile Leu Glu Lys Asn  Phe Thr Phe Leu Ser
                1220                 1225                 1230

aaa ggc ggt ggt caa  gct aac gta cct aac  cta tta aac act atg        4227
Lys Gly Gly Gly Gln  Ala Asn Val Pro Asn  Leu Leu Asn Thr Met
                1235                 1240                 1245

atg gaa ttg cgg aag  tgc tgc aat cat ccg  tac ctt atc aat ggt        4272
Met Glu Leu Arg Lys  Cys Cys Asn His Pro  Tyr Leu Ile Asn Gly
                1250                 1255                 1260

gct gaa gag aaa att  ttg gaa gag ttt aaa  gaa aca cac aat gca        4317
Ala Glu Glu Lys Ile  Leu Glu Glu Phe Lys  Glu Thr His Asn Ala
                1265                 1270                 1275

gag tct cca gat ttt  cag ctc cag gca atg  atc cag gct gct ggc        4362
Glu Ser Pro Asp Phe  Gln Leu Gln Ala Met  Ile Gln Ala Ala Gly
                1280                 1285                 1290

aag cta gtg ctg att  gac aag ctg ctg cca  aaa ctg aag gct ggt        4407
Lys Leu Val Leu Ile  Asp Lys Leu Leu Pro  Lys Leu Lys Ala Gly
                1295                 1300                 1305
```

```
ggc cac agg gtg ctt  atc ttt tcc cag atg  gtg cgc tgc ttg gac          4452
Gly His Arg Val Leu  Ile Phe Ser Gln Met  Val Arg Cys Leu Asp
                1310                 1315                 1320

ata ctg gaa gac tac  ctc att caa aga cgg  tac cca tat gaa agg          4497
Ile Leu Glu Asp Tyr  Leu Ile Gln Arg Arg  Tyr Pro Tyr Glu Arg
                1325                 1330                 1335

atc gac ggc cga gta  aga ggc aac ctc cgc  cag gca gct atc gac          4542
Ile Asp Gly Arg Val  Arg Gly Asn Leu Arg  Gln Ala Ala Ile Asp
                1340                 1345                 1350

aga ttc tcc aaa cct  gat tct gat agg ttt  gtt ttc ctc ctg tgt          4587
Arg Phe Ser Lys Pro  Asp Ser Asp Arg Phe  Val Phe Leu Leu Cys
                1355                 1360                 1365

aca agg gca gga ggt  tta ggc att aac ctc  act gct gct gat acc          4632
Thr Arg Ala Gly Gly  Leu Gly Ile Asn Leu  Thr Ala Ala Asp Thr
                1370                 1375                 1380

tgc atc atc ttt gat  tca gac tgg aat ccc  caa aat gac ctc cag          4677
Cys Ile Ile Phe Asp  Ser Asp Trp Asn Pro  Gln Asn Asp Leu Gln
                1385                 1390                 1395

gct cag gct aga tgt  cat aga ata gga cag  agc aaa tct gtg aaa          4722
Ala Gln Ala Arg Cys  His Arg Ile Gly Gln  Ser Lys Ser Val Lys
                1400                 1405                 1410

atc tac agg ctg att  aca aga aat tcc tat  gaa agg gaa atg ttc          4767
Ile Tyr Arg Leu Ile  Thr Arg Asn Ser Tyr  Glu Arg Glu Met Phe
                1415                 1420                 1425

gac aag gct agt ttg  aaa ctg ggc ctg gat  aaa gct gtg cta cag          4812
Asp Lys Ala Ser Leu  Lys Leu Gly Leu Asp  Lys Ala Val Leu Gln
                1430                 1435                 1440

tct atg agt gga aga  gaa aat gct acc aat  ggg gta caa cag ctt          4857
Ser Met Ser Gly Arg  Glu Asn Ala Thr Asn  Gly Val Gln Gln Leu
                1445                 1450                 1455

tcc aag aaa gaa ata  gag gat ctt cta cga  aaa ggg gcc tat ggt          4902
Ser Lys Lys Glu Ile  Glu Asp Leu Leu Arg  Lys Gly Ala Tyr Gly
                1460                 1465                 1470

gca ctc atg gat gag  gag gat gaa ggg tct  aaa ttc tgt gaa gaa          4947
Ala Leu Met Asp Glu  Glu Asp Glu Gly Ser  Lys Phe Cys Glu Glu
                1475                 1480                 1485

gat att gat cag atc  ctc cta cgt cga acc  cac acc att acc att          4992
Asp Ile Asp Gln Ile  Leu Leu Arg Arg Thr  His Thr Ile Thr Ile
                1490                 1495                 1500

gag tca gaa ggg aaa  ggt tcc aca ttt gct  aag gcc agt ttt gtt          5037
Glu Ser Glu Gly Lys  Gly Ser Thr Phe Ala  Lys Ala Ser Phe Val
                1505                 1510                 1515

gca tct gga aat agg  aca gat att tcc ttg  gat gat cca aat ttc          5082
Ala Ser Gly Asn Arg  Thr Asp Ile Ser Leu  Asp Asp Pro Asn Phe
                1520                 1525                 1530

tgg caa aag tgg gct  aag aag gct gaa ttg  gat att gat gcc tta          5127
Trp Gln Lys Trp Ala  Lys Lys Ala Glu Leu  Asp Ile Asp Ala Leu
                1535                 1540                 1545
```

```
aat ggg agg aac aac  ctg gtt att gat act  cca aga gtg aga aag        5172
Asn Gly Arg Asn Asn  Leu Val Ile Asp Thr  Pro Arg Val Arg Lys
                1550                 1555                 1560

cag acc agg ctc tac  agt gca gtg aag gaa  gat gag ctg atg gag        5217
Gln Thr Arg Leu Tyr  Ser Ala Val Lys Glu  Asp Glu Leu Met Glu
                1565                 1570                 1575

ttc tca gac ttg gaa  agt gat tct gaa gaa  aag ccc tgt gca aag        5262
Phe Ser Asp Leu Glu  Ser Asp Ser Glu Glu  Lys Pro Cys Ala Lys
                1580                 1585                 1590

cca cgg cgt ccc cag  gat aag tca cag ggc  tat gca agg agt gaa        5307
Pro Arg Arg Pro Gln  Asp Lys Ser Gln Gly  Tyr Ala Arg Ser Glu
                1595                 1600                 1605

tgt ttc agg gtg gag  aag aat ctg ctt gtc  tat ggt tgg gga cgg        5352
Cys Phe Arg Val Glu  Lys Asn Leu Leu Val  Tyr Gly Trp Gly Arg
                1610                 1615                 1620

tgg aca gac att ctt  tcc cac gga cgc tat  aaa cgc caa ctc act        5397
Trp Thr Asp Ile Leu  Ser His Gly Arg Tyr  Lys Arg Gln Leu Thr
                1625                 1630                 1635

gag caa gat gta gaa  acc atc tgc aga acc  atc ctg gtg tac tgt        5442
Glu Gln Asp Val Glu  Thr Ile Cys Arg Thr  Ile Leu Val Tyr Cys
                1640                 1645                 1650

ctt aat cat tac aaa  ggg gat gag aat atc  aaa agc ttc atc tgg        5487
Leu Asn His Tyr Lys  Gly Asp Glu Asn Ile  Lys Ser Phe Ile Trp
                1655                 1660                 1665

gat ctg atc aca ccc  aca gcg gat ggc cag  act cga gcc ttg gtc        5532
Asp Leu Ile Thr Pro  Thr Ala Asp Gly Gln  Thr Arg Ala Leu Val
                1670                 1675                 1680

aac cat tcc ggt ttg  tca gct cct gtg cca  agg gga agg aag gga        5577
Asn His Ser Gly Leu  Ser Ala Pro Val Pro  Arg Gly Arg Lys Gly
                1685                 1690                 1695

aag aag gtg aaa gcc  cag agc aca cag ccg  gtg gtg cag gat gcc        5622
Lys Lys Val Lys Ala  Gln Ser Thr Gln Pro  Val Val Gln Asp Ala
                1700                 1705                 1710

gac tgg ctg gcc agc  tgc aac cca gat gcc  ctg ttc cag gag gac        5667
Asp Trp Leu Ala Ser  Cys Asn Pro Asp Ala  Leu Phe Gln Glu Asp
                1715                 1720                 1725

agc tac aag aaa cac  ctg aag cat cac tgt  aac aag gtc ctg ctg        5712
Ser Tyr Lys Lys His  Leu Lys His His Cys  Asn Lys Val Leu Leu
                1730                 1735                 1740

cgt gtc cgc atg ctg  tac tac cta aga caa  gaa gtg ata gga gac        5757
Arg Val Arg Met Leu  Tyr Tyr Leu Arg Gln  Glu Val Ile Gly Asp
                1745                 1750                 1755

cag gcg gat aag atc  tta gag ggt gct gac  tca agt gaa gcc gat        5802
Gln Ala Asp Lys Ile  Leu Glu Gly Ala Asp  Ser Ser Glu Ala Asp
                1760                 1765                 1770

gtg tgg atc cct gaa  cct ttc cat gct gaa  gtt cct gca gat tgg        5847
Val Trp Ile Pro Glu  Pro Phe His Ala Glu  Val Pro Ala Asp Trp
```

```
                1775                1780                1785

tgg gat aag gaa gca  gac aaa tcc ctc tta  att gga gtg ttc aaa         5892
Trp Asp Lys Glu Ala  Asp Lys Ser Leu Leu  Ile Gly Val Phe Lys
                1790                1795                1800

cat ggc tat gag aag  tac aac tcc atg cga  gct gac ccc gcg ctg         5937
His Gly Tyr Glu Lys  Tyr Asn Ser Met Arg  Ala Asp Pro Ala Leu
                1805                1810                1815

tgc ttt ctg gaa cga  gtc ggt atg cct gat  gcc aag gcc ata gct         5982
Cys Phe Leu Glu Arg  Val Gly Met Pro Asp  Ala Lys Ala Ile Ala
                1820                1825                1830

gcc gag caa aga gga  aca gac atg cta gca  gat ggt ggt gac ggg         6027
Ala Glu Gln Arg Gly  Thr Asp Met Leu Ala  Asp Gly Gly Asp Gly
                1835                1840                1845

gga gaa ttt gat aga  gaa gat gaa gac cca  gaa tat aaa cca acc         6072
Gly Glu Phe Asp Arg  Glu Asp Glu Asp Pro  Glu Tyr Lys Pro Thr
                1850                1855                1860

aga aca ccg ttc aaa  gat gaa ata gat gaa  ttt gca aat tct cct         6117
Arg Thr Pro Phe Lys  Asp Glu Ile Asp Glu  Phe Ala Asn Ser Pro
                1865                1870                1875

tca gag gat aag gaa  gaa tcc atg gaa ata  cat gcc aca ggc aag         6162
Ser Glu Asp Lys Glu  Glu Ser Met Glu Ile  His Ala Thr Gly Lys
                1880                1885                1890

cac agt gag agt aat  gct gag tta ggc caa  ctt tac tgg cct aac         6207
His Ser Glu Ser Asn  Ala Glu Leu Gly Gln  Leu Tyr Trp Pro Asn
                1895                1900                1905

act tca acc ctg act  aca cgt ctg cgc cgg  ctc att act gcc tat         6252
Thr Ser Thr Leu Thr  Thr Arg Leu Arg Arg  Leu Ile Thr Ala Tyr
                1910                1915                1920

cag cgc agc tat aaa  agg caa cag atg agg  caa gag gcc cta atg         6297
Gln Arg Ser Tyr Lys  Arg Gln Gln Met Arg  Gln Glu Ala Leu Met
                1925                1930                1935

aag act gac cgg cgc  aga cgg cgg cct cga  gag gaa gtg aga gct         6342
Lys Thr Asp Arg Arg  Arg Arg Arg Pro Arg  Glu Glu Val Arg Ala
                1940                1945                1950

ctg gaa gcg gaa agg  gaa gct att ata tct  gag aag cgg caa aag         6387
Leu Glu Ala Glu Arg  Glu Ala Ile Ile Ser  Glu Lys Arg Gln Lys
                1955                1960                1965

tgg aca aga aga gaa  gag gct gat ttt tac  cgt gtg gta tcc acc         6432
Trp Thr Arg Arg Glu  Glu Ala Asp Phe Tyr  Arg Val Val Ser Thr
                1970                1975                1980

ttt ggg gtt att ttt  gac cct gtg aaa cag  caa ttt gac tgg aac         6477
Phe Gly Val Ile Phe  Asp Pro Val Lys Gln  Gln Phe Asp Trp Asn
                1985                1990                1995

caa ttt aga gcc ttt  gcc agg ctt gac aaa  aaa tct gat gag agt         6522
Gln Phe Arg Ala Phe  Ala Arg Leu Asp Lys  Lys Ser Asp Glu Ser
                2000                2005                2010

ttg gag aaa tac ttc  agt tgt ttt gtg gcc  atg tgt agg cga gta         6567
```

```
Leu Glu Lys Tyr Phe  Ser Cys Phe Val Ala  Met Cys Arg Arg Val
                2015                 2020                 2025

tgt cga atg ccc gtc  aag cca gat gat gaa  ccg ccc gac ctc tcc        6612
Cys Arg Met Pro Val  Lys Pro Asp Asp Glu  Pro Pro Asp Leu Ser
                2030                 2035                 2040

tcc ata att gag ccg  atc aca gag gag cga  gcc tct cga act ctg        6657
Ser Ile Ile Glu Pro  Ile Thr Glu Glu Arg  Ala Ser Arg Thr Leu
                2045                 2050                 2055

tac cgc att gag ctg  cta cgg aag atc cgc  gag cag gtt ctc cat        6702
Tyr Arg Ile Glu Leu  Leu Arg Lys Ile Arg  Glu Gln Val Leu His
                2060                 2065                 2070

cac ccc cag ctg gga  gag agg ctt aag ctc  tgc cag cca agc ttg        6747
His Pro Gln Leu Gly  Glu Arg Leu Lys Leu  Cys Gln Pro Ser Leu
                2075                 2080                 2085

gat ctg cca gag tgg  tgg gag tgt gga cgg  cat gac cga gac ttg        6792
Asp Leu Pro Glu Trp  Trp Glu Cys Gly Arg  His Asp Arg Asp Leu
                2090                 2095                 2100

ctg gtt ggt gct gct  aaa cac ggg gtc agt  cgg acg gat tat cac        6837
Leu Val Gly Ala Ala  Lys His Gly Val Ser  Arg Thr Asp Tyr His
                2105                 2110                 2115

atc ctc aat gac cct  gag tta tcc ttc ttg  gat gca cat aaa aac        6882
Ile Leu Asn Asp Pro  Glu Leu Ser Phe Leu  Asp Ala His Lys Asn
                2120                 2125                 2130

ttt gct caa aac aga  ggg gca ggt aat aca  tct tcc ttg aac cca        6927
Phe Ala Gln Asn Arg  Gly Ala Gly Asn Thr  Ser Ser Leu Asn Pro
                2135                 2140                 2145

ctg gca gtt gga ttt  gtc cag act cct cca  gtc atc tca tct gct        6972
Leu Ala Val Gly Phe  Val Gln Thr Pro Pro  Val Ile Ser Ser Ala
                2150                 2155                 2160

cat att caa gat gag  agg gta ctg gaa caa  gcc gaa ggc aaa gtg        7017
His Ile Gln Asp Glu  Arg Val Leu Glu Gln  Ala Glu Gly Lys Val
                2165                 2170                 2175

gag gag cct gaa aac  cca gct gcc aag gag  aaa tgt gag ggc aaa        7062
Glu Glu Pro Glu Asn  Pro Ala Ala Lys Glu  Lys Cys Glu Gly Lys
                2180                 2185                 2190

gaa gag gaa gaa gaa  acc gat ggc agc ggg  aag gag agc aag cag        7107
Glu Glu Glu Glu Glu  Thr Asp Gly Ser Gly  Lys Glu Ser Lys Gln
                2195                 2200                 2205

gaa tgt gag gca gag  gcc agc tct gtg aaa  aat gaa ctg aaa ggt        7152
Glu Cys Glu Ala Glu  Ala Ser Ser Val Lys  Asn Glu Leu Lys Gly
                2210                 2215                 2220

gtt gag gtc ggc gca  gac act ggg tcc aaa  tct att tca gag aaa        7197
Val Glu Val Gly Ala  Asp Thr Gly Ser Lys  Ser Ile Ser Glu Lys
                2225                 2230                 2235

ggt tcc gaa gag gat  gaa gag gaa aag ctg  gag gat gac gat aag        7242
Gly Ser Glu Glu Asp  Glu Glu Glu Lys Leu  Glu Asp Asp Asp Lys
                2240                 2245                 2250
```

```
tcg gaa gag tct tcc  cag ccc gaa gca gga  gct gtc tct aga ggg        7287
Ser Glu Glu Ser Ser  Gln Pro Glu Ala Gly  Ala Val Ser Arg Gly
                2255                 2260                 2265

aag aat ttt gat gaa  gaa agc aat gct tcc  atg agc act gct aga        7332
Lys Asn Phe Asp Glu  Glu Ser Asn Ala Ser  Met Ser Thr Ala Arg
                2270                 2275                 2280

gat gaa acc cga gat  gga ttc tac atg gag  gac gga gat cct tca        7377
Asp Glu Thr Arg Asp  Gly Phe Tyr Met Glu  Asp Gly Asp Pro Ser
                2285                 2290                 2295

gta gct cag ctc ctt  cat gaa aga aca ttt  gcc ttc tcg ttt tgg        7422
Val Ala Gln Leu Leu  His Glu Arg Thr Phe  Ala Phe Ser Phe Trp
                2300                 2305                 2310

cct aag gat aga gta  atg ata aac cgc tta  gac aac atc tgt gaa        7467
Pro Lys Asp Arg Val  Met Ile Asn Arg Leu  Asp Asn Ile Cys Glu
                2315                 2320                 2325

gca gtg ttg aaa ggc  aaa tgg cca gta aat  agg cgc cag atg ttt        7512
Ala Val Leu Lys Gly  Lys Trp Pro Val Asn  Arg Arg Gln Met Phe
                2330                 2335                 2340

gat ttc caa ggc ctc  atc cca ggt tac aca  ccc acc aca gtg gac        7557
Asp Phe Gln Gly Leu  Ile Pro Gly Tyr Thr  Pro Thr Thr Val Asp
                2345                 2350                 2355

agc ccc ttg cag aag  agg agc ttt gct gag  ctc tcc atg gtc ggc        7602
Ser Pro Leu Gln Lys  Arg Ser Phe Ala Glu  Leu Ser Met Val Gly
                2360                 2365                 2370

caa gcc agc att agt  ggg agt gag gac atc  act acg tct cct cag        7647
Gln Ala Ser Ile Ser  Gly Ser Glu Asp Ile  Thr Thr Ser Pro Gln
                2375                 2380                 2385

ttg tca aag gaa gat  gcc ctc aac ctc tct  gtc cct cgc cag cgg        7692
Leu Ser Lys Glu Asp  Ala Leu Asn Leu Ser  Val Pro Arg Gln Arg
                2390                 2395                 2400

agg agg agg agg aga  aaa atc gaa att gag  gcc gaa aga gct gcc        7737
Arg Arg Arg Arg Arg  Lys Ile Glu Ile Glu  Ala Glu Arg Ala Ala
                2405                 2410                 2415

aag agg cga aat ctc  atg gag atg gtt gcc  cag ctt cga gag tct        7782
Lys Arg Arg Asn Leu  Met Glu Met Val Ala  Gln Leu Arg Glu Ser
                2420                 2425                 2430

cag gtg gtc tca gaa  aat gga caa gaa aaa  gtt gta gat tta tca        7827
Gln Val Val Ser Glu  Asn Gly Gln Glu Lys  Val Val Asp Leu Ser
                2435                 2440                 2445

aag gcc tca aga gag  gca aca agc tct acc  tca aat ttt tca tct        7872
Lys Ala Ser Arg Glu  Ala Thr Ser Ser Thr  Ser Asn Phe Ser Ser
                2450                 2455                 2460

ctt tct tca aag ttt  atc ttg cct aat gtc  tca aca cca gtg tct        7917
Leu Ser Ser Lys Phe  Ile Leu Pro Asn Val  Ser Thr Pro Val Ser
                2465                 2470                 2475

gat gcc ttt aag act  caa atg gaa ctg ctc  caa gca ggc ctt tcg        7962
Asp Ala Phe Lys Thr  Gln Met Glu Leu Leu  Gln Ala Gly Leu Ser
                2480                 2485                 2490
```

```
cgc aca ccc aca agg  cat ctc ctt aat ggc  tcc cta gtg gat gga        8007
Arg Thr Pro Thr Arg  His Leu Leu Asn Gly  Ser Leu Val Asp Gly
                2495                 2500                 2505

gag cct ccc atg aag  agg agg cgg gga agg  agg aaa aat gtg gag        8052
Glu Pro Pro Met Lys  Arg Arg Arg Gly Arg  Arg Lys Asn Val Glu
                2510                 2515                 2520

gga ctt gat ctg ctt  ttc atg agc cac aaa  cgg acg tca ttg agt        8097
Gly Leu Asp Leu Leu  Phe Met Ser His Lys  Arg Thr Ser Leu Ser
                2525                 2530                 2535

gca gag gat gct gag  gtg acc aaa gct ttt  gaa gaa gat ata gag        8142
Ala Glu Asp Ala Glu  Val Thr Lys Ala Phe  Glu Glu Asp Ile Glu
                2540                 2545                 2550

acc cca cca aca aga  aac att cct tct ccc  gga cag ctg gac cca        8187
Thr Pro Pro Thr Arg  Asn Ile Pro Ser Pro  Gly Gln Leu Asp Pro
                2555                 2560                 2565

gac aca cgg atc cct  gtt atc aat ctt gaa  gat ggg act agg ctg        8232
Asp Thr Arg Ile Pro  Val Ile Asn Leu Glu  Asp Gly Thr Arg Leu
                2570                 2575                 2580

gtg ggg gaa gat gct  cct aaa aat aag gat  tta gtt gaa tgg ctg        8277
Val Gly Glu Asp Ala  Pro Lys Asn Lys Asp  Leu Val Glu Trp Leu
                2585                 2590                 2595

aag ctg cac cct act  tac act gtt gat atg  cca agt tat gta cca        8322
Lys Leu His Pro Thr  Tyr Thr Val Asp Met  Pro Ser Tyr Val Pro
                2600                 2605                 2610

aag aat gca gat gtg  ctg ttt tcc tca ttt  cag aaa ccg aaa cag        8367
Lys Asn Ala Asp Val  Leu Phe Ser Ser Phe  Gln Lys Pro Lys Gln
                2615                 2620                 2625

aaa cga cat aga tgt  cga aac cct aat aaa  ttg gat ata aac act        8412
Lys Arg His Arg Cys  Arg Asn Pro Asn Lys  Leu Asp Ile Asn Thr
                2630                 2635                 2640

ttg aca gga gaa gaa  agg gtg cct gtt gtc  aat aaa cga aat ggg        8457
Leu Thr Gly Glu Glu  Arg Val Pro Val Val  Asn Lys Arg Asn Gly
                2645                 2650                 2655

aag aag atg ggt gga  gct atg gcg cct cca  atg aag gat cta ccc        8502
Lys Lys Met Gly Gly  Ala Met Ala Pro Pro  Met Lys Asp Leu Pro
                2660                 2665                 2670

agg tgg ctg gaa gaa  aat cct gaa ttt gca  gtt gct cca gac tgg        8547
Arg Trp Leu Glu Glu  Asn Pro Glu Phe Ala  Val Ala Pro Asp Trp
                2675                 2680                 2685

act gat ata gtt aag  cag tct ggt ttt gtt  cct gag tcg atg ttt        8592
Thr Asp Ile Val Lys  Gln Ser Gly Phe Val  Pro Glu Ser Met Phe
                2690                 2695                 2700

gac cgc ctt ctc act  ggg cct gta gtg cgg  gga gag gga gcg agc        8637
Asp Arg Leu Leu Thr  Gly Pro Val Val Arg  Gly Glu Gly Ala Ser
                2705                 2710                 2715

aga aga gga aga agg  ccc aaa agt gag atc  gcc aga gca gcc gcg        8682
Arg Arg Gly Arg Arg  Pro Lys Ser Glu Ile  Ala Arg Ala Ala Ala
```

```
                    2720                2725                2730

gcc gcc gct gct gtg  gcc tcc acg tca ggg  atc aac cct ttg ctg        8727
Ala Ala Ala Ala Val  Ala Ser Thr Ser Gly  Ile Asn Pro Leu Leu
                    2735                2740                2745

gtg aac agc ctg ttt  gct gga atg gac ctg  acg agc ctt cag aat        8772
Val Asn Ser Leu Phe  Ala Gly Met Asp Leu  Thr Ser Leu Gln Asn
                    2750                2755                2760

ctc cag aat ctc cag  tcg ctc cag ctg gca  ggc ctc atg ggc ttc        8817
Leu Gln Asn Leu Gln  Ser Leu Gln Leu Ala  Gly Leu Met Gly Phe
                    2765                2770                2775

cct cca gga ctg gca  aca gct gcc acc gcc  gga ggc gat gcg aag        8862
Pro Pro Gly Leu Ala  Thr Ala Ala Thr Ala  Gly Gly Asp Ala Lys
                    2780                2785                2790

aac cct gct gct gtg  ctg ccc ctg atg ctg  cca gga atg gcg ggc        8907
Asn Pro Ala Ala Val  Leu Pro Leu Met Leu  Pro Gly Met Ala Gly
                    2795                2800                2805

ctg ccc aac gtg ttt  ggc ttg ggc ggg ctg  ttg aat aac cct ctg        8952
Leu Pro Asn Val Phe  Gly Leu Gly Gly Leu  Leu Asn Asn Pro Leu
                    2810                2815                2820

tca gct gct act gga  aac acc act act gct  tct agt caa gga gaa        8997
Ser Ala Ala Thr Gly  Asn Thr Thr Thr Ala  Ser Ser Gln Gly Glu
                    2825                2830                2835

ccg gaa gac agc act  tca aaa gga gag gag  aaa gga aat gag aat        9042
Pro Glu Asp Ser Thr  Ser Lys Gly Glu Glu  Lys Gly Asn Glu Asn
                    2840                2845                2850

gaa gac gag aac aaa  gac tct gag aaa agc  aca gat gct gtt tcg        9087
Glu Asp Glu Asn Lys  Asp Ser Glu Lys Ser  Thr Asp Ala Val Ser
                    2855                2860                2865

gct gct gac tct gcg  aat gga tct gtt ggt  gct gct act gcc ccg        9132
Ala Ala Asp Ser Ala  Asn Gly Ser Val Gly  Ala Ala Thr Ala Pro
                    2870                2875                2880

gct gga ttg ccc tca  aac ccg cta gcc ttc  aac cct ttc ctc ctg        9177
Ala Gly Leu Pro Ser  Asn Pro Leu Ala Phe  Asn Pro Phe Leu Leu
                    2885                2890                2895

tcc aca atg gcc ccg  ggc ctc ttc tac cca  tcc atg ttt cta cct        9222
Ser Thr Met Ala Pro  Gly Leu Phe Tyr Pro  Ser Met Phe Leu Pro
                    2900                2905                2910

cca gga ctg ggg gga  ttg acg ctg cct ggg  ttc cca gca ttg gca        9267
Pro Gly Leu Gly Gly  Leu Thr Leu Pro Gly  Phe Pro Ala Leu Ala
                    2915                2920                2925

gga ctt cag aat gcc  gtg ggc tcc agc gaa  gaa aag gct gct gac        9312
Gly Leu Gln Asn Ala  Val Gly Ser Ser Glu  Glu Lys Ala Ala Asp
                    2930                2935                2940

aag gct gag gga gga  ccc ttt aaa gat gga  gag acc ctt gaa ggc        9357
Lys Ala Glu Gly Gly  Pro Phe Lys Asp Gly  Glu Thr Leu Glu Gly
                    2945                2950                2955

agc gat gcc gag gag  agc ctg gat aag act  gca gag tcc tcc ctc        9402
```

```
Ser Asp Ala Glu Glu  Ser Leu Asp Lys Thr  Ala Glu Ser Ser Leu
                2960                 2965                 2970

tta gaa gac gaa ata  gca cag ggt gaa gag  cta gac tca ctt gat        9447
Leu Glu Asp Glu Ile  Ala Gln Gly Glu Glu  Leu Asp Ser Leu Asp
                2975                 2980                 2985

ggg ggg gat gaa ata  gaa aac aat gaa aat  gat gaa taa ccagtaccag     9496
Gly Gly Asp Glu Ile  Glu Asn Asn Glu Asn  Asp Glu
                2990                 2995

ttccagttca agtgtttaaa acttttgaca agtggtagtc ctactgttta cactcacagt    9556

taatgttcat acctagtttt ataagctgtt ctgtaacata gtgtagcaaa aaaaaaagtt    9616

caagtcatgt tatacaggtg tgtcaaaagg tatcttggtc attaagtatt gtgcagtgca    9676

ttatttatta tccctaggag agatgaaatt tgagaggtga tcatgtcttt ttaaggaaac    9736

ttacataatg ctctgctttt tttttttctc ttggtaccat tggtattata ataaagagca    9796

atttgtaact gagtggcact aatggaagaa agtgctgctc aaaggaagta tgaagttata    9856

tatttaattt tttaatttta atttttaatt tttttgctgt gaaggtcaag ctgaaattta    9916

ccatacatat catacttgct catttgtttc ccttttttgac tgtatggggg ttcccacact    9976

cgtgcataca cacacatcca tacactctga caatctccac gctagtgtga acgcctctgt   10036

cccgaggcgc agcaataata aggcagctgt tgaatgtgaa gggtcccttt ggaaaattaa   10096

cctactggga gggttcttgc cagacagaac tacagttcca ttgtctcgtg gtcttgtaat   10156

gcactggtaa aaacaaaata aatagatgaa taaataaaga gtgagagaag agagaatcag   10216

gtaccttttt taaattaaag gactttgtta ctttagccac aaagctaaaa cagcattacc   10276

tcagctctaa actagccttg aagtttacag acatgacttt gtaaatgtat tgtttttctt   10336

tgttgtgatg tccttttatt tttttctttg aaaactgcta tcatgtaaga taaaatgtaa   10396

attgctgcca actgtagtaa tgatgctttt aataaaagtg acccatgata tgcagagatg   10456

taattataga atgtagtata tagggaaact ggtgttcact ctattttttt tgtattcgca   10516

atagatgcaa atacagcatt ctggcttttg tacagtttct tgatatatcc tcttatacta   10576

gattagcttt tggtaagaca ctaaactgtc tcgaagacta gacaggaagg aaaaccttga   10636

attactctca cataattcca ctccagatat ctcaacagaa atgcatacaa aaagctccta   10696

ttactccctc aaaagggcat ctgagaccga gaatacttag aaatgtgtgc agcgtgtgat   10756

aatgtggtac actgaagaac aaaagggcaa aagaaaaatg aggctttaac aggcacaata   10816

tctaggtcat ttatccttgg ttaatgggta gaaaaacaca atgcggtagt gtcagcaagg   10876

gacacaaagg cactctggtg tcctgcagac cagcgctcga tgccagaaac cagtgtgtgg   10936

aaaaacccat gtggaattga aacagaccca cttaagcacg cacgcgcgca cgcacggtct   10996

caggagctac tgatttgtgg accccttttt gacctttggt atttaaagta aaatataatt   11056
```

tgagatctac tgttttcacc tttttatgtc acctgaacca acacaaagcc atatttccat 11116

ccagttaaaa agcaggggaa gggatgtgga cgagagtgtt tcgtgtgtgt tgccttcctc 11176

cacacccttc ccccaggacg tccgcacaca gtccacctcc agccagtgtg ctgccagaca 11236

ggtggtctaa attcctccca gactgtgaga ttcagttcgt ttatgcccca agatgaaaat 11296

atgcccctta atgattctgg gaaagaaaca acctgaaccc tccaccttac agatcctgtg 11356

attgctttta tttatcatcg tcgttgtctg ctgaaagtga atgggccgtg cacactggag 11416

gaaagtgcct tgaagagaat attttttgaa agggaattat ttgaacacgg gaaagtgaaa 11476

ctaggtctgc atgaagtata ggaaatttaa gtatttaagt aacaaagatg ttagcaggga 11536

gaatttgctt aataaaatga gtcattaaag ggtgtttttt ttaaaaaaaa aaa 11589

<210> 2
<211> 2997
<212> PRT
<213> Homo sapiens

<400> 2

Met Ala Asp Pro Gly Met Met Ser Leu Phe Gly Glu Asp Gly Asn Ile
1 5 10 15

Phe Ser Glu Gly Leu Glu Gly Leu Gly Glu Cys Gly Tyr Pro Glu Asn
20 25 30

Pro Val Asn Pro Met Gly Gln Gln Met Pro Ile Asp Gln Gly Phe Ala
35 40 45

Ser Leu Gln Pro Ser Leu His His Pro Ser Thr Asn Gln Asn Gln Thr
50 55 60

Lys Leu Thr His Phe Asp His Tyr Asn Gln Tyr Glu Gln Gln Lys Met
65 70 75 80

His Leu Met Asp Gln Pro Asn Arg Met Met Ser Asn Thr Pro Gly Asn
85 90 95

Gly Leu Ala Ser Pro His Ser Gln Tyr His Thr Pro Pro Val Pro Gln
100 105 110

Val Pro His Gly Gly Ser Gly Gly Gly Gln Met Gly Val Tyr Pro Gly
115 120 125

Met Gln Asn Glu Arg His Gly Gln Ser Phe Val Asp Ser Ser Ser Met
130 135 140

Trp Gly Pro Arg Ala Val Gln Val Pro Asp Gln Ile Arg Ala Pro Tyr

```
145                 150                 155                 160

Gln Gln Gln Gln Pro Gln Pro Gln Pro Pro Gln Pro Ala Pro Ser Gly
                165                 170                 175

Pro Pro Ala Gln Gly His Pro Gln His Met Gln Gln Met Gly Ser Tyr
            180                 185                 190

Met Ala Arg Gly Asp Phe Ser Met Gln Gln His Gly Gln Pro Gln Gln
        195                 200                 205

Arg Met Ser Gln Phe Ser Gln Gly Gln Glu Gly Leu Asn Gln Gly Asn
    210                 215                 220

Pro Phe Ile Ala Thr Ser Gly Pro Gly His Leu Ser His Val Pro Gln
225                 230                 235                 240

Gln Ser Pro Ser Met Ala Pro Ser Leu Arg His Ser Val Gln Gln Phe
                245                 250                 255

His His His Pro Ser Thr Ala Leu His Gly Glu Ser Val Ala His Ser
            260                 265                 270

Pro Arg Phe Ser Pro Asn Pro Pro Gln Gln Gly Ala Val Arg Pro Gln
        275                 280                 285

Thr Leu Asn Phe Ser Ser Arg Ser Gln Thr Val Pro Ser Pro Thr Ile
    290                 295                 300

Asn Asn Ser Gly Gln Tyr Ser Arg Tyr Pro Tyr Ser Asn Leu Asn Gln
305                 310                 315                 320

Gly Leu Val Asn Asn Thr Gly Met Asn Gln Asn Leu Gly Leu Thr Asn
                325                 330                 335

Asn Thr Pro Met Asn Gln Ser Val Pro Arg Tyr Pro Asn Ala Val Gly
            340                 345                 350

Phe Pro Ser Asn Ser Gly Gln Gly Leu Met His Gln Gln Pro Ile His
        355                 360                 365

Pro Ser Gly Ser Leu Asn Gln Met Asn Thr Gln Thr Met His Pro Ser
    370                 375                 380

Gln Pro Gln Gly Thr Tyr Ala Ser Pro Pro Pro Met Ser Pro Met Lys
385                 390                 395                 400
```

Ala Met Ser Asn Pro Ala Gly Thr Pro Pro Pro Gln Val Arg Pro Gly
405 410 415

Ser Ala Gly Ile Pro Met Glu Val Gly Ser Tyr Pro Asn Met Pro His
420 425 430

Pro Gln Pro Ser His Gln Pro Pro Gly Ala Met Gly Ile Gly Gln Arg
435 440 445

Asn Met Gly Pro Arg Asn Met Gln Gln Ser Arg Pro Phe Ile Gly Met
450 455 460

Ser Ser Ala Pro Arg Glu Leu Thr Gly His Met Arg Pro Asn Gly Cys
465 470 475 480

Pro Gly Val Gly Leu Gly Asp Pro Gln Ala Ile Gln Glu Arg Leu Ile
485 490 495

Pro Gly Gln Gln His Pro Gly Gln Gln Pro Ser Phe Gln Gln Leu Pro
500 505 510

Thr Cys Pro Pro Leu Gln Pro His Pro Gly Leu His His Gln Ser Ser
515 520 525

Pro Pro His Pro His His Gln Pro Trp Ala Gln Leu His Pro Ser Pro
530 535 540

Gln Asn Thr Pro Gln Lys Val Pro Val His Gln His Ser Pro Ser Glu
545 550 555 560

Pro Phe Leu Glu Lys Pro Val Pro Asp Met Thr Gln Val Ser Gly Pro
565 570 575

Asn Ala Gln Leu Val Lys Ser Asp Asp Tyr Leu Pro Ser Ile Glu Gln
580 585 590

Gln Pro Gln Gln Lys Lys Lys Lys Lys Lys Asn Asn His Ile Val Ala
595 600 605

Glu Asp Pro Ser Lys Gly Phe Gly Lys Asp Asp Phe Pro Gly Gly Val
610 615 620

Asp Asn Gln Glu Leu Asn Arg Asn Ser Leu Asp Gly Ser Gln Glu Glu
625 630 635 640

Lys Lys Lys Lys Lys Arg Ser Lys Ala Lys Lys Asp Pro Lys Glu Pro
645 650 655

Lys Glu Pro Lys Glu Lys Lys Glu Pro Lys Glu Pro Lys Thr Pro Lys
660 665 670

Ala Pro Lys Ile Pro Lys Glu Pro Lys Glu Lys Lys Ala Lys Thr Ala
675 680 685

Thr Pro Lys Pro Lys Ser Ser Lys Lys Ser Ser Asn Lys Lys Pro Asp
690 695 700

Ser Glu Ala Ser Ala Leu Lys Lys Lys Val Asn Lys Gly Lys Thr Glu
705 710 715 720

Gly Ser Glu Asn Ser Asp Leu Asp Lys Thr Pro Pro Pro Ser Pro Pro
725 730 735

Pro Glu Glu Asp Glu Asp Pro Gly Val Gln Lys Arg Arg Ser Ser Arg
740 745 750

Gln Val Lys Arg Lys Arg Tyr Thr Glu Asp Leu Glu Phe Lys Ile Ser
755 760 765

Asp Glu Glu Ala Asp Asp Ala Asp Ala Ala Gly Arg Asp Ser Pro Ser
770 775 780

Asn Thr Ser Gln Ser Glu Gln Gln Glu Ser Val Asp Ala Glu Gly Pro
785 790 795 800

Val Val Glu Lys Ile Met Ser Ser Arg Ser Val Lys Lys Gln Lys Glu
805 810 815

Ser Gly Glu Glu Val Glu Ile Glu Glu Phe Tyr Val Lys Tyr Lys Asn
820 825 830

Phe Ser Tyr Leu His Cys Gln Trp Ala Ser Ile Glu Asp Leu Glu Lys
835 840 845

Asp Lys Arg Ile Gln Gln Lys Ile Lys Arg Phe Lys Ala Lys Gln Gly
850 855 860

Gln Asn Lys Phe Leu Ser Glu Ile Glu Asp Glu Leu Phe Asn Pro Asp
865 870 875 880

Tyr Val Glu Val Asp Arg Ile Met Asp Phe Ala Arg Ser Thr Asp Asp
885 890 895

Arg Gly Glu Pro Val Thr His Tyr Leu Val Lys Trp Cys Ser Leu Pro
900 905 910

```
Tyr Glu Asp Ser Thr Trp Glu Arg Arg Gln Asp Ile Asp Gln Ala Lys
        915                 920                 925

Ile Glu Glu Phe Glu Lys Leu Met Ser Arg Glu Pro Glu Thr Glu Arg
    930                 935                 940

Val Glu Arg Pro Pro Ala Asp Asp Trp Lys Lys Ser Glu Ser Ser Arg
945                 950                 955                 960

Glu Tyr Lys Asn Asn Asn Lys Leu Arg Glu Tyr Gln Leu Glu Gly Val
                965                 970                 975

Asn Trp Leu Leu Phe Asn Trp Tyr Asn Met Arg Asn Cys Ile Leu Ala
            980                 985                 990

Asp Glu Met Gly Leu Gly Lys Thr  Ile Gln Ser Ile Thr  Phe Leu Tyr
        995                 1000                 1005

Glu Ile  Tyr Leu Lys Gly Ile  His Gly Pro Phe Leu  Val Ile Ala
    1010                 1015                 1020

Pro Leu  Ser Thr Ile Pro Asn  Trp Glu Arg Glu Phe  Arg Thr Trp
    1025                 1030                 1035

Thr Glu  Leu Asn Val Val Val  Tyr His Gly Ser Gln  Ala Ser Arg
    1040                 1045                 1050

Arg Thr  Ile Gln Leu Tyr Glu  Met Tyr Phe Lys Asp  Pro Gln Gly
    1055                 1060                 1065

Arg Val  Ile Lys Gly Ser Tyr  Lys Phe His Ala Ile  Ile Thr Thr
    1070                 1075                 1080

Phe Glu  Met Ile Leu Thr Asp  Cys Pro Glu Leu Arg  Asn Ile Pro
    1085                 1090                 1095

Trp Arg  Cys Val Val Ile Asp  Glu Ala His Arg Leu  Lys Asn Arg
    1100                 1105                 1110

Asn Cys  Lys Leu Leu Glu Gly  Leu Lys Met Met Asp  Leu Glu His
    1115                 1120                 1125

Lys Val  Leu Leu Thr Gly Thr  Pro Leu Gln Asn Thr  Val Glu Glu
    1130                 1135                 1140

Leu Phe  Ser Leu Leu His Phe  Leu Glu Pro Ser Arg  Phe Pro Ser
```

1145 1150 1155

Glu Thr Thr Phe Met Gln Glu Phe Gly Asp Leu Lys Thr Glu Glu
1160 1165 1170

Gln Val Gln Lys Leu Gln Ala Ile Leu Lys Pro Met Met Leu Arg
1175 1180 1185

Arg Leu Lys Glu Asp Val Glu Lys Asn Leu Ala Pro Lys Glu Glu
1190 1195 1200

Thr Ile Ile Glu Val Glu Leu Thr Asn Ile Gln Lys Lys Tyr Tyr
1205 1210 1215

Arg Ala Ile Leu Glu Lys Asn Phe Thr Phe Leu Ser Lys Gly Gly
1220 1225 1230

Gly Gln Ala Asn Val Pro Asn Leu Leu Asn Thr Met Met Glu Leu
1235 1240 1245

Arg Lys Cys Cys Asn His Pro Tyr Leu Ile Asn Gly Ala Glu Glu
1250 1255 1260

Lys Ile Leu Glu Glu Phe Lys Glu Thr His Asn Ala Glu Ser Pro
1265 1270 1275

Asp Phe Gln Leu Gln Ala Met Ile Gln Ala Ala Gly Lys Leu Val
1280 1285 1290

Leu Ile Asp Lys Leu Leu Pro Lys Leu Lys Ala Gly Gly His Arg
1295 1300 1305

Val Leu Ile Phe Ser Gln Met Val Arg Cys Leu Asp Ile Leu Glu
1310 1315 1320

Asp Tyr Leu Ile Gln Arg Arg Tyr Pro Tyr Glu Arg Ile Asp Gly
1325 1330 1335

Arg Val Arg Gly Asn Leu Arg Gln Ala Ala Ile Asp Arg Phe Ser
1340 1345 1350

Lys Pro Asp Ser Asp Arg Phe Val Phe Leu Leu Cys Thr Arg Ala
1355 1360 1365

Gly Gly Leu Gly Ile Asn Leu Thr Ala Ala Asp Thr Cys Ile Ile
1370 1375 1380

Phe Asp Ser Asp Trp Asn Pro Gln Asn Asp Leu Gln Ala Gln Ala
1385 1390 1395

Arg Cys His Arg Ile Gly Gln Ser Lys Ser Val Lys Ile Tyr Arg
1400 1405 1410

Leu Ile Thr Arg Asn Ser Tyr Glu Arg Glu Met Phe Asp Lys Ala
1415 1420 1425

Ser Leu Lys Leu Gly Leu Asp Lys Ala Val Leu Gln Ser Met Ser
1430 1435 1440

Gly Arg Glu Asn Ala Thr Asn Gly Val Gln Gln Leu Ser Lys Lys
1445 1450 1455

Glu Ile Glu Asp Leu Leu Arg Lys Gly Ala Tyr Gly Ala Leu Met
1460 1465 1470

Asp Glu Glu Asp Glu Gly Ser Lys Phe Cys Glu Glu Asp Ile Asp
1475 1480 1485

Gln Ile Leu Leu Arg Arg Thr His Thr Ile Thr Ile Glu Ser Glu
1490 1495 1500

Gly Lys Gly Ser Thr Phe Ala Lys Ala Ser Phe Val Ala Ser Gly
1505 1510 1515

Asn Arg Thr Asp Ile Ser Leu Asp Asp Pro Asn Phe Trp Gln Lys
1520 1525 1530

Trp Ala Lys Lys Ala Glu Leu Asp Ile Asp Ala Leu Asn Gly Arg
1535 1540 1545

Asn Asn Leu Val Ile Asp Thr Pro Arg Val Arg Lys Gln Thr Arg
1550 1555 1560

Leu Tyr Ser Ala Val Lys Glu Asp Glu Leu Met Glu Phe Ser Asp
1565 1570 1575

Leu Glu Ser Asp Ser Glu Glu Lys Pro Cys Ala Lys Pro Arg Arg
1580 1585 1590

Pro Gln Asp Lys Ser Gln Gly Tyr Ala Arg Ser Glu Cys Phe Arg
1595 1600 1605

Val Glu Lys Asn Leu Leu Val Tyr Gly Trp Gly Arg Trp Thr Asp
1610 1615 1620

Ile Leu Ser His Gly Arg Tyr Lys Arg Gln Leu Thr Glu Gln Asp
1625 1630 1635

Val Glu Thr Ile Cys Arg Thr Ile Leu Val Tyr Cys Leu Asn His
1640 1645 1650

Tyr Lys Gly Asp Glu Asn Ile Lys Ser Phe Ile Trp Asp Leu Ile
1655 1660 1665

Thr Pro Thr Ala Asp Gly Gln Thr Arg Ala Leu Val Asn His Ser
1670 1675 1680

Gly Leu Ser Ala Pro Val Pro Arg Gly Arg Lys Gly Lys Lys Val
1685 1690 1695

Lys Ala Gln Ser Thr Gln Pro Val Val Gln Asp Ala Asp Trp Leu
1700 1705 1710

Ala Ser Cys Asn Pro Asp Ala Leu Phe Gln Glu Asp Ser Tyr Lys
1715 1720 1725

Lys His Leu Lys His His Cys Asn Lys Val Leu Leu Arg Val Arg
1730 1735 1740

Met Leu Tyr Tyr Leu Arg Gln Glu Val Ile Gly Asp Gln Ala Asp
1745 1750 1755

Lys Ile Leu Glu Gly Ala Asp Ser Ser Glu Ala Asp Val Trp Ile
1760 1765 1770

Pro Glu Pro Phe His Ala Glu Val Pro Ala Asp Trp Trp Asp Lys
1775 1780 1785

Glu Ala Asp Lys Ser Leu Leu Ile Gly Val Phe Lys His Gly Tyr
1790 1795 1800

Glu Lys Tyr Asn Ser Met Arg Ala Asp Pro Ala Leu Cys Phe Leu
1805 1810 1815

Glu Arg Val Gly Met Pro Asp Ala Lys Ala Ile Ala Ala Glu Gln
1820 1825 1830

Arg Gly Thr Asp Met Leu Ala Asp Gly Gly Asp Gly Gly Glu Phe
1835 1840 1845

Asp Arg Glu Asp Glu Asp Pro Glu Tyr Lys Pro Thr Arg Thr Pro
1850 1855 1860

Phe Lys Asp Glu Ile Asp Glu Phe Ala Asn Ser Pro Ser Glu Asp
1865 1870 1875

Lys Glu Glu Ser Met Glu Ile His Ala Thr Gly Lys His Ser Glu
1880 1885 1890

Ser Asn Ala Glu Leu Gly Gln Leu Tyr Trp Pro Asn Thr Ser Thr
1895 1900 1905

Leu Thr Thr Arg Leu Arg Arg Leu Ile Thr Ala Tyr Gln Arg Ser
1910 1915 1920

Tyr Lys Arg Gln Gln Met Arg Gln Glu Ala Leu Met Lys Thr Asp
1925 1930 1935

Arg Arg Arg Arg Arg Pro Arg Glu Glu Val Arg Ala Leu Glu Ala
1940 1945 1950

Glu Arg Glu Ala Ile Ile Ser Glu Lys Arg Gln Lys Trp Thr Arg
1955 1960 1965

Arg Glu Glu Ala Asp Phe Tyr Arg Val Val Ser Thr Phe Gly Val
1970 1975 1980

Ile Phe Asp Pro Val Lys Gln Gln Phe Asp Trp Asn Gln Phe Arg
1985 1990 1995

Ala Phe Ala Arg Leu Asp Lys Lys Ser Asp Glu Ser Leu Glu Lys
2000 2005 2010

Tyr Phe Ser Cys Phe Val Ala Met Cys Arg Arg Val Cys Arg Met
2015 2020 2025

Pro Val Lys Pro Asp Asp Glu Pro Pro Asp Leu Ser Ser Ile Ile
2030 2035 2040

Glu Pro Ile Thr Glu Glu Arg Ala Ser Arg Thr Leu Tyr Arg Ile
2045 2050 2055

Glu Leu Leu Arg Lys Ile Arg Glu Gln Val Leu His His Pro Gln
2060 2065 2070

Leu Gly Glu Arg Leu Lys Leu Cys Gln Pro Ser Leu Asp Leu Pro
2075 2080 2085

Glu Trp Trp Glu Cys Gly Arg His Asp Arg Asp Leu Leu Val Gly

2090 2095 2100

Ala Ala Lys His Gly Val Ser Arg Thr Asp Tyr His Ile Leu Asn
2105 2110 2115

Asp Pro Glu Leu Ser Phe Leu Asp Ala His Lys Asn Phe Ala Gln
2120 2125 2130

Asn Arg Gly Ala Gly Asn Thr Ser Ser Leu Asn Pro Leu Ala Val
2135 2140 2145

Gly Phe Val Gln Thr Pro Pro Val Ile Ser Ser Ala His Ile Gln
2150 2155 2160

Asp Glu Arg Val Leu Glu Gln Ala Glu Gly Lys Val Glu Glu Pro
2165 2170 2175

Glu Asn Pro Ala Ala Lys Glu Lys Cys Glu Gly Lys Glu Glu Glu
2180 2185 2190

Glu Glu Thr Asp Gly Ser Gly Lys Glu Ser Lys Gln Glu Cys Glu
2195 2200 2205

Ala Glu Ala Ser Ser Val Lys Asn Glu Leu Lys Gly Val Glu Val
2210 2215 2220

Gly Ala Asp Thr Gly Ser Lys Ser Ile Ser Glu Lys Gly Ser Glu
2225 2230 2235

Glu Asp Glu Glu Glu Lys Leu Glu Asp Asp Asp Lys Ser Glu Glu
2240 2245 2250

Ser Ser Gln Pro Glu Ala Gly Ala Val Ser Arg Gly Lys Asn Phe
2255 2260 2265

Asp Glu Glu Ser Asn Ala Ser Met Ser Thr Ala Arg Asp Glu Thr
2270 2275 2280

Arg Asp Gly Phe Tyr Met Glu Asp Gly Asp Pro Ser Val Ala Gln
2285 2290 2295

Leu Leu His Glu Arg Thr Phe Ala Phe Ser Phe Trp Pro Lys Asp
2300 2305 2310

Arg Val Met Ile Asn Arg Leu Asp Asn Ile Cys Glu Ala Val Leu
2315 2320 2325

Lys Gly Lys Trp Pro Val Asn Arg Arg Gln Met Phe Asp Phe Gln
2330 2335 2340

Gly Leu Ile Pro Gly Tyr Thr Pro Thr Thr Val Asp Ser Pro Leu
2345 2350 2355

Gln Lys Arg Ser Phe Ala Glu Leu Ser Met Val Gly Gln Ala Ser
2360 2365 2370

Ile Ser Gly Ser Glu Asp Ile Thr Thr Ser Pro Gln Leu Ser Lys
2375 2380 2385

Glu Asp Ala Leu Asn Leu Ser Val Pro Arg Gln Arg Arg Arg Arg
2390 2395 2400

Arg Arg Lys Ile Glu Ile Glu Ala Glu Arg Ala Ala Lys Arg Arg
2405 2410 2415

Asn Leu Met Glu Met Val Ala Gln Leu Arg Glu Ser Gln Val Val
2420 2425 2430

Ser Glu Asn Gly Gln Glu Lys Val Val Asp Leu Ser Lys Ala Ser
2435 2440 2445

Arg Glu Ala Thr Ser Ser Thr Ser Asn Phe Ser Ser Leu Ser Ser
2450 2455 2460

Lys Phe Ile Leu Pro Asn Val Ser Thr Pro Val Ser Asp Ala Phe
2465 2470 2475

Lys Thr Gln Met Glu Leu Leu Gln Ala Gly Leu Ser Arg Thr Pro
2480 2485 2490

Thr Arg His Leu Leu Asn Gly Ser Leu Val Asp Gly Glu Pro Pro
2495 2500 2505

Met Lys Arg Arg Arg Gly Arg Arg Lys Asn Val Glu Gly Leu Asp
2510 2515 2520

Leu Leu Phe Met Ser His Lys Arg Thr Ser Leu Ser Ala Glu Asp
2525 2530 2535

Ala Glu Val Thr Lys Ala Phe Glu Glu Asp Ile Glu Thr Pro Pro
2540 2545 2550

Thr Arg Asn Ile Pro Ser Pro Gly Gln Leu Asp Pro Asp Thr Arg
2555 2560 2565

Ile Pro Val Ile Asn Leu Glu Asp Gly Thr Arg Leu Val Gly Glu
2570 2575 2580

Asp Ala Pro Lys Asn Lys Asp Leu Val Glu Trp Leu Lys Leu His
2585 2590 2595

Pro Thr Tyr Thr Val Asp Met Pro Ser Tyr Val Pro Lys Asn Ala
2600 2605 2610

Asp Val Leu Phe Ser Ser Phe Gln Lys Pro Lys Gln Lys Arg His
2615 2620 2625

Arg Cys Arg Asn Pro Asn Lys Leu Asp Ile Asn Thr Leu Thr Gly
2630 2635 2640

Glu Glu Arg Val Pro Val Val Asn Lys Arg Asn Gly Lys Lys Met
2645 2650 2655

Gly Gly Ala Met Ala Pro Pro Met Lys Asp Leu Pro Arg Trp Leu
2660 2665 2670

Glu Glu Asn Pro Glu Phe Ala Val Ala Pro Asp Trp Thr Asp Ile
2675 2680 2685

Val Lys Gln Ser Gly Phe Val Pro Glu Ser Met Phe Asp Arg Leu
2690 2695 2700

Leu Thr Gly Pro Val Val Arg Gly Glu Gly Ala Ser Arg Arg Gly
2705 2710 2715

Arg Arg Pro Lys Ser Glu Ile Ala Arg Ala Ala Ala Ala Ala Ala
2720 2725 2730

Ala Val Ala Ser Thr Ser Gly Ile Asn Pro Leu Leu Val Asn Ser
2735 2740 2745

Leu Phe Ala Gly Met Asp Leu Thr Ser Leu Gln Asn Leu Gln Asn
2750 2755 2760

Leu Gln Ser Leu Gln Leu Ala Gly Leu Met Gly Phe Pro Pro Gly
2765 2770 2775

Leu Ala Thr Ala Ala Thr Ala Gly Gly Asp Ala Lys Asn Pro Ala
2780 2785 2790

Ala Val Leu Pro Leu Met Leu Pro Gly Met Ala Gly Leu Pro Asn
2795 2800 2805

```
Val Phe Gly Leu Gly Gly Leu Leu Asn Asn Pro Leu Ser Ala Ala
    2810                2815                2820

Thr Gly Asn Thr Thr Thr Ala Ser Ser Gln Gly Glu Pro Glu Asp
    2825                2830                2835

Ser Thr Ser Lys Gly Glu Glu Lys Gly Asn Glu Asn Glu Asp Glu
    2840                2845                2850

Asn Lys Asp Ser Glu Lys Ser Thr Asp Ala Val Ser Ala Ala Asp
    2855                2860                2865

Ser Ala Asn Gly Ser Val Gly Ala Ala Thr Ala Pro Ala Gly Leu
    2870                2875                2880

Pro Ser Asn Pro Leu Ala Phe Asn Pro Phe Leu Leu Ser Thr Met
    2885                2890                2895

Ala Pro Gly Leu Phe Tyr Pro Ser Met Phe Leu Pro Pro Gly Leu
    2900                2905                2910

Gly Gly Leu Thr Leu Pro Gly Phe Pro Ala Leu Ala Gly Leu Gln
    2915                2920                2925

Asn Ala Val Gly Ser Ser Glu Glu Lys Ala Ala Asp Lys Ala Glu
    2930                2935                2940

Gly Gly Pro Phe Lys Asp Gly Glu Thr Leu Glu Gly Ser Asp Ala
    2945                2950                2955

Glu Glu Ser Leu Asp Lys Thr Ala Glu Ser Ser Leu Leu Glu Asp
    2960                2965                2970

Glu Ile Ala Gln Gly Glu Glu Leu Asp Ser Leu Asp Gly Gly Asp
    2975                2980                2985

Glu Ile Glu Asn Asn Glu Asn Asp Glu
    2990                2995


<210>  3
<211>  5124
<212>  DNA
<213>  Homo sapiens


<220>
<221>  CDS
<222>  (175)..(3021)
```

<400> 3
gacctatatc cagactttgc ctgacactgc agggtccaag agaattaaag aaatatggaa 60

tgacatgaag aagattagtt aaggattata ggctttgagg gcaaacacct cagtgaagtg 120

aagcacaggc aagctcctga gctgtggttt ggaggagccg tgtgttggaa gaag atg 177
Met
1

gca gat cca gga atg atg agt ctt ttt ggc gag gat ggg aat att ttc 225
Ala Asp Pro Gly Met Met Ser Leu Phe Gly Glu Asp Gly Asn Ile Phe
5 10 15

agt gaa ggt ctt gaa ggc ctc gga gaa tgt ggt tac ccg gaa aat cca 273
Ser Glu Gly Leu Glu Gly Leu Gly Glu Cys Gly Tyr Pro Glu Asn Pro
20 25 30

gta aat cct atg ggt cag caa atg cca ata gac caa ggc ttt gcc tct 321
Val Asn Pro Met Gly Gln Gln Met Pro Ile Asp Gln Gly Phe Ala Ser
35 40 45

tta cag cca tcc ctt cat cat cct tca act aat caa aat caa aca aag 369
Leu Gln Pro Ser Leu His His Pro Ser Thr Asn Gln Asn Gln Thr Lys
50 55 60 65

ctg aca cat ttt gat cac tat aat cag tat gaa caa caa aag atg cat 417
Leu Thr His Phe Asp His Tyr Asn Gln Tyr Glu Gln Gln Lys Met His
70 75 80

ctg atg gat cag ccg aac aga atg atg agc aac acc cct ggg aac gga 465
Leu Met Asp Gln Pro Asn Arg Met Met Ser Asn Thr Pro Gly Asn Gly
85 90 95

ctc gcg tct ccg cac tcg cag tat cac acc cct ccc gtt cct cag gtg 513
Leu Ala Ser Pro His Ser Gln Tyr His Thr Pro Pro Val Pro Gln Val
100 105 110

ccc cat ggt ggc agt ggt ggc ggt cag atg ggt gtc tac cct ggc atg 561
Pro His Gly Gly Ser Gly Gly Gly Gln Met Gly Val Tyr Pro Gly Met
115 120 125

cag aat gag agg cat ggg caa tcc ttt gtg gac agc agc tcc atg tgg 609
Gln Asn Glu Arg His Gly Gln Ser Phe Val Asp Ser Ser Ser Met Trp
130 135 140 145

ggc ccc agg gct gtt cag gta cca gac cag ata cga gcc ccc tac cag 657
Gly Pro Arg Ala Val Gln Val Pro Asp Gln Ile Arg Ala Pro Tyr Gln
150 155 160

cag cag cag cca cag ccg cag cca ccg cag ccg gct ccg tcg ggg ccc 705
Gln Gln Gln Pro Gln Pro Gln Pro Pro Gln Pro Ala Pro Ser Gly Pro
165 170 175

cct gca cag ggc cac cct cag cac atg cag cag atg ggc agc tat atg 753
Pro Ala Gln Gly His Pro Gln His Met Gln Gln Met Gly Ser Tyr Met
180 185 190

gca cgt ggg gat ttt tcc atg cag cag cat ggt cag cca cag cag agg 801
Ala Arg Gly Asp Phe Ser Met Gln Gln His Gly Gln Pro Gln Gln Arg
195 200 205

atg agc cag ttt tcc caa ggc caa gag ggc ctc aat cag gga aat cct 849

```
Met Ser Gln Phe Ser Gln Gly Gln Glu Gly Leu Asn Gln Gly Asn Pro
210                 215                 220                 225

ttt att gcc acc tca gga cct ggc cac ttg tcc cac gtg ccc cag cag      897
Phe Ile Ala Thr Ser Gly Pro Gly His Leu Ser His Val Pro Gln Gln
                230                 235                 240

agt ccc agc atg gca cct tcc ttg cgt cac tcg gtg cag cag ttc cat      945
Ser Pro Ser Met Ala Pro Ser Leu Arg His Ser Val Gln Gln Phe His
            245                 250                 255

cac cac ccc tct act gct ctc cat gga gaa tcc gtt gcc cac agt ccc      993
His His Pro Ser Thr Ala Leu His Gly Glu Ser Val Ala His Ser Pro
        260                 265                 270

aga ttc tcc ccg aat cct ccc caa caa ggg gct gtt agg ccg caa acc     1041
Arg Phe Ser Pro Asn Pro Pro Gln Gln Gly Ala Val Arg Pro Gln Thr
    275                 280                 285

ctt aac ttt agt tct cgg agc cag aca gtc ccc tct cct act ata aac     1089
Leu Asn Phe Ser Ser Arg Ser Gln Thr Val Pro Ser Pro Thr Ile Asn
290                 295                 300                 305

aac tca ggg cag tat tct cga tat cct tac agt aac cta aat cag gga     1137
Asn Ser Gly Gln Tyr Ser Arg Tyr Pro Tyr Ser Asn Leu Asn Gln Gly
                310                 315                 320

tta gtt aac aat aca ggg atg aat caa aat tta ggc ctt aca aat aat     1185
Leu Val Asn Asn Thr Gly Met Asn Gln Asn Leu Gly Leu Thr Asn Asn
            325                 330                 335

act cca atg aat cag tcc gta cca aga tac ccc aat gct gta gga ttc     1233
Thr Pro Met Asn Gln Ser Val Pro Arg Tyr Pro Asn Ala Val Gly Phe
        340                 345                 350

cca tca aac agt ggt caa gga cta atg cac cag cag ccc atc cac ccc     1281
Pro Ser Asn Ser Gly Gln Gly Leu Met His Gln Gln Pro Ile His Pro
    355                 360                 365

agt ggc tca ctt aac caa atg aac aca caa act atg cat cct tca cag     1329
Ser Gly Ser Leu Asn Gln Met Asn Thr Gln Thr Met His Pro Ser Gln
370                 375                 380                 385

cct cag gga act tat gcc tct cca cct ccc atg tca ccc atg aaa gca     1377
Pro Gln Gly Thr Tyr Ala Ser Pro Pro Pro Met Ser Pro Met Lys Ala
                390                 395                 400

atg agt aat cca gca ggc act cct cct cca caa gtc agg ccg gga agt     1425
Met Ser Asn Pro Ala Gly Thr Pro Pro Pro Gln Val Arg Pro Gly Ser
            405                 410                 415

gct ggg ata cca atg gaa gtt ggc agt tat cca aat atg ccc cat cct     1473
Ala Gly Ile Pro Met Glu Val Gly Ser Tyr Pro Asn Met Pro His Pro
        420                 425                 430

cag cca tct cac cag ccc cct ggt gcc atg gga atc gga cag agg aat     1521
Gln Pro Ser His Gln Pro Pro Gly Ala Met Gly Ile Gly Gln Arg Asn
    435                 440                 445

atg ggc ccc aga aac atg cag cag tct cgt cca ttt ata ggc atg tcc     1569
Met Gly Pro Arg Asn Met Gln Gln Ser Arg Pro Phe Ile Gly Met Ser
450                 455                 460                 465
```

```
tcg gca cca agg gaa ttg act ggg cac atg agg cca aat ggt tgt cct        1617
Ser Ala Pro Arg Glu Leu Thr Gly His Met Arg Pro Asn Gly Cys Pro
                470                 475                 480

ggt gtt ggc ctt gga gac cca caa gca atc cag gaa cga ctg ata cct        1665
Gly Val Gly Leu Gly Asp Pro Gln Ala Ile Gln Glu Arg Leu Ile Pro
            485                 490                 495

ggc caa caa cat cct ggt caa cag cca tct ttt cag cag ttg cca acc        1713
Gly Gln Gln His Pro Gly Gln Gln Pro Ser Phe Gln Gln Leu Pro Thr
        500                 505                 510

tgt cct cca ctg cag cct cac ccg ggc ttg cac cac cag tct tca cct        1761
Cys Pro Pro Leu Gln Pro His Pro Gly Leu His His Gln Ser Ser Pro
    515                 520                 525

cca cac cct cat cac cag cct tgg gca cag ctc cac cca tca ccc cag        1809
Pro His Pro His His Gln Pro Trp Ala Gln Leu His Pro Ser Pro Gln
530                 535                 540                 545

aac acc ccg cag aaa gtg cct gtg cat cag cat tcc ccg tcg gag ccc        1857
Asn Thr Pro Gln Lys Val Pro Val His Gln His Ser Pro Ser Glu Pro
                550                 555                 560

ttt cta gag aaa cca gtg ccg gat atg act cag aaa ccg aaa cag aaa        1905
Phe Leu Glu Lys Pro Val Pro Asp Met Thr Gln Lys Pro Lys Gln Lys
            565                 570                 575

cga cat aga tgt cga aac cct aat aaa ttg gat ata aac act ttg aca        1953
Arg His Arg Cys Arg Asn Pro Asn Lys Leu Asp Ile Asn Thr Leu Thr
        580                 585                 590

gga gaa gaa agg gtg cct gtt gtc aat aaa cga aat ggg aag aag atg        2001
Gly Glu Glu Arg Val Pro Val Val Asn Lys Arg Asn Gly Lys Lys Met
    595                 600                 605

ggt gga gct atg gcg cct cca atg aag gat cta ccc agg tgg ctg gaa        2049
Gly Gly Ala Met Ala Pro Pro Met Lys Asp Leu Pro Arg Trp Leu Glu
610                 615                 620                 625

gaa aat cct gaa ttt gca gtt gct cca gac tgg act gat ata gtt aag        2097
Glu Asn Pro Glu Phe Ala Val Ala Pro Asp Trp Thr Asp Ile Val Lys
                630                 635                 640

cag tct ggt ttt gtt cct gag tcg atg ttt gac cgc ctt ctc act ggg        2145
Gln Ser Gly Phe Val Pro Glu Ser Met Phe Asp Arg Leu Leu Thr Gly
            645                 650                 655

cct gta gtg cgg gga gag gga gcg agc aga aga gga aga agg ccc aaa        2193
Pro Val Val Arg Gly Glu Gly Ala Ser Arg Arg Gly Arg Arg Pro Lys
        660                 665                 670

agt gag atc gcc aga gca gcc gcg gcc gcc gct gct gtg gcc tcc acg        2241
Ser Glu Ile Ala Arg Ala Ala Ala Ala Ala Ala Ala Val Ala Ser Thr
    675                 680                 685

tca ggg atc aac cct ttg ctg gtg aac agc ctg ttt gct gga atg gac        2289
Ser Gly Ile Asn Pro Leu Leu Val Asn Ser Leu Phe Ala Gly Met Asp
690                 695                 700                 705

ctg acg agc ctt cag aat ctc cag aat ctc cag tcg ctc cag ctg gca        2337
Leu Thr Ser Leu Gln Asn Leu Gln Asn Leu Gln Ser Leu Gln Leu Ala
                710                 715                 720
```

```
ggc ctc atg ggc ttc cct cca gga ctg gca aca gct gcc acc gcc gga        2385
Gly Leu Met Gly Phe Pro Pro Gly Leu Ala Thr Ala Ala Thr Ala Gly
            725                 730                 735

ggc gat gcg aag aac cct gct gct gtg ctg ccc ctg atg ctg cca gga        2433
Gly Asp Ala Lys Asn Pro Ala Ala Val Leu Pro Leu Met Leu Pro Gly
        740                 745                 750

atg gcg ggc ctg ccc aac gtg ttt ggc ttg ggc ggg ctg ttg aat aac        2481
Met Ala Gly Leu Pro Asn Val Phe Gly Leu Gly Gly Leu Leu Asn Asn
    755                 760                 765

cct ctg tca gct gct act gga aac acc act act gct tct agt caa gga        2529
Pro Leu Ser Ala Ala Thr Gly Asn Thr Thr Thr Ala Ser Ser Gln Gly
770                 775                 780                 785

gaa ccg gaa gac agc act tca aaa gga gag gag aaa gga aat gag aat        2577
Glu Pro Glu Asp Ser Thr Ser Lys Gly Glu Glu Lys Gly Asn Glu Asn
                790                 795                 800

gaa gac gag aac aaa gac tct gag aaa agc aca gat gct gtt tcg gct        2625
Glu Asp Glu Asn Lys Asp Ser Glu Lys Ser Thr Asp Ala Val Ser Ala
            805                 810                 815

gct gac tct gcg aat gga tct gtt ggt gct gct act gcc ccg gct gga        2673
Ala Asp Ser Ala Asn Gly Ser Val Gly Ala Ala Thr Ala Pro Ala Gly
        820                 825                 830

ttg ccc tca aac ccg cta gcc ttc aac cct ttc ctc ctg tcc aca atg        2721
Leu Pro Ser Asn Pro Leu Ala Phe Asn Pro Phe Leu Leu Ser Thr Met
    835                 840                 845

gcc ccg ggc ctc ttc tac cca tcc atg ttt cta cct cca gga ctg ggg        2769
Ala Pro Gly Leu Phe Tyr Pro Ser Met Phe Leu Pro Pro Gly Leu Gly
850                 855                 860                 865

gga ttg acg ctg cct ggg ttc cca gca ttg gca gga ctt cag aat gcc        2817
Gly Leu Thr Leu Pro Gly Phe Pro Ala Leu Ala Gly Leu Gln Asn Ala
                870                 875                 880

gtg ggc tcc agc gaa gaa aag gct gct gac aag gct gag gga gga ccc        2865
Val Gly Ser Ser Glu Glu Lys Ala Ala Asp Lys Ala Glu Gly Gly Pro
            885                 890                 895

ttt aaa gat gga gag acc ctt gaa ggc agc gat gcc gag gag agc ctg        2913
Phe Lys Asp Gly Glu Thr Leu Glu Gly Ser Asp Ala Glu Glu Ser Leu
        900                 905                 910

gat aag act gca gag tcc tcc ctc tta gaa gac gaa ata gca cag ggt        2961
Asp Lys Thr Ala Glu Ser Ser Leu Leu Glu Asp Glu Ile Ala Gln Gly
    915                 920                 925

gaa gag cta gac tca ctt gat ggg ggg gat gaa ata gaa aac aat gaa        3009
Glu Glu Leu Asp Ser Leu Asp Gly Gly Asp Glu Ile Glu Asn Asn Glu
930                 935                 940                 945

aat gat gaa taa ccagtaccag ttccagttca agtgtttaaa acttttgaca            3061
Asn Asp Glu

agtggtagtc ctactgttta cactcacagt taatgttcat acctagtttt ataagctgtt      3121
```

ctgtaacata gtgtagcaaa aaaaaaagtt caagtcatgt tatacaggtg tgtcaaaagg 3181
tatcttggtc attaagtatt gtgcagtgca ttatttatta tccctaggag agatgaaatt 3241
tgagaggtga tcatgtcttt ttaaggaaac ttacataatg ctctgctttt tttttttctc 3301
ttggtaccat tggtattata ataaagagca atttgtaact gagtggcact aatggaagaa 3361
agtgctgctc aaaggaagta tgaagttata tatttaattt tttaatttta atttttaatt 3421
tttttgctgt gaaggtcaag ctgaaattta ccatacatat catacttgct catttgtttc 3481
cctttttgac tgtatggggg ttcccacact cgtgcataca cacacatcca tacactctga 3541
caatctccac gctagtgtga acgcctctgt cccgaggcgc agcaataata aggcagctgt 3601
tgaatgtgaa gggtcccttt ggaaaattaa cctactggga gggttcttgc cagacagaac 3661
tacagttcca ttgtctcgtg gtcttgtaat gcactggtaa aaacaaaata aatagatgaa 3721
taaataaaga gtgagagaag agagaatcag gtaccttttt taaattaaag gactttgtta 3781
ctttagccac aaagctaaaa cagcattacc tcagctctaa actagccttg aagtttacag 3841
acatgacttt gtaaatgtat tgtttttctt tgttgtgatg tccttttatt tttttctttg 3901
aaaactgcta tcatgtaaga taaaatgtaa attgctgcca actgtagtaa tgatgctttt 3961
aataaaagtg acccatgata tgcagagatg taattataga atgtagtata tagggaaact 4021
ggtgttcact ctattttttt tgtattcgca atagatgcaa atacagcatt ctggcttttg 4081
tacagtttct tgatatatcc tcttatacta gattagcttt tggtaagaca ctaaactgtc 4141
tcgaagacta gacaggaagg aaaaccttga attactctca cataattcca ctccagatat 4201
ctcaacagaa atgcatacaa aaagctccta ttactccctc aaaagggcat ctgagaccga 4261
gaatacttag aaatgtgtgc agcgtgtgat aatgtggtac actgaagaac aaaagggcaa 4321
aagaaaaatg aggctttaac aggcacaata tctaggtcat ttatccttgg ttaatgggta 4381
gaaaaacaca atgcggtagt gtcagcaagg gacacaaagg cactctggtg tcctgcagac 4441
cagcgctcga tgccagaaac cagtgtgtgg aaaaacccat gtggaattga aacagaccca 4501
cttaagcacg cacgcgcgca cgcacggtct caggagctac tgatttgtgg accccttttt 4561
gacctttggt atttaaagta aaatataatt tgagatctac tgttttcacc tttttatgtc 4621
acctgaacca acacaaagcc atatttccat ccagttaaaa agcaggggaa gggatgtgga 4681
cgagagtgtt tcgtgtgtgt tgccttcctc cacacccttc ccccaggacg tccgcacaca 4741
gtccacctcc agccagtgtg ctgccagaca ggtggtctaa attcctccca gactgtgaga 4801
ttcagttcgt ttatgcccca agatgaaaat atgcccctta atgattctgg gaaagaaaca 4861
acctgaaccc tccaccttac agatcctgtg attgctttta tttatcatcg tcgttgtctg 4921
ctgaaagtga atgggccgtg cacactggag gaaagtgcct tgaagagaat atttttttgaa 4981
agggaattat ttgaacacgg gaaagtgaaa ctaggtctgc atgaagtata ggaaatttaa 5041

gtatttaagt aacaaagatg ttagcaggga gaatttgctt aataaaatga gtcattaaag 5101

ggtgtttttt ttaaaaaaaa aaa 5124

<210> 4
<211> 948
<212> PRT
<213> Homo sapiens

<400> 4

Met Ala Asp Pro Gly Met Met Ser Leu Phe Gly Glu Asp Gly Asn Ile
1 5 10 15

Phe Ser Glu Gly Leu Glu Gly Leu Gly Glu Cys Gly Tyr Pro Glu Asn
20 25 30

Pro Val Asn Pro Met Gly Gln Gln Met Pro Ile Asp Gln Gly Phe Ala
35 40 45

Ser Leu Gln Pro Ser Leu His His Pro Ser Thr Asn Gln Asn Gln Thr
50 55 60

Lys Leu Thr His Phe Asp His Tyr Asn Gln Tyr Glu Gln Gln Lys Met
65 70 75 80

His Leu Met Asp Gln Pro Asn Arg Met Met Ser Asn Thr Pro Gly Asn
85 90 95

Gly Leu Ala Ser Pro His Ser Gln Tyr His Thr Pro Pro Val Pro Gln
100 105 110

Val Pro His Gly Gly Ser Gly Gly Gly Gln Met Gly Val Tyr Pro Gly
115 120 125

Met Gln Asn Glu Arg His Gly Gln Ser Phe Val Asp Ser Ser Ser Met
130 135 140

Trp Gly Pro Arg Ala Val Gln Val Pro Asp Gln Ile Arg Ala Pro Tyr
145 150 155 160

Gln Gln Gln Gln Pro Gln Pro Gln Pro Pro Gln Pro Ala Pro Ser Gly
165 170 175

Pro Pro Ala Gln Gly His Pro Gln His Met Gln Gln Met Gly Ser Tyr
180 185 190

Met Ala Arg Gly Asp Phe Ser Met Gln Gln His Gly Gln Pro Gln Gln
195 200 205

Arg Met Ser Gln Phe Ser Gln Gly Gln Glu Gly Leu Asn Gln Gly Asn
    210                 215                 220

Pro Phe Ile Ala Thr Ser Gly Pro Gly His Leu Ser His Val Pro Gln
225                 230                 235                 240

Gln Ser Pro Ser Met Ala Pro Ser Leu Arg His Ser Val Gln Gln Phe
                245                 250                 255

His His His Pro Ser Thr Ala Leu His Gly Glu Ser Val Ala His Ser
            260                 265                 270

Pro Arg Phe Ser Pro Asn Pro Pro Gln Gln Gly Ala Val Arg Pro Gln
        275                 280                 285

Thr Leu Asn Phe Ser Ser Arg Ser Gln Thr Val Pro Ser Pro Thr Ile
    290                 295                 300

Asn Asn Ser Gly Gln Tyr Ser Arg Tyr Pro Tyr Ser Asn Leu Asn Gln
305                 310                 315                 320

Gly Leu Val Asn Asn Thr Gly Met Asn Gln Asn Leu Gly Leu Thr Asn
                325                 330                 335

Asn Thr Pro Met Asn Gln Ser Val Pro Arg Tyr Pro Asn Ala Val Gly
            340                 345                 350

Phe Pro Ser Asn Ser Gly Gln Gly Leu Met His Gln Gln Pro Ile His
        355                 360                 365

Pro Ser Gly Ser Leu Asn Gln Met Asn Thr Gln Thr Met His Pro Ser
    370                 375                 380

Gln Pro Gln Gly Thr Tyr Ala Ser Pro Pro Pro Met Ser Pro Met Lys
385                 390                 395                 400

Ala Met Ser Asn Pro Ala Gly Thr Pro Pro Pro Gln Val Arg Pro Gly
                405                 410                 415

Ser Ala Gly Ile Pro Met Glu Val Gly Ser Tyr Pro Asn Met Pro His
            420                 425                 430

Pro Gln Pro Ser His Gln Pro Pro Gly Ala Met Gly Ile Gly Gln Arg
        435                 440                 445

Asn Met Gly Pro Arg Asn Met Gln Gln Ser Arg Pro Phe Ile Gly Met

450 455 460

Ser Ser Ala Pro Arg Glu Leu Thr Gly His Met Arg Pro Asn Gly Cys
465 470 475 480

Pro Gly Val Gly Leu Gly Asp Pro Gln Ala Ile Gln Glu Arg Leu Ile
485 490 495

Pro Gly Gln Gln His Pro Gly Gln Gln Pro Ser Phe Gln Gln Leu Pro
500 505 510

Thr Cys Pro Pro Leu Gln Pro His Pro Gly Leu His His Gln Ser Ser
515 520 525

Pro Pro His Pro His His Gln Pro Trp Ala Gln Leu His Pro Ser Pro
530 535 540

Gln Asn Thr Pro Gln Lys Val Pro Val His Gln His Ser Pro Ser Glu
545 550 555 560

Pro Phe Leu Glu Lys Pro Val Pro Asp Met Thr Gln Lys Pro Lys Gln
565 570 575

Lys Arg His Arg Cys Arg Asn Pro Asn Lys Leu Asp Ile Asn Thr Leu
580 585 590

Thr Gly Glu Glu Arg Val Pro Val Val Asn Lys Arg Asn Gly Lys Lys
595 600 605

Met Gly Gly Ala Met Ala Pro Pro Met Lys Asp Leu Pro Arg Trp Leu
610 615 620

Glu Glu Asn Pro Glu Phe Ala Val Ala Pro Asp Trp Thr Asp Ile Val
625 630 635 640

Lys Gln Ser Gly Phe Val Pro Glu Ser Met Phe Asp Arg Leu Leu Thr
645 650 655

Gly Pro Val Val Arg Gly Glu Gly Ala Ser Arg Arg Gly Arg Arg Pro
660 665 670

Lys Ser Glu Ile Ala Arg Ala Ala Ala Ala Ala Ala Ala Val Ala Ser
675 680 685

Thr Ser Gly Ile Asn Pro Leu Leu Val Asn Ser Leu Phe Ala Gly Met
690 695 700

```
Asp Leu Thr Ser Leu Gln Asn Leu Gln Asn Leu Gln Ser Leu Gln Leu
705                 710                 715                 720

Ala Gly Leu Met Gly Phe Pro Pro Gly Leu Ala Thr Ala Ala Thr Ala
                725                 730                 735

Gly Gly Asp Ala Lys Asn Pro Ala Ala Val Leu Pro Leu Met Leu Pro
            740                 745                 750

Gly Met Ala Gly Leu Pro Asn Val Phe Gly Leu Gly Gly Leu Leu Asn
        755                 760                 765

Asn Pro Leu Ser Ala Ala Thr Gly Asn Thr Thr Thr Ala Ser Ser Gln
    770                 775                 780

Gly Glu Pro Glu Asp Ser Thr Ser Lys Gly Glu Glu Lys Gly Asn Glu
785                 790                 795                 800

Asn Glu Asp Glu Asn Lys Asp Ser Glu Lys Ser Thr Asp Ala Val Ser
                805                 810                 815

Ala Ala Asp Ser Ala Asn Gly Ser Val Gly Ala Ala Thr Ala Pro Ala
            820                 825                 830

Gly Leu Pro Ser Asn Pro Leu Ala Phe Asn Pro Phe Leu Leu Ser Thr
        835                 840                 845

Met Ala Pro Gly Leu Phe Tyr Pro Ser Met Phe Leu Pro Pro Gly Leu
    850                 855                 860

Gly Gly Leu Thr Leu Pro Gly Phe Pro Ala Leu Ala Gly Leu Gln Asn
865                 870                 875                 880

Ala Val Gly Ser Ser Glu Glu Lys Ala Ala Asp Lys Ala Glu Gly Gly
                885                 890                 895

Pro Phe Lys Asp Gly Glu Thr Leu Glu Gly Ser Asp Ala Glu Glu Ser
            900                 905                 910

Leu Asp Lys Thr Ala Glu Ser Ser Leu Leu Glu Asp Glu Ile Ala Gln
        915                 920                 925

Gly Glu Glu Leu Asp Ser Leu Asp Gly Gly Asp Glu Ile Glu Asn Asn
    930                 935                 940

Glu Asn Asp Glu
945
```

```
<210>  5
<211>  5465
<212>  DNA
<213>  Homo sapiens


<220>
<221>  CDS
<222>  (514)..(5379)

<400>  5
aggcgctggc gtgctggggc cgcggcggcg gcggcggcgg cggcggcagc ggcggcggcg        60

gcggcggcgc gggggttgag tcgtggtggt gcggacgcgc tcgtgctcgg gaactatcgg       120

attaaacttg aatcgagtga aattacacaa aggagcgccg cggaggaggc ggcccgggga       180

cccggacacc ctgaaactca ccagagaccc gttcgccccc ggccaactcc gtgcccgtgg       240

attcagcccc ctggccgcag ctgccgagcc aactccggag cccgctctgc gttttgtttt       300

cccctcggca ctaggcagcg gaggagcccg accgacccgg acctatatcc agactttgcc       360

tgacactgca gggtccaaga gaattaaaga aatatggaat gacatgaaga agattagtta       420

aggattatag gctttgaggg caaacacctc agtgaagtga agcacaggca agctcctgag       480

ctgtggtttg gaggagccgt gtgttggaag aag atg gca gat cca gga atg atg        534
                                     Met Ala Asp Pro Gly Met Met
                                     1               5

agt ctt ttt ggc gag gat ggg aat att ttc agt gaa ggt ctt gaa ggc         582
Ser Leu Phe Gly Glu Asp Gly Asn Ile Phe Ser Glu Gly Leu Glu Gly
        10                  15                  20

ctc gga gaa tgt ggt tac ccg gaa aat cca gta aat cct atg ggt cag         630
Leu Gly Glu Cys Gly Tyr Pro Glu Asn Pro Val Asn Pro Met Gly Gln
    25                  30                  35

caa atg cca ata gac caa ggc ttt gcc tct tta cag cca tcc ctt cat         678
Gln Met Pro Ile Asp Gln Gly Phe Ala Ser Leu Gln Pro Ser Leu His
40                  45                  50                  55

cat cct tca act aat caa aat caa aca aag ctg aca cat ttt gat cac         726
His Pro Ser Thr Asn Gln Asn Gln Thr Lys Leu Thr His Phe Asp His
                60                  65                  70

tat aat cag tat gaa caa caa aag atg cat ctg atg gat cag ccg aac         774
Tyr Asn Gln Tyr Glu Gln Gln Lys Met His Leu Met Asp Gln Pro Asn
            75                  80                  85

aga atg atg agc aac acc cct ggg aac gga ctc gcg tct ccg cac tcg         822
Arg Met Met Ser Asn Thr Pro Gly Asn Gly Leu Ala Ser Pro His Ser
        90                  95                  100

cag tat cac acc cct ccc gtt cct cag gtg ccc cat ggt ggc agt ggt         870
Gln Tyr His Thr Pro Pro Val Pro Gln Val Pro His Gly Gly Ser Gly
    105                 110                 115

ggc ggt cag atg ggt gtc tac cct ggc atg cag aat gag agg cat ggg         918
Gly Gly Gln Met Gly Val Tyr Pro Gly Met Gln Asn Glu Arg His Gly
120                 125                 130                 135
```

```
caa tcc ttt gtg gac agc agc tcc atg tgg ggc ccc agg gct gtt cag        966
Gln Ser Phe Val Asp Ser Ser Ser Met Trp Gly Pro Arg Ala Val Gln
                140                 145                 150

gta cca gac cag ata cga gcc ccc tac cag cag cag cag cca cag ccg       1014
Val Pro Asp Gln Ile Arg Ala Pro Tyr Gln Gln Gln Gln Pro Gln Pro
            155                 160                 165

cag cca ccg cag ccg gct ccg tcg ggg ccc cct gca cag ggc cac cct       1062
Gln Pro Pro Gln Pro Ala Pro Ser Gly Pro Pro Ala Gln Gly His Pro
        170                 175                 180

cag cac atg cag cag atg ggc agc tat atg gca cgt ggg gat ttt tcc       1110
Gln His Met Gln Gln Met Gly Ser Tyr Met Ala Arg Gly Asp Phe Ser
    185                 190                 195

atg cag cag cat ggt cag cca cag cag agg atg agc cag ttt tcc caa       1158
Met Gln Gln His Gly Gln Pro Gln Gln Arg Met Ser Gln Phe Ser Gln
200                 205                 210                 215

ggc caa gag ggc ctc aat cag gga aat cct ttt att gcc acc tca gga       1206
Gly Gln Glu Gly Leu Asn Gln Gly Asn Pro Phe Ile Ala Thr Ser Gly
                220                 225                 230

cct ggc cac ttg tcc cac gtg ccc cag cag agt ccc agc atg gca cct       1254
Pro Gly His Leu Ser His Val Pro Gln Gln Ser Pro Ser Met Ala Pro
            235                 240                 245

tcc ttg cgt cac tcg gtg cag cag ttc cat cac cac ccc tct act gct       1302
Ser Leu Arg His Ser Val Gln Gln Phe His His His Pro Ser Thr Ala
        250                 255                 260

ctc cat gga gaa tcc gtt gcc cac agt ccc aga ttc tcc ccg aat cct       1350
Leu His Gly Glu Ser Val Ala His Ser Pro Arg Phe Ser Pro Asn Pro
    265                 270                 275

ccc caa caa ggg gct gtt agg ccg caa acc ctt aac ttt agt tct cgg       1398
Pro Gln Gln Gly Ala Val Arg Pro Gln Thr Leu Asn Phe Ser Ser Arg
280                 285                 290                 295

agc cag aca gtc ccc tct cct act ata aac aac tca ggg cag tat tct       1446
Ser Gln Thr Val Pro Ser Pro Thr Ile Asn Asn Ser Gly Gln Tyr Ser
                300                 305                 310

cga tat cct tac agt aac cta aat cag gga tta gtt aac aat aca ggg       1494
Arg Tyr Pro Tyr Ser Asn Leu Asn Gln Gly Leu Val Asn Asn Thr Gly
            315                 320                 325

atg aat caa aat tta ggc ctt aca aat aat act cca atg aat cag tcc       1542
Met Asn Gln Asn Leu Gly Leu Thr Asn Asn Thr Pro Met Asn Gln Ser
        330                 335                 340

gta cca aga tac ccc aat gct gta gga ttc cca tca aac agt ggt caa       1590
Val Pro Arg Tyr Pro Asn Ala Val Gly Phe Pro Ser Asn Ser Gly Gln
    345                 350                 355

gga cta atg cac cag cag ccc atc cac ccc agt ggc tca ctt aac caa       1638
Gly Leu Met His Gln Gln Pro Ile His Pro Ser Gly Ser Leu Asn Gln
360                 365                 370                 375

atg aac aca caa act atg cat cct tca cag cct cag gga act tat gcc       1686
Met Asn Thr Gln Thr Met His Pro Ser Gln Pro Gln Gly Thr Tyr Ala
```

```
                380                 385                 390

tct cca cct ccc atg tca ccc atg aaa gca atg agt aat cca gca ggc      1734
Ser Pro Pro Pro Met Ser Pro Met Lys Ala Met Ser Asn Pro Ala Gly
            395                 400                 405

act cct cct cca caa gtc agg ccg gga agt gct ggg ata cca atg gaa      1782
Thr Pro Pro Pro Gln Val Arg Pro Gly Ser Ala Gly Ile Pro Met Glu
        410                 415                 420

gtt ggc agt tat cca aat atg ccc cat cct cag cca tct cac cag ccc      1830
Val Gly Ser Tyr Pro Asn Met Pro His Pro Gln Pro Ser His Gln Pro
    425                 430                 435

cct ggt gcc atg gga atc gga cag agg aat atg ggc ccc aga aac atg      1878
Pro Gly Ala Met Gly Ile Gly Gln Arg Asn Met Gly Pro Arg Asn Met
440                 445                 450                 455

cag cag tct cgt cca ttt ata ggc atg tcc tcg gca cca agg gaa ttg      1926
Gln Gln Ser Arg Pro Phe Ile Gly Met Ser Ser Ala Pro Arg Glu Leu
                460                 465                 470

act ggg cac atg agg cca aat ggt tgt cct ggt gtt ggc ctt gga gac      1974
Thr Gly His Met Arg Pro Asn Gly Cys Pro Gly Val Gly Leu Gly Asp
            475                 480                 485

cca caa gca atc cag gaa cga ctg ata cct ggc caa caa cat cct ggt      2022
Pro Gln Ala Ile Gln Glu Arg Leu Ile Pro Gly Gln Gln His Pro Gly
        490                 495                 500

caa cag cca tct ttt cag cag ttg cca acc tgt cct cca ctg cag cct      2070
Gln Gln Pro Ser Phe Gln Gln Leu Pro Thr Cys Pro Pro Leu Gln Pro
    505                 510                 515

cac ccg ggc ttg cac cac cag tct tca cct cca cac cct cat cac cag      2118
His Pro Gly Leu His His Gln Ser Ser Pro Pro His Pro His His Gln
520                 525                 530                 535

cct tgg gca cag ctc cac cca tca ccc cag aac acc ccg cag aaa gtg      2166
Pro Trp Ala Gln Leu His Pro Ser Pro Gln Asn Thr Pro Gln Lys Val
                540                 545                 550

cct gtg cat cag cat tcc ccg tcg gag ccc ttt cta gag aaa cca gtg      2214
Pro Val His Gln His Ser Pro Ser Glu Pro Phe Leu Glu Lys Pro Val
            555                 560                 565

ccg gat atg act cag gtt agt gga ccg aat gct cag cta gtg aag agt      2262
Pro Asp Met Thr Gln Val Ser Gly Pro Asn Ala Gln Leu Val Lys Ser
        570                 575                 580

gat gat tac ctg cca tca ata gaa cag cag cca caa caa aag aag aag      2310
Asp Asp Tyr Leu Pro Ser Ile Glu Gln Gln Pro Gln Gln Lys Lys Lys
    585                 590                 595

aaa aag aaa aac aac cac att gta gca gag gat ccc agt aaa ggt ttt      2358
Lys Lys Lys Asn Asn His Ile Val Ala Glu Asp Pro Ser Lys Gly Phe
600                 605                 610                 615

ggt aaa gat gac ttc cct ggt ggg gta gat aac caa gaa cta aat agg      2406
Gly Lys Asp Asp Phe Pro Gly Gly Val Asp Asn Gln Glu Leu Asn Arg
                620                 625                 630

aac tca ctg gat ggg tcc caa gaa gaa aaa aag aaa aag aaa agg tca      2454
```

```
Asn Ser Leu Asp Gly Ser Gln Glu Glu Lys Lys Lys Lys Lys Arg Ser
            635                 640                 645

aag gca aaa aaa gac ccg aag gaa ccg aaa gaa ccc aag gag aaa aaa        2502
Lys Ala Lys Lys Asp Pro Lys Glu Pro Lys Glu Pro Lys Glu Lys Lys
        650                 655                 660

gag ccc aag gaa ccc aag acc ccg aaa gcc cct aag att ccc aaa gag        2550
Glu Pro Lys Glu Pro Lys Thr Pro Lys Ala Pro Lys Ile Pro Lys Glu
    665                 670                 675

cca aag gaa aag aaa gca aaa act gcc acg cca aaa ccc aaa tcc agc        2598
Pro Lys Glu Lys Lys Ala Lys Thr Ala Thr Pro Lys Pro Lys Ser Ser
680                 685                 690                 695

aaa aag tca agt aat aag aaa cct gac tca gaa gca agt gct ttg aag        2646
Lys Lys Ser Ser Asn Lys Lys Pro Asp Ser Glu Ala Ser Ala Leu Lys
                700                 705                 710

aaa aag gtc aac aag gga aaa aca gaa ggt tct gaa aat tca gac tta        2694
Lys Lys Val Asn Lys Gly Lys Thr Glu Gly Ser Glu Asn Ser Asp Leu
            715                 720                 725

gac aaa aca ccc cca cca tct cct cct cct gaa gaa gat gag gac cca        2742
Asp Lys Thr Pro Pro Pro Ser Pro Pro Pro Glu Glu Asp Glu Asp Pro
        730                 735                 740

ggt gtt cag aag aga cgg tcc agc aga cag gtg aag aga aag cgc tac        2790
Gly Val Gln Lys Arg Arg Ser Ser Arg Gln Val Lys Arg Lys Arg Tyr
    745                 750                 755

act gaa gac ctg gag ttc aag att tct gat gag gag gca gat gat gca        2838
Thr Glu Asp Leu Glu Phe Lys Ile Ser Asp Glu Glu Ala Asp Asp Ala
760                 765                 770                 775

gat gct gct ggg agg gat tcc ccc tcc aac acc tcc cag tca gaa cag        2886
Asp Ala Ala Gly Arg Asp Ser Pro Ser Asn Thr Ser Gln Ser Glu Gln
                780                 785                 790

cag gaa tct gtt gat gca gaa ggc cca gtg gta gaa aaa att atg agc        2934
Gln Glu Ser Val Asp Ala Glu Gly Pro Val Val Glu Lys Ile Met Ser
            795                 800                 805

agt cgt tca gta aaa aag cag aag gaa tct gga gag gag gta gaa att        2982
Ser Arg Ser Val Lys Lys Gln Lys Glu Ser Gly Glu Glu Val Glu Ile
        810                 815                 820

gag gaa ttc tat gtg aaa tac aaa aac ttc tct tat ctt cat tgt cag        3030
Glu Glu Phe Tyr Val Lys Tyr Lys Asn Phe Ser Tyr Leu His Cys Gln
    825                 830                 835

tgg gca tct ata gaa gat ctg gaa aaa gat aag aga att cag caa aaa        3078
Trp Ala Ser Ile Glu Asp Leu Glu Lys Asp Lys Arg Ile Gln Gln Lys
840                 845                 850                 855

att aaa cga ttt aag gca aag cag ggc cag aac aag ttc ctt tca gag        3126
Ile Lys Arg Phe Lys Ala Lys Gln Gly Gln Asn Lys Phe Leu Ser Glu
                860                 865                 870

att gag gat gag ctt ttt aat cca gat tat gtg gag gtt gac cgg ata        3174
Ile Glu Asp Glu Leu Phe Asn Pro Asp Tyr Val Glu Val Asp Arg Ile
            875                 880                 885
```

```
atg gac ttt gca cgt agc aca gat gac cgg gga gag cct gtg act cac        3222
Met Asp Phe Ala Arg Ser Thr Asp Asp Arg Gly Glu Pro Val Thr His
        890                 895                 900

tat ctg gtg aag tgg tgt tca ctt cct tat gaa gac agc acg tgg gag        3270
Tyr Leu Val Lys Trp Cys Ser Leu Pro Tyr Glu Asp Ser Thr Trp Glu
    905                 910                 915

cgg agg cag gac ata gat caa gca aag atc gag gag ttt gag aaa cta        3318
Arg Arg Gln Asp Ile Asp Gln Ala Lys Ile Glu Glu Phe Glu Lys Leu
920                 925                 930                 935

atg tcc agg gag ccg gaa aca gag cgt gtg gag cga cct cct gct gat        3366
Met Ser Arg Glu Pro Glu Thr Glu Arg Val Glu Arg Pro Pro Ala Asp
                940                 945                 950

gat tgg aag aaa tcg gag agt tcc agg gag tat aaa aac aat aac aaa        3414
Asp Trp Lys Lys Ser Glu Ser Ser Arg Glu Tyr Lys Asn Asn Asn Lys
            955                 960                 965

ctc agg gaa tac cag ttg gag gga gta aac tgg cta ctt ttc aat tgg        3462
Leu Arg Glu Tyr Gln Leu Glu Gly Val Asn Trp Leu Leu Phe Asn Trp
        970                 975                 980

tac aac atg cga aac tgc att tta gca gat gaa atg ggt ttg gga aaa        3510
Tyr Asn Met Arg Asn Cys Ile Leu Ala Asp Glu Met Gly Leu Gly Lys
    985                 990                 995

act  atc cag tcc att aca  ttt ctc tat gag ata  tat ttg aaa gga         3555
Thr  Ile Gln Ser Ile Thr  Phe Leu Tyr Glu Ile  Tyr Leu Lys Gly
1000                 1005                 1010

atc  cat ggc cct ttt tta  gta att gcc cca ttg  tcc aca atc ccc         3600
Ile  His Gly Pro Phe Leu  Val Ile Ala Pro Leu  Ser Thr Ile Pro
1015                 1020                 1025

aac  tgg gaa agg gaa ttc  cga acc tgg aca gag  ttg aac gtg gtt         3645
Asn  Trp Glu Arg Glu Phe  Arg Thr Trp Thr Glu  Leu Asn Val Val
1030                 1035                 1040

gtg  tat cat ggg agt caa  gct agt cgt cgg acc  att cag ttg tat         3690
Val  Tyr His Gly Ser Gln  Ala Ser Arg Arg Thr  Ile Gln Leu Tyr
1045                 1050                 1055

gaa  atg tac ttc aaa gat  ccc cag ggt cga gtg  ata aag ggg tcc         3735
Glu  Met Tyr Phe Lys Asp  Pro Gln Gly Arg Val  Ile Lys Gly Ser
1060                 1065                 1070

tat  aag ttt cat gcc atc  atc act aca ttt gag  atg att ttg act         3780
Tyr  Lys Phe His Ala Ile  Ile Thr Thr Phe Glu  Met Ile Leu Thr
1075                 1080                 1085

gat  tgt cct gag ctg cgg  aat att cca tgg cgc  tgt gta gtc att         3825
Asp  Cys Pro Glu Leu Arg  Asn Ile Pro Trp Arg  Cys Val Val Ile
1090                 1095                 1100

gat  gaa gcc cac agg ctg  aag aac agg aac tgc  aag ctg ttg gag         3870
Asp  Glu Ala His Arg Leu  Lys Asn Arg Asn Cys  Lys Leu Leu Glu
1105                 1110                 1115

gga  ctc aag atg atg gac  ttg gaa cac aaa gtg  ctg ctg acg gga         3915
Gly  Leu Lys Met Met Asp  Leu Glu His Lys Val  Leu Leu Thr Gly
1120                 1125                 1130
```

```
acc  cca ctc cag aac act  gtg gaa gaa ctc ttc  agc ttg ctt cat        3960
Thr  Pro Leu Gln Asn Thr  Val Glu Glu Leu Phe  Ser Leu Leu His
1135                1140                 1145

ttc  ttg gaa cca agt cgc  ttc cct tca gaa acc  aca ttt atg caa        4005
Phe  Leu Glu Pro Ser Arg  Phe Pro Ser Glu Thr  Thr Phe Met Gln
1150                1155                 1160

gaa  ttt ggt gat cta aaa  aca gaa gag cag gtg  caa aaa ctt caa        4050
Glu  Phe Gly Asp Leu Lys  Thr Glu Glu Gln Val  Gln Lys Leu Gln
1165                1170                 1175

gct  att cta aag cca atg  atg ttg aga cgt ctc  aaa gag gat gta        4095
Ala  Ile Leu Lys Pro Met  Met Leu Arg Arg Leu  Lys Glu Asp Val
1180                1185                 1190

gaa  aag aac ttg gcc ccc  aaa gaa gaa act att  att gaa gtt gag        4140
Glu  Lys Asn Leu Ala Pro  Lys Glu Glu Thr Ile  Ile Glu Val Glu
1195                1200                 1205

cta  aca aac att cag aag  aaa tat tac cga gcc  atc ctt gag aag        4185
Leu  Thr Asn Ile Gln Lys  Lys Tyr Tyr Arg Ala  Ile Leu Glu Lys
1210                1215                 1220

aat  ttc aca ttt ctt tcc  aaa ggc ggt ggt caa  gct aac gta cct        4230
Asn  Phe Thr Phe Leu Ser  Lys Gly Gly Gly Gln  Ala Asn Val Pro
1225                1230                 1235

aac  cta tta aac act atg  atg gaa ttg cgg aag  tgc tgc aat cat        4275
Asn  Leu Leu Asn Thr Met  Met Glu Leu Arg Lys  Cys Cys Asn His
1240                1245                 1250

ccg  tac ctt atc aat ggt  gct gaa gag aaa att  ttg gaa gag ttt        4320
Pro  Tyr Leu Ile Asn Gly  Ala Glu Glu Lys Ile  Leu Glu Glu Phe
1255                1260                 1265

aaa  gaa aca cac aat gca  gag tct cca gat ttt  cag ctc cag gca        4365
Lys  Glu Thr His Asn Ala  Glu Ser Pro Asp Phe  Gln Leu Gln Ala
1270                1275                 1280

atg  atc cag gct gct ggc  aag cta gtg ctg att  gac aag ctg ctg        4410
Met  Ile Gln Ala Ala Gly  Lys Leu Val Leu Ile  Asp Lys Leu Leu
1285                1290                 1295

cca  aaa ctg aag gct ggt  ggc cac agg gtg ctt  atc ttt tcc cag        4455
Pro  Lys Leu Lys Ala Gly  Gly His Arg Val Leu  Ile Phe Ser Gln
1300                1305                 1310

atg  gtg cgc tgc ttg gac  ata ctg gaa gac tac  ctc att caa aga        4500
Met  Val Arg Cys Leu Asp  Ile Leu Glu Asp Tyr  Leu Ile Gln Arg
1315                1320                 1325

cgg  tac cca tat gaa agg  atc gac ggc cga gta  aga ggc aac ctc        4545
Arg  Tyr Pro Tyr Glu Arg  Ile Asp Gly Arg Val  Arg Gly Asn Leu
1330                1335                 1340

cgc  cag gca gct atc gac  aga ttc tcc aaa cct  gat tct gat agg        4590
Arg  Gln Ala Ala Ile Asp  Arg Phe Ser Lys Pro  Asp Ser Asp Arg
1345                1350                 1355

ttt  gtt ttc ctc ctg tgt  aca agg gca gga ggt  tta ggc att aac        4635
Phe  Val Phe Leu Leu Cys  Thr Arg Ala Gly Gly  Leu Gly Ile Asn
```

1360 1365 1370

ctc act gct gct gat acc tgc atc atc ttt gat tca gac tgg aat 4680
Leu Thr Ala Ala Asp Thr Cys Ile Ile Phe Asp Ser Asp Trp Asn
1375 1380 1385

ccc caa aat gac ctc cag gct cag gct aga tgt cat aga ata gga 4725
Pro Gln Asn Asp Leu Gln Ala Gln Ala Arg Cys His Arg Ile Gly
1390 1395 1400

cag agc aaa tct gtg aaa atc tac agg ctg att aca aga aat tcc 4770
Gln Ser Lys Ser Val Lys Ile Tyr Arg Leu Ile Thr Arg Asn Ser
1405 1410 1415

tat gaa agg gaa atg ttc gac aag gct agt ttg aaa ctg ggc ctg 4815
Tyr Glu Arg Glu Met Phe Asp Lys Ala Ser Leu Lys Leu Gly Leu
1420 1425 1430

gat aaa gct gtg cta cag tct atg agt gga aga gaa aat gct acc 4860
Asp Lys Ala Val Leu Gln Ser Met Ser Gly Arg Glu Asn Ala Thr
1435 1440 1445

aat ggg gta caa cag ctt tcc aag aaa gaa ata gag gat ctt cta 4905
Asn Gly Val Gln Gln Leu Ser Lys Lys Glu Ile Glu Asp Leu Leu
1450 1455 1460

cga aaa ggg gcc tat ggt gca ctc atg gat gag gag gat gaa ggg 4950
Arg Lys Gly Ala Tyr Gly Ala Leu Met Asp Glu Glu Asp Glu Gly
1465 1470 1475

tct aaa ttc tgt gaa gaa gat att gat cag atc ctc cta cgt cga 4995
Ser Lys Phe Cys Glu Glu Asp Ile Asp Gln Ile Leu Leu Arg Arg
1480 1485 1490

acc cac acc att acc att gag tca gaa ggg aaa ggt tcc aca ttt 5040
Thr His Thr Ile Thr Ile Glu Ser Glu Gly Lys Gly Ser Thr Phe
1495 1500 1505

gct aag gcc agt ttt gtt gca tct gga aat agg aca gat att tcc 5085
Ala Lys Ala Ser Phe Val Ala Ser Gly Asn Arg Thr Asp Ile Ser
1510 1515 1520

ttg gat gat cca aat ttc tgg caa aag tgg gct aag aag gct gaa 5130
Leu Asp Asp Pro Asn Phe Trp Gln Lys Trp Ala Lys Lys Ala Glu
1525 1530 1535

ttg gat att gat gcc tta aat ggg agg aac aac ctg gtt att gat 5175
Leu Asp Ile Asp Ala Leu Asn Gly Arg Asn Asn Leu Val Ile Asp
1540 1545 1550

act cca aga gtg aga aag cag acc agg ctc tac agt gca gtg aag 5220
Thr Pro Arg Val Arg Lys Gln Thr Arg Leu Tyr Ser Ala Val Lys
1555 1560 1565

gaa gat gag ctg atg gag ttc tca gac ttg gaa agt gat tct gaa 5265
Glu Asp Glu Leu Met Glu Phe Ser Asp Leu Glu Ser Asp Ser Glu
1570 1575 1580

gaa aag ccc tgt gca aag cca cgg cgt ccc cag gat aag tca cag 5310
Glu Lys Pro Cys Ala Lys Pro Arg Arg Pro Gln Asp Lys Ser Gln
1585 1590 1595

ggc tat gca agg agt gaa tgt ttc agg gtg gag aag aat ctg ctt 5355

Gly Tyr Ala Arg Ser Glu Cys Phe Arg Val Glu Lys Asn Leu Leu
1600 1605 1610

gtc tat ggt gtg aat tgt tgc tag ttttgaccca ggatttccca 5399
Val Tyr Gly Val Asn Cys Cys
1615 1620

acgctggtac ctgacttaaa gtaaagccat aaatcaaaac tcacaggcac ctctgcatgc 5459

tggata 5465

<210> 6
<211> 1621
<212> PRT
<213> Homo sapiens

<400> 6

Met Ala Asp Pro Gly Met Met Ser Leu Phe Gly Glu Asp Gly Asn Ile
1 5 10 15

Phe Ser Glu Gly Leu Glu Gly Leu Gly Glu Cys Gly Tyr Pro Glu Asn
20 25 30

Pro Val Asn Pro Met Gly Gln Gln Met Pro Ile Asp Gln Gly Phe Ala
35 40 45

Ser Leu Gln Pro Ser Leu His His Pro Ser Thr Asn Gln Asn Gln Thr
50 55 60

Lys Leu Thr His Phe Asp His Tyr Asn Gln Tyr Glu Gln Gln Lys Met
65 70 75 80

His Leu Met Asp Gln Pro Asn Arg Met Met Ser Asn Thr Pro Gly Asn
85 90 95

Gly Leu Ala Ser Pro His Ser Gln Tyr His Thr Pro Pro Val Pro Gln
100 105 110

Val Pro His Gly Gly Ser Gly Gly Gly Gln Met Gly Val Tyr Pro Gly
115 120 125

Met Gln Asn Glu Arg His Gly Gln Ser Phe Val Asp Ser Ser Ser Met
130 135 140

Trp Gly Pro Arg Ala Val Gln Val Pro Asp Gln Ile Arg Ala Pro Tyr
145 150 155 160

Gln Gln Gln Gln Pro Gln Pro Gln Pro Pro Gln Pro Ala Pro Ser Gly
165 170 175

Pro Pro Ala Gln Gly His Pro Gln His Met Gln Gln Met Gly Ser Tyr
180 185 190

Met Ala Arg Gly Asp Phe Ser Met Gln Gln His Gly Gln Pro Gln Gln
195 200 205

Arg Met Ser Gln Phe Ser Gln Gly Gln Glu Gly Leu Asn Gln Gly Asn
210 215 220

Pro Phe Ile Ala Thr Ser Gly Pro Gly His Leu Ser His Val Pro Gln
225 230 235 240

Gln Ser Pro Ser Met Ala Pro Ser Leu Arg His Ser Val Gln Gln Phe
245 250 255

His His His Pro Ser Thr Ala Leu His Gly Glu Ser Val Ala His Ser
260 265 270

Pro Arg Phe Ser Pro Asn Pro Pro Gln Gln Gly Ala Val Arg Pro Gln
275 280 285

Thr Leu Asn Phe Ser Ser Arg Ser Gln Thr Val Pro Ser Pro Thr Ile
290 295 300

Asn Asn Ser Gly Gln Tyr Ser Arg Tyr Pro Tyr Ser Asn Leu Asn Gln
305 310 315 320

Gly Leu Val Asn Asn Thr Gly Met Asn Gln Asn Leu Gly Leu Thr Asn
325 330 335

Asn Thr Pro Met Asn Gln Ser Val Pro Arg Tyr Pro Asn Ala Val Gly
340 345 350

Phe Pro Ser Asn Ser Gly Gln Gly Leu Met His Gln Gln Pro Ile His
355 360 365

Pro Ser Gly Ser Leu Asn Gln Met Asn Thr Gln Thr Met His Pro Ser
370 375 380

Gln Pro Gln Gly Thr Tyr Ala Ser Pro Pro Pro Met Ser Pro Met Lys
385 390 395 400

Ala Met Ser Asn Pro Ala Gly Thr Pro Pro Pro Gln Val Arg Pro Gly
405 410 415

Ser Ala Gly Ile Pro Met Glu Val Gly Ser Tyr Pro Asn Met Pro His
420 425 430

Pro Gln Pro Ser His Gln Pro Pro Gly Ala Met Gly Ile Gly Gln Arg
435 440 445

Asn Met Gly Pro Arg Asn Met Gln Gln Ser Arg Pro Phe Ile Gly Met
450 455 460

Ser Ser Ala Pro Arg Glu Leu Thr Gly His Met Arg Pro Asn Gly Cys
465 470 475 480

Pro Gly Val Gly Leu Gly Asp Pro Gln Ala Ile Gln Glu Arg Leu Ile
485 490 495

Pro Gly Gln Gln His Pro Gly Gln Gln Pro Ser Phe Gln Gln Leu Pro
500 505 510

Thr Cys Pro Pro Leu Gln Pro His Pro Gly Leu His His Gln Ser Ser
515 520 525

Pro Pro His Pro His His Gln Pro Trp Ala Gln Leu His Pro Ser Pro
530 535 540

Gln Asn Thr Pro Gln Lys Val Pro Val His Gln His Ser Pro Ser Glu
545 550 555 560

Pro Phe Leu Glu Lys Pro Val Pro Asp Met Thr Gln Val Ser Gly Pro
565 570 575

Asn Ala Gln Leu Val Lys Ser Asp Asp Tyr Leu Pro Ser Ile Glu Gln
580 585 590

Gln Pro Gln Gln Lys Lys Lys Lys Lys Lys Asn Asn His Ile Val Ala
595 600 605

Glu Asp Pro Ser Lys Gly Phe Gly Lys Asp Asp Phe Pro Gly Gly Val
610 615 620

Asp Asn Gln Glu Leu Asn Arg Asn Ser Leu Asp Gly Ser Gln Glu Glu
625 630 635 640

Lys Lys Lys Lys Lys Arg Ser Lys Ala Lys Lys Asp Pro Lys Glu Pro
645 650 655

Lys Glu Pro Lys Glu Lys Lys Glu Pro Lys Glu Pro Lys Thr Pro Lys
660 665 670

Ala Pro Lys Ile Pro Lys Glu Pro Lys Glu Lys Lys Ala Lys Thr Ala
675 680 685

```
Thr Pro Lys Pro Lys Ser Ser Lys Lys Ser Ser Asn Lys Lys Pro Asp
    690                 695                 700

Ser Glu Ala Ser Ala Leu Lys Lys Lys Val Asn Lys Gly Lys Thr Glu
705                 710                 715                 720

Gly Ser Glu Asn Ser Asp Leu Asp Lys Thr Pro Pro Pro Ser Pro Pro
                725                 730                 735

Pro Glu Glu Asp Glu Asp Pro Gly Val Gln Lys Arg Arg Ser Ser Arg
            740                 745                 750

Gln Val Lys Arg Lys Arg Tyr Thr Glu Asp Leu Glu Phe Lys Ile Ser
        755                 760                 765

Asp Glu Glu Ala Asp Asp Ala Asp Ala Ala Gly Arg Asp Ser Pro Ser
    770                 775                 780

Asn Thr Ser Gln Ser Glu Gln Gln Glu Ser Val Asp Ala Glu Gly Pro
785                 790                 795                 800

Val Val Glu Lys Ile Met Ser Ser Arg Ser Val Lys Lys Gln Lys Glu
                805                 810                 815

Ser Gly Glu Glu Val Glu Ile Glu Glu Phe Tyr Val Lys Tyr Lys Asn
            820                 825                 830

Phe Ser Tyr Leu His Cys Gln Trp Ala Ser Ile Glu Asp Leu Glu Lys
        835                 840                 845

Asp Lys Arg Ile Gln Gln Lys Ile Lys Arg Phe Lys Ala Lys Gln Gly
    850                 855                 860

Gln Asn Lys Phe Leu Ser Glu Ile Glu Asp Glu Leu Phe Asn Pro Asp
865                 870                 875                 880

Tyr Val Glu Val Asp Arg Ile Met Asp Phe Ala Arg Ser Thr Asp Asp
                885                 890                 895

Arg Gly Glu Pro Val Thr His Tyr Leu Val Lys Trp Cys Ser Leu Pro
            900                 905                 910

Tyr Glu Asp Ser Thr Trp Glu Arg Arg Gln Asp Ile Asp Gln Ala Lys
        915                 920                 925

Ile Glu Glu Phe Glu Lys Leu Met Ser Arg Glu Pro Glu Thr Glu Arg
```

930 935 940

Val Glu Arg Pro Pro Ala Asp Asp Trp Lys Lys Ser Glu Ser Ser Arg
945 950 955 960

Glu Tyr Lys Asn Asn Asn Lys Leu Arg Glu Tyr Gln Leu Glu Gly Val
965 970 975

Asn Trp Leu Leu Phe Asn Trp Tyr Asn Met Arg Asn Cys Ile Leu Ala
980 985 990

Asp Glu Met Gly Leu Gly Lys Thr Ile Gln Ser Ile Thr Phe Leu Tyr
995 1000 1005

Glu Ile Tyr Leu Lys Gly Ile His Gly Pro Phe Leu Val Ile Ala
1010 1015 1020

Pro Leu Ser Thr Ile Pro Asn Trp Glu Arg Glu Phe Arg Thr Trp
1025 1030 1035

Thr Glu Leu Asn Val Val Val Tyr His Gly Ser Gln Ala Ser Arg
1040 1045 1050

Arg Thr Ile Gln Leu Tyr Glu Met Tyr Phe Lys Asp Pro Gln Gly
1055 1060 1065

Arg Val Ile Lys Gly Ser Tyr Lys Phe His Ala Ile Ile Thr Thr
1070 1075 1080

Phe Glu Met Ile Leu Thr Asp Cys Pro Glu Leu Arg Asn Ile Pro
1085 1090 1095

Trp Arg Cys Val Val Ile Asp Glu Ala His Arg Leu Lys Asn Arg
1100 1105 1110

Asn Cys Lys Leu Leu Glu Gly Leu Lys Met Met Asp Leu Glu His
1115 1120 1125

Lys Val Leu Leu Thr Gly Thr Pro Leu Gln Asn Thr Val Glu Glu
1130 1135 1140

Leu Phe Ser Leu Leu His Phe Leu Glu Pro Ser Arg Phe Pro Ser
1145 1150 1155

Glu Thr Thr Phe Met Gln Glu Phe Gly Asp Leu Lys Thr Glu Glu
1160 1165 1170

Gln Val Gln Lys Leu Gln Ala Ile Leu Lys Pro Met Met Leu Arg
1175 1180 1185

Arg Leu Lys Glu Asp Val Glu Lys Asn Leu Ala Pro Lys Glu Glu
1190 1195 1200

Thr Ile Ile Glu Val Glu Leu Thr Asn Ile Gln Lys Lys Tyr Tyr
1205 1210 1215

Arg Ala Ile Leu Glu Lys Asn Phe Thr Phe Leu Ser Lys Gly Gly
1220 1225 1230

Gly Gln Ala Asn Val Pro Asn Leu Leu Asn Thr Met Met Glu Leu
1235 1240 1245

Arg Lys Cys Cys Asn His Pro Tyr Leu Ile Asn Gly Ala Glu Glu
1250 1255 1260

Lys Ile Leu Glu Glu Phe Lys Glu Thr His Asn Ala Glu Ser Pro
1265 1270 1275

Asp Phe Gln Leu Gln Ala Met Ile Gln Ala Ala Gly Lys Leu Val
1280 1285 1290

Leu Ile Asp Lys Leu Leu Pro Lys Leu Lys Ala Gly Gly His Arg
1295 1300 1305

Val Leu Ile Phe Ser Gln Met Val Arg Cys Leu Asp Ile Leu Glu
1310 1315 1320

Asp Tyr Leu Ile Gln Arg Arg Tyr Pro Tyr Glu Arg Ile Asp Gly
1325 1330 1335

Arg Val Arg Gly Asn Leu Arg Gln Ala Ala Ile Asp Arg Phe Ser
1340 1345 1350

Lys Pro Asp Ser Asp Arg Phe Val Phe Leu Leu Cys Thr Arg Ala
1355 1360 1365

Gly Gly Leu Gly Ile Asn Leu Thr Ala Ala Asp Thr Cys Ile Ile
1370 1375 1380

Phe Asp Ser Asp Trp Asn Pro Gln Asn Asp Leu Gln Ala Gln Ala
1385 1390 1395

Arg Cys His Arg Ile Gly Gln Ser Lys Ser Val Lys Ile Tyr Arg
1400 1405 1410

```
Leu Ile  Thr Arg Asn Ser Tyr  Glu Arg Glu Met Phe  Asp Lys Ala
    1415                 1420                 1425

Ser Leu  Lys Leu Gly Leu Asp  Lys Ala Val Leu Gln  Ser Met Ser
    1430                 1435                 1440

Gly Arg  Glu Asn Ala Thr Asn  Gly Val Gln Gln Leu  Ser Lys Lys
    1445                 1450                 1455

Glu Ile  Glu Asp Leu Leu Arg  Lys Gly Ala Tyr Gly  Ala Leu Met
    1460                 1465                 1470

Asp Glu  Glu Asp Glu Gly Ser  Lys Phe Cys Glu Glu  Asp Ile Asp
    1475                 1480                 1485

Gln Ile  Leu Leu Arg Arg Thr  His Thr Ile Thr Ile  Glu Ser Glu
    1490                 1495                 1500

Gly Lys  Gly Ser Thr Phe Ala  Lys Ala Ser Phe Val  Ala Ser Gly
    1505                 1510                 1515

Asn Arg  Thr Asp Ile Ser Leu  Asp Asp Pro Asn Phe  Trp Gln Lys
    1520                 1525                 1530

Trp Ala  Lys Lys Ala Glu Leu  Asp Ile Asp Ala Leu  Asn Gly Arg
    1535                 1540                 1545

Asn Asn  Leu Val Ile Asp Thr  Pro Arg Val Arg Lys  Gln Thr Arg
    1550                 1555                 1560

Leu Tyr  Ser Ala Val Lys Glu  Asp Glu Leu Met Glu  Phe Ser Asp
    1565                 1570                 1575

Leu Glu  Ser Asp Ser Glu Glu  Lys Pro Cys Ala Lys  Pro Arg Arg
    1580                 1585                 1590

Pro Gln  Asp Lys Ser Gln Gly  Tyr Ala Arg Ser Glu  Cys Phe Arg
    1595                 1600                 1605

Val Glu  Lys Asn Leu Leu Val  Tyr Gly Val Asn Cys  Cys
    1610                 1615                 1620
```

<210>  7
<211>  20
<212>  DNA
<213>  Artificial Sequence

<220>

<223> primer

<400> 7
gaaacccaca ctgcagcaga 20

<210> 8
<211> 18
<212> DNA
<213> Artificial Sequence

<220>
<223> primer

<400> 8
tcgcttgccc ttctggcg 18

<210> 9
<211> 26
<212> DNA
<213> Artificial Sequence

<220>
<223> primer

<400> 9
gggaaatggg aggggtgcaa aagagg 26

<210> 10
<211> 26
<212> DNA
<213> Artificial Sequence

<220>
<223> primer

<400> 10
ttgcgtgagt gtggatggga ttggtg 26

<210> 11
<211> 23
<212> DNA
<213> Artificial Sequence

<220>
<223> primer

<400> 11
ctcagctaca aacaggtgaa gac 23

<210> 12
<211> 23
<212> DNA
<213> Artificial Sequence

<220>
<223> primer

<400> 12

tccctggtgg taggaagagt aaa 23

<210> 13
<211> 23
<212> DNA
<213> Artificial Sequence

<220>
<223> primer

<400> 13
ttgaatgtac agagtgcgga aga 23

<210> 14
<211> 22
<212> DNA
<213> Artificial Sequence

<220>
<223> primer

<400> 14
aacagccatc acagacgaat cc 22

<210> 15
<211> 20
<212> DNA
<213> Artificial Sequence

<220>
<223> primer

<400> 15
ggttcccaca ctcgtgcata 20

<210> 16
<211> 16
<212> DNA
<213> Artificial Sequence

<220>
<223> primer

<400> 16
tgcgcctcgg gacaga 16

<210> 17
<211> 23
<212> DNA
<213> Artificial Sequence

<220>
<223> primer

<400> 17
cccatgaaag caatgagtaa tcc 23

<210> 18
<211> 22
<212> DNA
<213> Artificial Sequence

<220>
<223> primer

<400> 18
tccattggta tcccagcact tc 22

<210> 19
<211> 23
<212> DNA
<213> Artificial Sequence

<220>
<223> primer

<400> 19
tgatggactt ggaacacaaa gtg 23

<210> 20
<211> 20
<212> DNA
<213> Artificial Sequence

<220>
<223> primer

<400> 20
tgaagggaag cgacttggtt 20

<210> 21
<211> 27
<212> DNA
<213> Artificial Sequence

<220>
<223> primer

<400> 21
caaacatggc tatgagaagt acaactc 27

<210> 22
<211> 20
<212> DNA
<213> Artificial Sequence

<220>
<223> primer

<400> 22
ccgactcgtt ccagaaagca 20

<210> 23
<211> 20
<212> DNA

<213>  Artificial Sequence

<220>
<223>  primer

<400>  23
ccactcctcc acctttgacg                                                  20

<210>  24
<211>  20
<212>  DNA
<213>  Artificial Sequence

<220>
<223>  primer

<400>  24
atgaggtcca ccaccctgtt                                                  20

<210>  25
<211>  30
<212>  DNA
<213>  Artificial Sequence

<220>
<223>  probe

<400>  25
tatgactcag aaaccgaaac agaaacgaca                                       30

<210>  26
<211>  22
<212>  DNA
<213>  Artificial Sequence

<220>
<223>  primer

<400>  26
gccctttcta gagaaaccag tg                                               22

<210>  27
<211>  20
<212>  DNA
<213>  Artificial Sequence

<220>
<223>  primer

<400>  27
aggcaccctt tcttctcctg                                                  20

<210>  28
<211>  28
<212>  DNA
<213>  Artificial Sequence

<220>

```
<223>  probe

<400>  28
cacggacgct ataaacgcca actcactg                                         28


<210>  29
<211>  23
<212>  DNA
<213>  Artificial Sequence

<220>
<223>  primer

<400>  29
gaatctgctt gtctatggtt ggg                                              23


<210>  30
<211>  20
<212>  DNA
<213>  Artificial Sequence

<220>
<223>  primer

<400>  30
aggatggttc tgcagatggt                                                  20
```